(19) Europäisches Patentamt / European Patent Office / Office européen des brevets

(11) EP 1 670 766 B1

(12) **EUROPÄISCHE PATENTSCHRIFT**

(45) Veröffentlichungstag und Bekanntmachung des Hinweises auf die Patenterteilung:
**21.03.2012 Patentblatt 2012/12**

(21) Anmeldenummer: **04786944.1**

(22) Anmeldetag: **14.09.2004**

(51) Int Cl.:
***C07D 217/24*** *(2006.01)* ***C07D 217/26*** *(2006.01)*
***A61K 31/47*** *(2006.01)* ***A61P 19/10*** *(2006.01)*

(86) Internationale Anmeldenummer:
**PCT/EP2004/010248**

(87) Internationale Veröffentlichungsnummer:
**WO 2005/030728 (07.04.2005 Gazette 2005/14)**

(54) **Bicyclische Iminosäurederivate zur Behandlung von (unter anderem) Erkrankungen des Bewegungsapparates**

Bicyclic imino acid derivatives for the treatment of (inter alia) diseases relating to the locomotor system

Derivés d'iminoacides bicycliques pour le traitement (entre autres) d'affections de l'appareil locomoteur

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **27.09.2003 DE 10344936**

(43) Veröffentlichungstag der Anmeldung:
**21.06.2006 Patentblatt 2006/25**

(73) Patentinhaber: **Sanofi-Aventis Deutschland GmbH**
**65929 Frankfurt am Main (DE)**

(72) Erfinder:
- **SCHUDOK, Manfred**
**65817 Eppstein/Ts. (DE)**
- **MATTER, Hans**
**63505 Langenselbold (DE)**
- **HOFMEISTER, Armin**
**55278 Dexheim (DE)**

(56) Entgegenhaltungen:
**WO-A-97/18194**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 670 766 B1

Printed by Jouve, 75001 PARIS (FR)

## Beschreibung

Die Erfindung betrifft neue Derivate gesättigter bicylischer Iminosäuren, wie insbesondere der Decahydroisochinolin-1-carbonsäure, Verfahren zu ihrer Herstellung und Verwendung derselben als Arzneimittel.

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP's). Die MMP's bilden eine Gruppe von Zn-abhängigen Enzymen, die am biologischen Abbau der extrazellulären Matrix beteiligt sind (D. Yip et al. in Investigational New Drugs 17 (1999), 387-399 und Michaelides et al. in Current Pharmaceutical Design 5 (1999) 787 -819). Diese MMP's sind insbesondere fähig, fibrilläres und nicht-fibrilläres Kollagen, sowie Proteoglycane abzubauen, die beide wichtige Matrixbestandteile darstellen. MMP's sind beteiligt an Prozessen der Wundheilung, der Tumorinvasion, Metastasenwanderung sowie an Angiogenese, multipler Sklerose und Herzversagen (Michaelides Seite 788; siehe oben). Insbesondere spielen sie eine wichtige Rolle beim Abbau der Gelenkmatrix in der Arthrose und der Arthritis, sei es nun die Osteoarthrose, Osteoarthritis oder die rheumatoide Arthritis.

Die Aktivität der MMP's ist weiterhin essentiell für viele der Prozesse, die bei der atherosklerotischen Plaque-Bildung eine Rolle spielen, wie die Infiltration inflammatorischer Zellen, der glatten Muskelzell-Migration sowie Proliferation und Angiogenese (S. J. George, Exp. Opin. Invest. Drugs (2000), 9 (5), 993-1007). Weiterhin kann die Matrix-Degradation durch MMPs Plaque-Instabilitäten bis hin zu Rupturen verursachen, was zu den klinischen Symptomen der Atherosklerose, instabilen Angina Pectoris, Myokardinfarkt oder Schlaganfall führen kann (E. J. M. Creemers et al, Circulation Res. 89, 201-210 (2001)). Insgesamt betrachtet kann die gesamte MMP-Familie alle Komponenten der extrazellulären Matrix der Blutgefäße abbauen; deshalb ist deren Aktivität in starkem Maße Regulationsmechanismen in normalen Blutgefäßen unterworfen. Die erhöhte MMP-Aktivität während der Plaque-Bildung und Plaque-Instabilität wird durch erhöhte Cytokin- und Growth-Faktor-stimulierte Gen-Transkription, erhöhte Zymogen-Aktivivierung und eine Imbalance des MMP-TIMP-Verhältnisses verursacht (Tissue inhibitors of metalloproteases). Deshalb erscheint es einleuchtend, dass eine MMP-Inhibierung oder die Wiedererlangung der MMP-TIMP-Balance hilfreich sein wird in der Behandlung der atherosklerotischen Erkrankungen. Ebenso wird es immer deutlicher, dass neben der Atherosklerose auch weitere cardiovaskuläre Erkrankungen durch eine erhöhte MMP-Aktivität zumindest mitverursacht werden, wie beispielsweise Restenose, dilatierte Cardiomyopathie und der schon erwähnte Myokardinfarkt. Es konnte gezeigt werden, dass durch die Applikation synthetischer Inhibitoren in experimentellen Tiermodellen dieser Erkrankungen deutliche Verbesserungen erzielt werden konnten, z. B. betreffend Bildung atherosklerotischer Läsionen, Neointima-Bildung, Linksventrikuläres Remodelling, Dysfunktion der Pumpleistung oder Infarkt-Heilung. In verschiedenen präklinischen Studien mit MMP-Inhibitoren zeigte sich bei detaillierter Gewebsanalyser eine reduzierte Kollagen-Schädigung, verbessertes extrazelluläres Matrix-Remodelling und verbesserte Struktrur und Funktion von Herzmuskel und Gefäßen. Von diesen Prozessen werden insbesondere die Matrix-Remodelling-Prozesse und MMP-regulierte Fibrosen als wichtige Komponenten im Fortschreiten von Herzerkrankungen (Infarkt) angesehen (Drugs 61, 1239-1252 (2001)).

MMP's spalten Matrixproteine wie Kollagen, Laminin, Proteoglykane, Elastin oder Gelatin sowie prozessieren (d. h. aktivieren oder desaktivieren) durch eine Spaltung eine Vielzahl weiterer Proteine und Enzyme unter physiologischen Bedingungen, so daß Ihnen eine wichtige Rolle im gesamten Organismus zukommt, mit besonderer Bedeutung im Bindegewebe und Knochen.

Eine Vielzahl von verschiedenen Inhibitoren der MMP's sind bekannt (EP 0 606 046; WO 94/28889; WO 96/27583; vgl. auch Übersichten wie z. B. Current Medicinal Chemistry 8, 425-74 (2001). Nach den ersten klinischen Studien an Menschen hat sich nun gezeigt, dass MMP's Nebenwirkungen hervorrufen. Die hauptsächlich genannten Nebenwirkungen sind muskuloskeletale Schmerzen oder Anthralgien. Der Stand der Technik besagt eindeutig, dass erwartet wird, dass selektivere Inhibitoren diese genannten Nebenwirkungen reduzieren können (Yip, Seite 387, siehe oben). Besonders hervorzuheben ist dabei eine Spezifität gegenüber MMP-1, da mit der Hemmung von MMP-1 offensichtlich diese unerwünschten Nebenwirkungen verstärkt auftreten.

Nachteil der bekannten Inhibitoren der MMP's sind daher häufig die mangelnde Spezifität. Die meisten MMP-Inhibitoren hemmen viele MMP's gleichzeitig, weil die katalytische Domäne der MMP's eine ähnliche Struktur aufweist. Demzufolge wirken die Inhibitoren in unerwünschter Weise auf die Enzyme, auch solche mit vitaler Funktion, ein (Massova I, et al., The FASEB Journal (1998) 12, 1075-1095).

In dem Bestreben, wirksame Verbindungen zur Behandlung von Bindegewebserkrankungen zu finden, wurde nun gefunden, dass die erfindungsgemäß eingesetzten Derivate starke Inhibitoren der Matrix-Metalloproteinasen MMP-2, MMP-3 MMP-8, MMP-9 und MMP-13 sind, während nur eine schwache Hemmung der MMP-1.

Die Erfindung betrifft daher eine Verbindung der Formel I

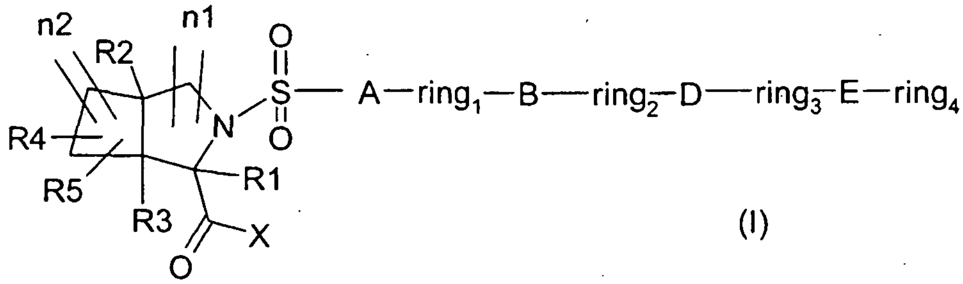

(I)

und/oder alle stereoisomeren Formen der Verbindung der Formel I und/oder Gemische dieser Formen in jedem Verhältnis, und/oder ein physiologisch verträgliches Salz der Verbindung der Formel I, wobei
A für -($C_0$-$C_4$)-Alkylen steht,
B, D und E gleich oder verschieden sind und unabhängig voneinander für -($C_0$-$C_4$)-Alkylen oder den Rest -B1-B2-B3- stehen, worin
B1 für -$(CH_2)_n$- steht, worin n die ganze Zahl Null, 1 oder 2 bedeutet,
B3 für -$(CH_2)_m$- steht, worin m die ganze Zahl Null, 1 oder 2 bedeutet, mit der Maßgabe, dass die Summe von n und m den Betrag Null, 1 oder 2 hat, und B2 für

1) -C(O)-
2) -($C_2$-$C_4$)-Alkenylen,
3) -$S(O)_O$-, wobei o die ganzen Zahlen Null, 1 oder 2 bedeutet,
4) -N(R6)-, worin R6 Wasserstoffatom, Methyl oder Ethyl bedeutet,
5) -N(R6)-C(Y)-, worin Y Sauerstoffatom oder Schwefelatom bedeutet und R6 wie oben definiert ist,
6) -C(Y)-N(R6)-, worin Y Sauerstoffatom oder Schwefelatom bedeutet und R6 wie oben definiert ist,
7) -N($R_6$)-$SO_2$-, worin R6 wie oben definiert ist,
8) -$SO_2$-N(R6)-, worin R6 wie oben definiert ist,
9) -N(R6)-$SO_2$-N(R6)-, worin R6 wie oben definiert ist,
10) -N(R6)-C(Y)-N(R6)-, worin Y Sauerstoffatom oder Schwefelatom bedeutet und R6 wie oben definiert ist,
11) -0-C(0)-N(R6)-,
12) -NH-C(O)-O-,
13) -O-,
14) -C(0)-0-,
15) -O-C(O)-,
16) -O-C(O)-O-,
17) -O-$CH_2$-C(O)-,
18) -O-$CH_2$-C(O)-O-,
19) -O-$CH_2$-C(O)-N(R6)-, worin R6 wie oben definiert ist,
20) -C(O)-$CH_2$-O-,
21) -O-C(O)-$CH_2$-O-,
22) -N(R6)-C(O)-$CH_2$-O-, worin R6 wie oben definiert ist,
23) -O-$(CH_2)_n$-O-, worin n die ganze Zahl 2 oder 3 bedeutet, oder
24) -O-$(CH_2)_m$-N(R6)-, worin m die ganze Zahl 2 oder 3 bedeutet und R6 wie oben definiert ist,
25) -N(R6)-$(CH_2)_m$-O-, worin m die ganze Zahl 2 oder 3 bedeutet und R6 wie oben definiert ist,
26) -N(R6)-N(R6)-, worin R6 wie oben definiert ist,
27) -N=N-,
28) -N(R6)-CH=N-, worin R6 wie oben definiert ist,
29) -N=CH-N(R6)-, worin R6 wie oben definiert ist,
30) -N(R6)-C(R7)=N-, worin R6 wie oben definiert ist und R7 -NH-R6 bedeutet,
31) -N=C(R7)-N(R6)-, worin R6 wie oben definiert ist und R7 -NH-R6 bedeutet, oder
32) -($C_2$-$C_6$)-Alkinylen, steht,

ring1, ring2 oder ring3 gleich oder verschieden sind und unabhängig voneinander für

1) kovalente Bindung,
2) -($C_6$-$C_{14}$)-Aryl, worin Aryl unsubstituiert oder unabhängig voneinander ein-, zwei- oder dreifach durch G substituiert

ist, oder
3) 4- bis 15-gliedriger Het-Ring, worin Het-Ring unsubstituiert oder unabhängig voneinander ein-, zwei- oder dreifach durch G substituiert ist, steht,

ring4 für

1) -($C_6$-$C_{14}$)-Aryl, worin Aryl unsubstituiert oder unabhängig voneinander ein-, zwei- oder dreifach durch G substituiert ist,
2) 4- bis 15-gliedriger Het-Ring, worin der Het-Ring unsubstituiert oder unabhängig voneinander ein-, zwei- oder dreifach durch G substituiert ist, oder
3) für einen der folgenden Reste

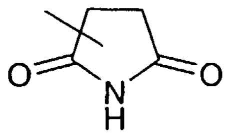 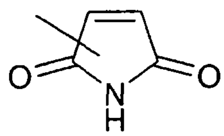

und diese Reste unsubstituiert oder einfach durch G substituiert sind, steht,

G für

1) Wasserstoffatom,
2) Halogen,
3) =0,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist,
5) -($C_6$-$C_{14}$)-Aryl,
6) Het-Ring,
7) -C(0)-0-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, oder
b) -($C_6$-$C_{14}$)-Aryl oder Het-Ring, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Het-Ring substituiert ist, oder
b) -($C_6$-$C_{14}$)-Aryl oder Het-Ring, bedeutet,

10) -C(S)-NH-R11, worin R11 wie oben definiert ist, bedeutet,
11) -0-R12, worin R12

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist,
c) -($C_6$-$C_{14}$)-Aryl,
d) Het-Ring,
e) -C(O)-O-R13, worin R13

e)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, oder
e)2) -($C_6$-$C_{14}$)-Aryl oder Het-Ring, bedeutet,

f) -C(S)-O-R13, worin R13 wie oben definiert ist,
g) -C(0)-NH-R14, worin R14

g)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, oder
g)2) -($C_6$-$C_{14}$)-Aryl oder Het-Ring bedeutet, oder

h) -C(S)-NH-R14, worin R14 wie oben definiert ist, bedeutet,

12) -C(0)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN oder
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, bedeutet und R12 wie oben definiert ist, oder

17) -$SO_2$-N(R12)-R1, worin R12 wie oben definiert ist und R1 wie unten definiert ist, steht,
X für -OH oder -NH-OH steht,
n1 für die ganze Zahl 2 steht,
n2 für die ganze Zahl 3 steht,

R1, R2, R3, R4 und R5 gleich oder verschieden sind und unabhängig voneinander für

1) Wasserstoffatom,
2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, steht,
3. -C(0)-0-R8, worin R8

3)1) Wasserstoffatom,
3)2) -($C_1$-$C_6$)-Alkyl, worin Alkyl unsubstituiert oder ein- oder zweifach durch - ($C_3$-$C_6$)-Cycloalkyl, -($C_2$-$C_6$)-Alkinyl, -($C_6$-$C_{14}$)-Aryl, oder Het-Ring oder einbis fünffach durch Fluor, substituiert ist, oder
3)3) -($C_6$-$C_{14}$)-Aryl oder Het-Ring bedeutet,

4) -0-R8, worin R8 die oben genannte Bedeutung hat, oder
5) -($C_3$-$C_6$)-Cycloalkyl, stehen.

Ein weiterer Gegenstand der Erfindung ist die Verbindung der Formel I, wobei
A für -($C_0$-$C_4$)-Alkylen steht,
B, D und E gleich oder verschieden sind und unabhängig voneinander für -($C_0$-$C_4$)-Alkylen oder den Rest -B1-B2-B3- stehen, worin
B1 für -$(CH_2)_n$- steht, worin n die ganze Zahl Null, 1 oder 2 bedeutet,
B3 für -$(CH_2)_m$- steht, worin m die ganze Zahl Null, 1 oder 2 bedeutet, mit der Maßgabe, dass die Summe von n und m den Betrag Null, 1 oder 2 hat, und B2 für

1) -C(O)- ,
2) -($C_2$-$C_4$)-Alkenylen,
3) -$S(O)_O$-, wobei o die ganzen Zahlen Null, 1 oder 2 bedeutet,
4) -N(R6)-, worin R6 Wasserstoffatom, Methyl oder Ethyl bedeutet,
5) -N(R6)-C(Y)-, worin Y Sauerstoffatom oder Schwefelatom bedeutet und R6 wie oben definiert ist,
6) -C(Y)-N(R6)-, worin Y Sauerstoffatom oder Schwefelatom bedeutet und R6 wie oben definiert ist,
7) -N(R6)-$SO_2$-, worin R6 wie oben definiert ist,
8) -$SO_2$-N(R6)-, worin R6 wie oben definiert ist,
9) -N(R6)-$SO_2$-N(R6)-, worin R6 wie oben definiert ist,
10) -N(R6)-C(Y)-N(R6)-, worin Y Sauerstoffatom oder Schwefelatom bedeutet und R6 wie oben definiert ist,
11) -O-C(O)-N(R6)-,
12) -NH-C(0)-0-,
13) -0-,

14) -C(0)-0-,
15) -O-C(O)-,
16) -O-C(O)-O-,
17) -O-$CH_2$-C(O)-,
18) -O-$CH_2$-C(O)-O-,
19) -O-$CH_2$-C(O)-N(R6)-, worin R6 wie oben definiert ist,
20) -C(O)-$CH_2$-O-,
21) -O-C(O)-$CH_2$-O-,
22) -N(R6)-C(O)-$CH_2$-O-, worin R6 wie oben definiert ist,
23) -O-$(CH_2)_n$-O-, worin n die ganze Zahl 2 oder 3 bedeutet, oder
24) -O-$(CH_2)_m$-N(R6)-, worin m die ganze Zahl 2 oder 3 bedeutet und R6 wie oben definiert ist,
25) -N(R6)-$(CH_2)_m$-O-, worin m die ganze Zahl 2 oder 3 bedeutet und R6 wie oben definiert ist,
26) -N(R6)-N(R6)-, worin R6 wie oben definiert ist,
27) -N=N-,
28) -N(R6)-CH=N-, worin R6 wie oben definiert ist,
29) -N=CH-N(R6)-, worin R6 wie oben definiert ist,
30) -N(R6)-C(R7)=N-, worin R6 wie oben definiert ist und R7 -NH-R6 bedeutet,
31) -N=C(R7)-N(R6)-, worin R6 wie oben definiert ist und R7 -NH-R6 bedeutet, oder
32) -($C_2$-$C_6$)-Alkinylen, steht,

ring1, ring2 oder ring3 gleich oder verschieden sind und unabhängig voneinander für

1) kovalente Bindung,
2) Phenyl oder Naphthyl bedeutet und unsubstituiert oder unabhängig voneinander ein-, zwei- oder dreifach durch G substituiert sind, oder
3) 4- bis 15-gliedriger Het-Ring steht, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pyridooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,4-Triazolyl, 1,2,5-Triazolyl, 1,3,4-Triazolyl und Xanthenyl ist und unsubstituiert oder unabhängig voneinander ein-, zwei- oder dreifach durch G substituiert sind,

ring4 für

1) -($C_6$-$C_{14}$)-Aryl steht, worin Aryl ein Rest aus der Reihe Phenyl, Naphthyl, 1-Naphthyl, 2-Naphthyl, Anthryl oder Fluorenyl bedeutet und unsubstituiert oder unabhängig voneinander ein-, zwei- oder dreifach durch G substituiert sind,
2) 4- bis 15-gliedriger Het-Ring steht, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, 2'-Methylbiphenyl-2-ol, Morpholinyl, Naphthyridinyl, Octahydroisochinolinyl, Oxadiazolyl, 1,2,3-Oxadiazolyl, 1;2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,4-Triazolyl, 1,2,5-Triazolyl, 1,3,4-Triazolyl und Xanthenyl ist und unsubstituiert oder unabhängig voneinander ein-, zwei- oder dreifach durch G substituiert sind, oder

3) für einen der folgenden Reste

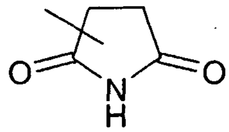

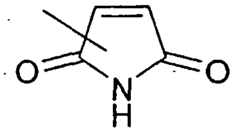

steht und diese Reste unsubstituiert oder einfach durch G substituiert sind,

G für

1) Wasserstoffatom,
2) Halogen,
3) =0,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist, wobei Aryl und Het-Ring wie oben definiert sind,
5) -($C_6$-$C_{14}$)-Aryl, wobei Aryl wie oben definiert ist,
6) Het-Ring, wobei Het-Ring wie oben definiert ist,
7) -C(O)-O-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, wobei Aryl und Het-Ring wie oben definiert sind, oder
b) -($C_6$-$C_{14}$)-Aryl oder Het-Ring, wobei Aryl und Het-Ring wie oben definiert sind, bedeutet,

8) -C(S)-0-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl , -($C_6$-$C_{14}$)-Aryl oder Het-Ring substituiert ist, wobei Aryl und Het-Ring wie oben definiert sind, oder
b) -($C_6$-$C_{14}$)-Aryl oder Het-Ring, wobei Aryl und Het-Ring wie oben definiert sind, bedeutet,

10) -C(S)-NH-R11, worin R11 wie oben definiert ist, bedeutet,
11) -0-R12, worin R12

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl , -($C_6$-$C_{14}$)-Aryl oder Het-Ring substituiert ist, wobei Aryl und Het-Ring wie oben definiert sind,
c) -($C_6$-$C_{14}$)-Aryl, wobei Aryl wie oben definiert ist,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl , -($C_6$-$C_{14}$)-Aryl, oder Het-Ring substituiert ist, wobei Aryl und Het-Ring wie oben definiert sind, oder
e)2) -($C_6$-$C_{14}$)-Aryl oder Het-Ring, wobei Aryl und Het-Ring wie oben definiert sind, bedeutet,

f) -C(S)-O-R13, worin R13 wie oben definiert ist,

g) -C(0)-NH-R14, worin R14

g)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl , -($C_6$-$C_{14}$)-Aryl oder Het-Ring substituiert ist, wobei Aryl und Het-Ring wie oben definiert sind, oder

g)2) -($C_6$-$C_{14}$)-Aryl oder Het-Ring bedeutet, wobei Aryl und Het-Ring wie oben definiert sind, oder

h) -C(S)-NH-R14, worin R14 wie oben definiert ist, bedeutet,

12) -C(0)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN oder
16) -N(R15)-R12, worin R15

16)1) Wasserstoffatom oder
16.2) -($C_1$-$C_6$)-Alkyl bedeutet und R12 wie oben definiert ist, steht,
16.3) -$SO_2$-($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist

17) -$SO_2$-N(R12)-R1, worin R12 wie oben definiert ist und R1 wie unten definiert ist,
X für -OH oder -NH-OH steht,
n1 für die ganze Zahl 2 steht,
n2 für die ganze Zahl 3 steht,

R1, R2, R3, R4 und R5 gleich oder verschieden sind und unabhängig voneinander für

1) Wasserstoffatom,
2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, steht,
3) -C(O)-O-R8, worin R8

3)1) Wasserstoffatom,
3)2) -($C_1$-$C_6$)-Alkyl, worin Alkyl unsubstituiert oder ein- oder zweifach durch - ($C_3$-$C_6$)-Cycloalkyl, -($C_2$-$C_6$)-Alkinyl, -($C_6$-$C_{14}$)-Aryl oder Het-Ring oder einbis fünffach durch Fluor, substituiert ist, oder
3)3) -($C_6$-$C_{14}$)-Aryl oder Het-Ring bedeutet,

4) -0-R8, worin R8 die oben genannte Bedeutung hat, oder
5) -($C_3$-$C_6$)-Cycloalkyl, stehen.

Ein weiterer Gegenstand der Erfindung ist die Verbindung der Formel I, wobei A für -($C_0$-$C_4$)-Alkylen steht, B, D und E gleich oder verschieden sind und unabhängig voneinander für -($C_0$-$C_4$)-Alkylen oder den Rest -B1-B2-B3- stehen, worin
B1 für -$(CH_2)_n$- steht, worin n die ganze Zahl Null, 1 oder 2 bedeutet,
B3 für -$(CH_2)_m$- steht, worin m die ganze Zahl Null, 1 oder 2 bedeutet,
mit der Maßgabe, dass die Summe von n und m den Betrag Null, 1 oder 2 hat, und B2 für

1) -($C_0$-$C_2$)-Alkylen,
2) Ethenylen,
3) Ethinylen,
4) -C(0)-
5) -N(R6)-C(0)-, worin R6 Wasserstoffatom, Methyl oder Ethyl bedeutet,
6) -C(0)-N(R6)-, worin R6 wie oben definiert ist,
7) -0- oder
8) -S-,

ring1, ring2 oder ring3 gleich oder verschieden sind und unabhängig voneinander für

1) kovalente Bindung stehen,
2) Phenyl oder Naphthyl bedeutet und unsubstituiert oder unabhängig voneinander ein- oder zweifach durch G substituiert sind, oder
3) Het-Ring stehen, worin der Het-Ring ein Rest aus der Reihe Dihydrofuranyl, Furanyl, Pyridinyl, Pyrimidinyl, Pyrrolyl, Thiadiazolyl, Thiazolyl oder Thiophenyl bedeutet und unsubstituiert oder unabhängig voneinander ein- oder zweifach durch G substituiert sind,

ring4 für

1) Phenyl oder Naphthyl steht und unsubstituiert oder unabhängig voneinander ein-, oder zweifach durch G substituiert ist,
2) Het-Ring steht, worin der Het-Ring ein Rest aus der Reihe Benzofuranyl, Dihydrofuranyl, Dibenzofuranyl, Dibenzothiophenyl, Furanyl, 2'-Methyl-biphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und unsubstituiert oder unabhängig voneinander ein- oder zweifach durch G substituiert ist, oder
3) für den folgenden Rest

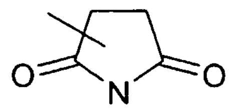

steht und dieser Rest unsubstituiert oder einfach durch G substituiert ist, G für

1) Wasserstoffatom,
2) Br, Cl oder F,
3) -($C_1$-$C_4$)-Alkyl, worin Alkyl unsubstituiert oder ein-, oder zweifach substituiert ist durch F, Phenyl, -$C_3$-Cycloalkyl oder Het- Ring, wobei Het- Ring wie oben definiert ist,
4) Phenyl,
5) Het-Ring, wobei Het-Ring wie oben definiert ist,
6) -C(O)-O-R10, worin R10

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

7) -C(O)-NH-R11, worin R11

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

8) -0-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch Cyclopropyl, Phenyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-O-R13, worin R13 wie oben definiert ist, oder

g) -C(0)-NH-R14, worin R14

g)1) $-(C_1-C_6)$-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) $-S(O)_p$-R12, worin R12 wie oben definiert ist und p die ganzen Zahlen 1 oder 2 bedeutet,
11) $-NO_2$,
12) -CN oder
13) -N(R15)-R12, worin R15

13)1) Wasserstoffatom oder
13)2) $-(C_1-C_6)$-Alkyl bedeutet und R12 wie oben definiert ist, steht,

X für -OH oder -NH-OH steht,
n1 für die ganze Zahl 2 steht,
n2 für die ganze Zahl 3 steht,
R1, R2 und R3 jeweils für Wasserstoffatom stehen,
R4 und R5 gleich oder verschieden sind und unabhängig voneinander für

1) Wasserstoffatom,
2) Methyl,
3) Ethyl oder
4) -OH stehen.

Ein weiterer Gegenstand der Erfindung ist die Verbindung der Formel I, wobei A für $-(C_0-C_4)$-Alkylen steht, B, D und E gleich oder verschieden sind und unabhängig voneinander für $-(C_0-C_4)$-Alkylen oder den Rest -B1-B2-B3- stehen, worin
B1 für $-(CH_2)_n$- steht, worin n die ganze Zahl Null, 1 oder 2 bedeutet,
B3 für $-(CH_2)_m$- steht, worin m die ganze Zahl Null, 1 oder 2 bedeutet,
mit der Maßgabe, dass die Summe von n und m den Betrag Null, 1 oder 2 hat, und B2 für

1) $-(C_0-C_2)$-Alkylen,
2) Ethenylen oder
3) Ethinylen,

ring1, ring2 oder ring3 gleich oder verschieden sind und unabhängig voneinander für

1) kovalente Bindung stehen,
2) Phenyl bedeutet und unsubstituiert oder unabhängig voneinander ein- oder zweifach durch G substituiert sind, oder
3) Het-Ring stehen, worin der Het-Ring ein Rest aus der Reihe Dihydrofuranyl, Furanyl, Morpholinyl, Piperazinyl, Piperidinyl, Pyridinyl, Pyrimidinyl, Pyrrolyl, Thiazolyl oder Thiophenyl bedeutet und unsubstituiert oder unabhängig voneinander ein- oder zweifach durch G substituiert sind,

ring4 für

1) Phenyl steht und unsubstituiert oder unabhängig voneinander ein-, oder zweifach durch G substituiert ist,
2) Het-Ring steht, worin der Het-Ring ein Rest aus der Reihe Benzofuranyl, Dihydrofuranyl, Dibenzofuranyl, Dibenzothiophenyl, Furanyl, 2'-Methylbiphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und bedeutet und unsubstituiert oder unabhängig voneinander ein- oder zweifach durch G substituiert ist, oder
3) für den folgenden Rest

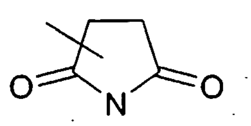

steht und dieser Rest unsubstituiert oder einfach durch G substituiert ist,

G für

1) Wasserstoffatom,
2) Br, Cl oder F,
3) -($C_1$-$C_4$)-Alkyl, worin Alkyl unsubstituiert oder ein-, zwei- oder dreifach durch Br, Cl, F, -$C_3$-Cycloalkyl, Phenyl oder Het- Ring, wobei Het- Ring wie oben definiert ist,
4) Phenyl,
5) Het-Ring, wobei Het-Ring wie oben definiert ist,
6) -C(O)-O-R10, worin R10

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

7) -C(O)-NH-R11, worin R11

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl oder Naphthyl, oder
c) Het-Ring, wobei Het-Ring wie oben definiert ist, bedeutet,

8) -0-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch Cyclopropyl, Phenyl, Naphthyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-0-R13, worin R13 wie oben definiert ist, oder
g) -C(0)-NH-R14, worin R14

g)1) -($C_1$-$C_6$)-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) -$S(O)_p$-R12, worin R12 wie oben definiert ist und p die ganzen Zahlen 1 oder 2 bedeutet,
11) -$NO_2$,
12) -CN oder
13) -N(R15)-R12, worin R15

13)1) Wasserstoffatom oder
13)2) -($C_1$-$C_6$)-Alkyl bedeutet und R12 wie oben definiert ist, steht,

X für -OH oder-NH-OH steht,
n1 für die ganze Zahl 2 steht,
n2 für die ganze Zahl 3 steht,
R1, R2, R3, R4 und R5 jeweils für Wasserstoffatom stehen
Ein weiterer Gegenstand der Erfindung ist die Verbindung der Formel I, wobei

A für eine kovalente Bindung oder $-CH_2-CH_2-$ steht,
B, D und E gleich oder verschieden sind und unabhängig voneinander für $-(C_0-C_4)$-Alkylen oder den Rest -B1-B2-B3- stehen, worin
B1 für $-(CH_2)_n-$ steht, worin n die ganze Zahl Null, 1 oder 2 bedeutet,
B3 für $-(CH_2)_m-$ steht, worin m die ganze Zahl Null, 1 oder 2 bedeutet, mit der Maßgabe, dass die Summe von n und m den Betrag Null, 1 oder 2 hat, und B2 für

1) -C(O)-
2) $-(C_2-C_4)$-Alkinylen,
3) $-S(O)_O-$, wobei o die ganzen Zahlen Null oder 1 bedeutet,
4) -N(R6)-C(Y)-, worin Y Sauerstoffatom und R6 Wasserstoffatom bedeutet,
5) -C(Y)-N(R6)-, worin Y Sauerstoffatom und R6 Wasserstoffatom bedeutet, oder
6) -0- steht

ring1, ring2 oder ring3 gleich oder verschieden sind und unabhängig voneinander für

1) kovalente Bindung stehen,
2) Phenyl bedeutet und unsubstituiert oder unabhängig voneinander ein- oder zweifach durch G substituiert sind, oder
3) Het-Ring stehen, worin der Het-Ring ein Rest aus der Reihe Furanyl, Pyridinyl, Pyrimidinyl oder Thiophenyl bedeutet und unsubstituiert oder unabhängig voneinander ein- oder zweifach durch G substituiert sind,

ring4 für

1) Phenyl steht und unsubstituiert oder unabhängig voneinander ein- oder zweifach durch G substituiert ist,
2) Het-Ring steht, worin der Het-Ring ein Rest aus der Reihe Benzofuranyl, Dibenzofuranyl, Furanyl, 2'-Methyl-biphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und unsubstituiert oder unabhängig voneinander ein- oder zweifach durch G substituiert ist, oder
3) für den folgenden Rest

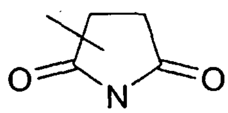

steht und dieser Rest unsubstituiert oder einfach durch G substituiert ist,

G für

1) Wasserstoffatom,
2) Br, Cl oder F,
3) $-(C_1-C_4)$-Alkyl, worin Alkyl unsubstituiert oder ein-, zwei- oder dreifach durch Br, Cl, F, $-C_3$-Cycloalkyl, Phenyl oder Het- Ring, wobei Het- Ring wie oben definiert ist,
4) Phenyl,
5) Het-Ring, wobei Het-Ring wie oben definiert ist,
6) -C(O)-O-R10, worin R10

a) $-(C_1-C_6)$-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

7) -C(O)-NH-R11, worin R11

a) $-(C_1-C_6)$-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

8) -0-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(0)-0-R13, worin R13

e)1) -($C_1$-$C_6$)-Alkyl, worin Alkyl unsubstituiert oder ein- oder zweifach durch Cyclopropyl, Phenyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-O-R13, worin R13 wie oben definiert ist, oder
g) -C(0)-NH-R14, worin R14

g)1) -($C_1$-$C_6$)-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) -$S(O)_p$-R12, worin R12 wie oben definiert ist und p die ganzen Zahlen Null, 1 oder 2 bedeutet,
11) -$NO_2$,
12) -CN oder
13) -N(R15)-R12, worin R15

13)1) Wasserstoffatom oder
13)2) -($C_1$-$C_6$)-Alkyl bedeutet und R12 wie oben definiert ist, steht,

X für -NH-OH steht,
n1 für die ganze Zahl 2 steht,
n2 für die ganze Zahl 3 steht, und
R1, R2, R3, R4 und R5 jeweils für Wasserstoffatom stehen.

Ein weiterer Gegenstand der Erfindung ist die Verbindung der Formel I aus der Reihe
2-(4'-Nitro-biphenyl-4-sulfonyl)-decahydro-isochinolin-1-(N-hydroxy)-carboxamid,
2-(4'-Chlor-biphenyl-4-sulfonyl)-decahydro-isochinolin-1-carbonsäure,
2-(4'-Chlor-biphenyl-4-sulfonyl)-decahydro-isochinolin-1-(N-hydroxy)-carboxamid,
2-(6-Phenoxy-pyridin-3-sulfonyl)-decahydro-isochinolin-1- carbonsäure; Trifluoracetat,
2-(6-Phenoxy-pyridin-3-sulfonyl)-decahydro-isochinolin-1-(N-hydroxy)-carboxamid; Trifluoracetat,
2-[2-(4'-Chlor-biphenyl-4-yl)-ethanesulfonyl]-decahydro-isochinolin-1- carbonsäure,
2-[2-(4'-Chlor-biphenyl-4-yl)-ethanesulfonyl]-decahydro-isochinolin-1-(N-hydroxy)-carboxamid,
2-[4-(Pyridin-4-yloxy)-benzensulfonyl]-decahydro-isochinolin-1- carbonsäure; Trifluoracetat,
2-[4-(Pyridin-4-yloxy)-benzensulfonyl]-decahydro-isochinolin-1-(N-hydroxy)-carboxamid; Trifluoracetat,
2-[4-(4-Methoxy-phenoxy)-benzenesulfonyl]-decahydro-isochinolin-1- carbonsäure,
2-[4-(4-Methoxy-phenoxy)-benzenesulfonyl]-decahydro-isochinolin-1-(N-hydroxy)-carboxamid,
2-{4-[4-(2,2,2-Trifluor-ethoxy)-phenoxy]-benzensulfonyl}-decahydro-isochinolin-1-carbonsäure,
2-{4-[4-(2,2,2-Trifluor-ethoxy)-phenoxy]-benzensulfonyl}-decahydro-isochinolin-1-(N-hydroxy)-carboxamid,
2-[4'-(2,2,2-Trifluor-ethoxy)-biphenyl-4-sulfonyl]-decahydro-isochinolin-1-carbonsäure,
2-(4'-Isopropoxycarbonylamino-biphenyl-4-sulfonyl)-decahydro-isochinolin-1- carbonsäure, [4'-(1-Hydroxycarbamoyl-octahydro-isochinolin-2-sulfonyl)-biphenyl-4-yl]-carboxamid isopropyl ester,
2-[4'-(2,2,2-Trifluor-ethoxy)-biphenyl-4-sulfonyl]-decahydro-isochinolin-1-carbonsäure hydroxyamid,
2-(4'-Trifluormethoxy-biphenyl-4-sulfonyl)-decahydro-isochinolin-1-carbonsäure hydroxyamid,
2-[4-(4-Fluor-phenoxy)-benzenesulfonyl]-decahydro-isochinolin-1-carbonsäure hydroxyamid,
2-[4-(4-Trifluormethoxy-phenoxy)-benzenesulfonyl]-decahydro-isochinolin-1-carbonsäure hydroxyamid,
2-[4-(4-Trifluormethoxy-phenoxy)-benzenesulfonyl]-decahydro-isochinolin-1- carbonsäure,
2-(Biphenyl-4-sulfonyl)-decahydro-isochinolin-1-carbonsäure hydroxyamid,
2-(Biphenyl-4-sulfonyl)-decahydro-isochinolin-1-carbonsäure,
2-[4-(4-Cyan-phenoxy)-benzensulfonyl]-decahydro-isochinolin-1-carbonsäure hydroxyamid,

2-(Dibenzofuran-2-sulfonyl)-decahydro-isochinolin-1-carbonsäure,
2-(Dibenzofuran-2-sulfonyl)-decahydro-isochinolin-1-carbonsäure hydroxyamid oder
2-[4-(4-Fluor-phenoxy)-benzenesulfonyl]-6-methoxy-decahydro-isochinolin-1-carbonsäure hydroxyamid, sowie alle isomeren Formen der oben genannten Verbindungen.

Unter dem Begriff "($C_1$-$C_6$)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, IsoPentyl, Neopentyl, Hexyl, 2,3-Dimethylbutan oder Neohexyl.

Unter dem Begriff "-($C_0$-$C_4$)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-$C_0$-Alkylen" ist eine kovalente Bindung.

Unter dem Begriff "$-(CH_2)_n-$, worin n die ganze Zahl Null, 1 oder 2 bedeutet" wird für n gleich Null eine kovalente Bindung, n gleich 1 der Rest Methylen und n gleich 2 der Rest Ethylen verstanden.

Unter dem Begriff "-($C_2$-$C_4$)-Alkenylen," werden Kohlenwasserstoffreste verstanden, deren Kohlenstoffkette geradkettig oder verzweigt ist und 2 bis 4 Kohlenstoffatome enthält und je nach Kettenlänge 1 oder 2 Doppelbindungen aufweisen, beispielsweise Ethenylen, Propenylen, Iso-Propenylen, Iso-Butenylen oder Butenylen; die Substituenten an der Doppelbindung können, sofern die prinzipielle Möglichkeit besteht, E- oder Z-ständig angeordnet sein.

Unter dem Begriff "-($C_2$-$C_6$)-Alkinylen," werden Kohlenwasserstoffreste verstanden, deren Kohlenstoffkette geradkettig oder verzweigt ist und 2 bis 6 Kohlenstoffatome enthält und je nach Kettenlänge 1 oder 2 Dreifachbindungen aufweisen, beispielsweise Ethinylen, Propenylen, Iso-Propinylen, Iso-Butinyl, Butinylen, Pentinylen oder Isomere von Pentinylen oder Hexinylen oder Isomere von Hexinylen.

Unter dem Begriff "($C_3$-$C_6$)-Cycloalkyl" werden Reste verstanden wie Verbindungen, die sich von 3- bis 6-gliedrige Monocyclen wie Cyclopropyl, Cyclobutyl, Cyclopentyl oder Cyclohexyl herleiten.

Unter den Resten

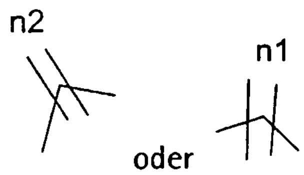

werden jeweils $-CH_2$-Reste im Ring der Formel I verstanden, wobei die Variablen n1 oder n2 jeweils die Anzahl der $-CH_2$-Reste im Ring der Formel I angeben. Für den Fall, dass n1 den Wert Null hat, ergibt sich eine kovalente Bindung und der erhaltene Teilring hat insgesamt 4 Ringatome. Für den Fall, dass n2 den Wert Null hat, ergibt sich eine kovalente Bindung und der erhaltene Teilring hat insgesamt 3 Ringatome. Für den Fall, dass n1 den Wert 1 hat, ergibt sich ein $-CH_2$-Rest und der erhaltene Teilring hat insgesamt 5 Ringatome. Für den Fall, dass n1 den Wert 2 hat, ergibt sich ein $-CH_2-CH_2$-Rest und der erhaltene Teilring hat insgesamt 6 Ringatome. Für den Fall, dass n1 den Wert 3 hat, ergibt sich ein $-CH_2-CH_2-CH_2$-Rest und der erhaltene Teilring hat insgesamt 7 Ringatome. Für n2 ergeben sich entsprechende Teilringe.

Unter dem Begriff "-($C_6$-$C_{14}$)-Aryl" werden aromatische Kohlenwasserstoffreste verstanden mit 6 bis 14 Kohlenstoffatomen im Ring. -($C_6$-$C_{14}$)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.

Unter dem Begriff "4- bis 15-gliedriger Het-Ring" oder "Het-Ring" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, 2'-Methyl-biphenyl-2-ol, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl,

6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,4-Triazolyl, 1,2,5-Triazolyl, 1,3,4-Triazolyl und Xanthenyl.

Bevorzugte Het-Ringe sind die Reste Benzofuranyl, Benzimidazolyl, Benzoxazolyl, Benzothiazolyl, Benzothiophenyl, 1,3-Benzodioxolyl, Chinazolinyl, Chinolinyl, Chinoxalinyl, Chromanyl, Cinnolinyl, Furanyl wie 2-Furanyl und 3-Furanyl; Imidazolyl, Indolyl, Indazolyl, Isochinolinyl, Isochromanyl, Isoindolyl, Isothiazolyl, Isoxazolyl, 2'-Methyl-biphenyl-2-ol, Oxazolyl, Phthalazinyl, Pteridinyl, Pyrazinyl, Pyrazolyl, Pyridazinyl, Pyridoimidazolyl, Pyridopyridinyl, Pyridopyrimidinyl, Pyridyl; wie 2-Pyridyl, 3-Pyridyl oder 4-Pyridyl; Pyrimidinyl, Pyrrolyl; wie 2-Pyrrolyl und 3-Pyrrolyl; Purinyl, Thiazolyl, Tetrazolyl oder Thienyl; wie 2-Thienyl und 3-Thienyl.

Unter dem Begriff "Halogen" wird Fluor, Chlor, Brom oder Jod verstanden.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man

a) eine Verbindung der Formel IV,

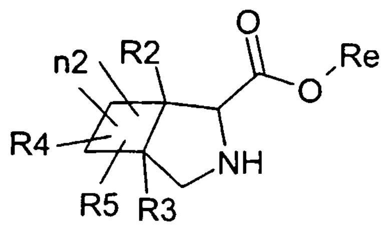

**(IV)**

worin Re ein Wasserstoffatom oder eine Ester-Schutzgruppe darstellt, mit einer Verbindung der Formel V,

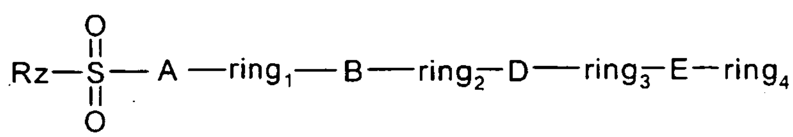

(V)

worin A, B, D, E und ring1, ring2, ring3, ring4 wie in Formel I definiert sind, und worin Rz Chloratom, Imidazoyl oder OH bedeutet,
in Gegenwart einer Base oder nach Silylierung mit einem geeigneten Silylierungsmittel oder mit einem geeigneten wasserentziehenden Mittel für den Fall Rz = OH zu einer Verbindung der Formel VI umsetzt,

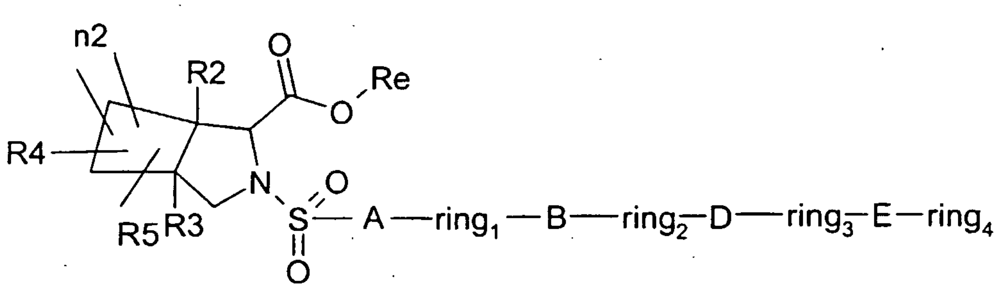

**(VI)**

worin A, B, D, E, Re und ring1, ring2, ring3 und ring4 wie oben definiert sind, und

b) für den Fall Re = Ester eine nach a) hergestellte Verbindung der Formel VI mit einer Alkalilauge wie NaOH oder LiOH und anschließender Säurebehandlung zu der erfindungsgemäßen Carbonsäure der Formel I, worin X = OH (entspricht VII) ist, umsetzt, wobei gegebenenfalls vorher noch Modifikationen in einer der Seitenketten der Ringe ring1-ring4 vorgenommen wurden; oder den genannten Ester durch Behandlung mit Mineralsäure wie Salzsäure zur freien Carbonsäure VII umsetzt

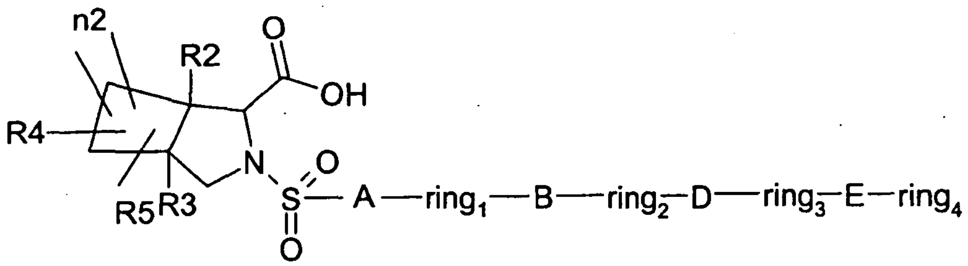

(VII)

und anschließend diese in die erfindungsgemäße Hydroxamsäure, worin X = NH-OH ist, der Formel I umwandelt,

c) eine nach Verfahren a) hergestellte Verbindung der Formel I, oder eine geeignete Vorstufe der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formel I entweder in freier Form isoliert oder im Falle des Vorliegens von sauren oder basischen Gruppen in physiologisch verträgliche Salze umwandelt.

Verbindungen der Art der Formel IV bis VII stellen nur beispielhafte Verbindungen dar, anstatt des Fünfringes können entsprechend der Formel I auch Vierringe, Sechsringe und Siebenringe aufgeführt werden.

Beispielsweise können die bicyclischen Grundgerüste der Formel IV mit $n_1$ = 2 und $n_2$ = 3 nach Formel I durch Hydrierung der Isochinolin-1-carbonsäure oder geeigneter Derivate der Isochinolin-1-carbonsäure, wie der Methyl- oder Ethylester, hergestellt werden. Diese Hydrierung ist beispielsweise in US 5430023, US 5726159 und EP 643073 beschrieben.

Ebenso ist es möglich, zur Herstellung dieser Verbindungen durch Hydrierung 1,2,3,4-Tetrahydroisochinolin-1-carbonsäure und deren Derivate einzusetzen. Dieses Verfahren hat den Vorteil, dass es möglich ist, eine breite Palette an Verfahren zur Synthese der 1,2,3,4-Tetrahydroisochinolin-1-carbonsäuren einzusetzen. Besonders bekannt und breit anwendbar sind beispielsweise Pictet-Spengler-artige Cyclisierungen, wie z. B. in US 4902695 beschrieben. Über derartige Verfahren können beispielsweise - je nach Art der eingesetzten Ausgangsprodukte-substituierte Verbindungen erhalten werden, d. h. Verbindungen, in denen die Substituenten R1, R4 und R5 nicht H-Atome bedeuten. Ein neues Beispiel für ringsubstituierte Verbindungen findet sich in WO 2003041641. Zahllose Beispiele finden sich für R1 und/oder R4 und R5 ungleich H und sind dem Fachmann leicht zugänglich.

Weitere Methoden zur Herstellung der cyclischen Grundgerüste sind beispielsweise über radikalische Cyclisierungsreaktionen möglich und in Tetrahedron 48, 4659-76 (1992) beschrieben.

Als Ester-Schutzgruppe Re können die in "Protective Groups in Organic Synthesis", T.H. Greene, P. G. M. Wuts, Wiley-Interscience, 1999, als Schutzgruppen für Ester verwendete Gruppen eingesetzt werden. Bevorzugte Ester-Schutzgruppen sind beispielsweise Methyl, Ethyl, Isopropyl, tert. Butyl oder Benzyl.

Unter bestimmten Bedingungen kann es sinnvoll sein, Verbindungen des Typs IV in N-geschütztem Zustand einzusetzen. Beispielsweise können derart geschützte Verbindungen besser aufgereinigt werden als die freien Iminosäuren, ebenso können diese u. U. auch besser zur Herstellung der enantiomeren- oder diastereomerenreinen Verbindungen eingesetzt werden. Als Schutzgruppen der Aminogruppe können die in "Protective Groups in Organic Synthesis", T.H. Greene, P. G. M. Wuts, Wiley-Interscience, 1999, beschriebenen Gruppen eingesetzt werden. Bevorzugte Amino- oder imino-Schutzgruppen sind beispielsweise Z, Boc, Fmoc, Aloc, Acetyl, Trifluoracetyl, Benzoyl, Benzyl und ähnliche.

Die eingesetzten Ausgangsprodukte und Reagenzien können entweder nach bekannten Verfahren hergestellt werden oder sind käuflich erhältlich.

Die Umsetzungen erfolgen beispielsweise wie in WO 97/18194 dargestellt. Die Umsetzung gemäß Verfahrensschritt a) erfolgt in Gegenwart einer Base wie KOH, NaOH, LiOH, N-Methylmorpholin (NMM), N-Ethylmorpholin (NEM), Triethylamin (TEA), Diisopropylethylamin (DIPEA), Pyridin, Collidin, Imidazol oder Natriumcarbonat, in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser. Für den Fall, dass die Umsetzung unter Verwendung von Silylierungsmitteln durchgeführt wird, setzt man beispielsweise N,0-Bis-(trimethylsilyl)acetamid (BSA) oder N,O-Bis-(trimethylsilyl)trifluoracetamid (BSTFA) zur Silylierung der Iminosäure ein, um anschließend die Sulfonamidbildung durchzuführen.

Modifikationen in der Seitenkette F bedeutet, dass beispielsweise eine Nitrogruppe mit dem Metallkatalysator Pd/C hydriert oder mit $SnCl_2$ bzw. Zn unter Standardbedingungen umgesetzt wird und die erhaltene Aminogruppe anschließend weiter modifiziert werden kann, beispielsweise durch Umsetzung mit Carbonsäurechloriden, Sulfonsäurechloriden, Chlorameisensäureestern, Isocyanaten, Isothiocyanaten oder anderen reaktiven oder aktivierbaren Reagenzien, um zu den Vorstufen der erfindungsgemäßen Verbindungen der Formel I zu gelangen. Für diesen Fall ist es oft günstig, dass Re in Verbindung VI ein Ester ist, da im Falle der ungeschützten Carbonsäure mit Nebenreaktionen zu rechnen ist.

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können ggf. auch andere oder vereinfachte Verfahren zur Aufreinigung der Endprodukte eingesetzt werden. Diese Ausgangsprodukte wurden zuvor nach Literaturbekannten Verfahren enantiomeren- oder diastereomerenrein hergestellt. Beispielsweise kann in dem Verfahren zur Herstellung der Decahydroisochinolin-1-carbonsäure entweder direkt die Isochinolin-1-carbonsäure eingesetzt werden, wie oben angeführt und zitiert. Dadurch, dass 3 Stereozentren vorhanden sind, können in diesem Fall maximal 8 Stereoisomere (4 enantiomere Diasteromerenpaare) gebildet werden. Bedingt durch die Art der Herstellung, beispielsweise Hydrierung, sind jedoch bestimmte Stereoisomere stark bevorzugt. So sollte es möglich sein, wie in der Literatur beschrieben, durch geeignete Wahl der Hydrierungsbedingungen (Katalysator, Druck, Lösemittel, Temperatur) z. B. eine starke Bevorzugung der Wasserstoff-Anlagerung an den Positionen der Ringverknüpfung zu erzielen. So kann unter den angegebenen Bedingungen die Bildung der cis-verknüpften Ringe erreicht werden. Somit bliebe dann noch die Position der Carbonsäure zu bestimmen, die Anzahl der möglichen Stereoisomeren wäre bereits auf 4 eingeschränkt. Bedingt durch die Natur des Hydriermechanismus kann besonders leicht eine Anlagerung der Wasserstoffe auf der gleichen Seite wie die der Brückenkopf-Wasserstoffe erfolgen, d. h. hiermit ist eine weitere Einschränkung der Möglichkeit der Isomerenbildung zu erwarten. Somit könnte im günstigsten Fall von der Bildung nur eines Enantiomerenpaares ausgegangen werden. Dieses sollte anschließend durch die oben genannten Methoden in die Enantiomeren auftrennbar sein. Allerdings muss bei diesen Überlegungen auch davon ausgegangen werden, dass niemals eine völlige Stereoselektion stattfindet, sondern dass praktisch immer auch zu mehr oder minder großen Anteilen die anderen Isomeren entstehen bzw. durch geeignete Methoden auch in kleinsten Mengen nachgewiesen werden können.

Für den Fall, dass enantiomerenreine 1,2,3,4-Tetrahydroisochinolin-1-carbonsäure-Derivate eingesetzt werden, wäre zu erwarten, dass bei gleichen oder ähnlichen Reaktionsbedingungen wie bei der Hydrierung der Isochinolin-1-carbonsäure analoge Überlegungen Gültigkeit besitzen und zu großen Anteilen wieder nur bevorzugte Stereoisomere gebildet werden; in genannten Fall sollte eine starke Bevorzugung eines einzigen Enantiomeren stattfinden, da bei dem Hydrierprozess unter analogen Bedingungen, die zur cis-Ringverknüpfung bei der Hydrierung der Isochinolin-1-carbonsäure führen, hier ebenso wieder nur Anlagerung der H-Atome von einer Seite erfolgen kann und somit analoge Produkte gebildet werden. Durch geeignete 2D-NMR-Experimente, X-Ray-Verfahren wie etwa der Kokristallisation oder andere, sowie Vergleichsanalytik oder chemische Derivatisierung und geeignete Analytik oder chemische Derivatisierung , die

zu bekannten und beschriebenen Isomeren führt, kann die Identität der Strukturen festgestellt werden.

Eine andere Möglichkeit zur Synthese enantiomeren- oder diastereomerenreiner Verbindungen besteht darin, geeignet chiral substituierte Ausgangsstoffe einzusetzen, um durch den chiralen Substituenten eine Induktion von Chiralität an anderen Chiralitätszentren zu erreichen. Beispielsweise könnten chirale Glyoxylsäureester in Pictet-Spengler-Cyclisierungen eingesetzt werden, um chirale Tic-Derivate zu erhalten und diese dann, wie oben bereits erwähnt, zu hydrieren.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren. Die Herstellung physiologisch verträglicher Salze aus zur Salzbildung befähigten Verbindungen der Formel I, einschließlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder Hilsstoff.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinasen beteiligt sind. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie, Herzversagen und septischem Schock. Ebenso eignen sich die Verbindungen zur Prophylaxe von Myocard- und Cerebral-Infarkten.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen. Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder

aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) und $^1$H-NMR (400 MHz, in DMSO-D6)bestimmt, angegeben sind jeweils der Hauptpeak oder die beiden Hauptpeaks. Temperaturangaben in Grad Celsius, RT bedeutet Raumtemperatur (21 ˚C bis 24 ˚C). Verwendete Abkürzungen sind entweder erläutert oder entsprechen den üblichen Konventionen. Nachfolgend ist die Erfindung an Hand von Beispielen näher erläutert.

Allgemeine Vorschrift 1: Sulfonamid aus Sulfonsäurechlorid und Carbonsäure

Die Carbonsäure (6,45 mmol) wurde in 20 ml Dimethylformamid (DMF) gelöst und bei 0 ˚C mit 3 Äquivalenten einer 3N NaOH-Lösung (6,45 ml) versetzt. Nach 10 min tropfte man eine Lösung des Arylsulfonylchlorids (1,1 Äquivalente, 7,1 mmol) in 10 bis 15 ml DMF langsam zu, nach dem Erreichen der Raumtemperatur (RT) wird der Ansatz noch für maximal 12 Stunden (h) bei Temperaturen zwischen 20 ˚C und 80 ˚C gerührt. Die genaue Zeit ergibt sich je nach erfolgtem Umsatz, der massenspektroskopisch festgestellt wurde. Danach wurde das Lösungsmittel unter verminderten Druck entfernt. Anschließend erfolgte wässrige Aufarbeitung (Ausschütteln mit 1N HCl und gesättigter NaCl-Lösung, Trocknen der organischen Phase wie Essigester, Methylenchlorid oder Chloroform mit Magnesium- oder Natriumsulfat, danach Einengen). Das Rohprodukt wurde entweder direkt weiter umgesetzt oder chromatographisch gereinigt.

Allgemeine Vorschrift 2: Sulfonamid aus Sulfonsäurechlorid und Carbonsäure Die Carbonsäure wurde in 0,5-2 molarer NaOH gelöst, eventuell unter Zugabe von 10-50 % Tetrahydrofuran (THF) oder DMF. Säurechlorid (1-1,2 Äquivalente, bevorzugt 1,1) wurde in THF gelöst (Konzentration 0,05 bis 1 M) und langsam zugetropft. Am Autotitrator erfolgte automatisch Zugabe von 2 N NaOH bei RT zur pH-Konstanthaltung. Eingestellter pH-Wert: 8 bis 12, bevorzugt 9 bis 11. Nach Beendigung der Reaktion, erkennbar an keinem weiteren NaOH-Verbrauch, wurde das organische Co-Lösungsmittel am Rotationsverdampfer entfernt, die wässrige Lösung oder Suspension mit Essigester versetzt und mit 1 N HCl angesäuert. Nach Abtrennung der organischen Phase und erneuter Extraktion der wässrigen Phase mit Essigester wurden die organischen Phasen vereinigt, über Natriumsulfat getrocknet und anschließend das Lösemittel unter verminderten Druck entfernt. Das Rohprodukt wurde entweder direkt weiter umgesetzt oder chromatographisch gereinigt.

Allgemeine Vorschrift 3: Sulfonamid aus Sulfonsäurechlorid und Carbonsäure. Diese Vorschrift ist besonders geeignet zur Umsetzung von Biphenylethylsulfonsäurechlorid mit Iminocarbonsäuren (siehe Beispiel 6 und Beispiel 7) oder ähnlichen, hydrolyselabileren Sulfonsäurechloriden.

8 mmol der Iminosäure wurden in 30 ml Acetonitril gelöst oder suspendiert. Bei RT und unter Inertgas ($N_2$) wurden 2,3 g (9 mmol) BSTFA (Bis-(trimethylsilyl)-trifluoracetamid) zugegeben und die Mischung für 2 h unter Rückfluss erhitzt. Zu dieser Lösung gab man 2,84 g (9 mmol) 4-Chlorbiphenylethansulfonylchlorid, gelöst in 30 ml Acetonitril und erhitzte unter Rückflussbedingungen erneut für 3 h. Nach Abkühlen der Reaktionsmischung gab man wässrige 1 N HCl zu, rührte für 1 h, entfernte das Lösemittel unter verminderten Druck am Rotationsverdampfer und gab anschließend Essigsäurethylester oder Chloroform hinzu, trennte die organische Phase ab, extrahierte diese mit gesättigter NaCl-Lösung, trocknete über Natriumsulfat und engte unter verminderten Druck ein. Je nach Reinheit des Reaktionsproduktes konnte dieses direkt weiter umgesetzt werden oder musste vorher über Kieselgel chromatographiert werden.

Allgemeine Vorschrift 4: Herstellung der Hydroxamsäure aus Carbonsäure über Chloroformat-Aktivierung

Die sulfonierte Carbonsäure wurde in 10 ml DMF gelöst und bei 0 ˚C mit 1,1 Äquivalenten Ethylchloroformiat, 2,2 Äquivalenten N-Ethylmorpholin sowie - nach einer Voraktivierungszeit von 30 min bis 1 h - mit 3 Äquivalenten Trimethylsilylhydroxylamin versetzt. Nachdem man für mindestens 4 h auf 80 ˚C erhitzte hat, entfernte man das Lösungsmittel unter verminderten Druck und reinigte das Rohprodukt mit chromatographischen Methoden.

Allgemeine Vorschrift 5: Herstellung der Hydroxamsäure über das korrespondierende Carbonsäurechlorid

Die sulfonierte Carbonsäure wurde in trockenem Chloroform (Ethanol-frei) vorgelegt (etwa 5 ml für 0,5 mmol) und bei RT mit 3 Äquivalenten Oxalylchlorid versetzt. Anschließend wurde für etwa 30 min auf 45 ˚C erwärmt. Zur Kontrolle der Chloridbildung wurde eine kleine Probe aus dem Reaktionskolben herausgenommen und mit wenig Benzylamin in THF versetzt. Die vollständige Umsetzung konnte an der quantitativen Benzylamid-Bildung erkannt werden, die Carbonsäure war nicht mehr nachweisbar (Kontrolle durch HPLC-MS). Gegebenenfalls muss für längere Zeit erwärmt werden oder unter Rückflussbedingungen erhitzt werden. Anschließend wurde das Lösemittel unter verminderten Druck abdestilliert, der Rückstand wurde mehrfach in trockenem Toluol aufgenommen und erneut einrotiert. Das Säurechlorid wurde nun erneut in Chloroform (10 ml pro 0,5 mmol) aufgenommen und bei RT mit 3 Äquivalenten 0-Trimethyl-silylhydroxylamin versetzt. Nach einer Reaktionszeit von mindestens 30 min (Reaktionskontrolle per HPLC-MS) wurde die Reaktionsmischung unter verminderten Druck eingedampft und der Rückstand direkt chromatographisch gereinigt.

Spezielle Vorschriften

4-Chloro-biphenylethansulfonylchlorid (Zwischenprodukt für Beispiel 7)

Schritt 1: 1-(2-Bromoethenon)-4-(4-chlorophenyl)-benzol

Zu einer gerührten Suspension von $AlCl_3$ (34,7 g, 0,26 mol) und Bromoacetylbromid (25,2 g, 0,125 mol) in 400

mL $CS_2$ wurde 4-Chlorobiphenyl (23,6 g, 0,125 mol) portionsweise bei 0˚ C eingetragen und dann unter Rückfluss für 3 h erhitzt. Die Reaktionsmischung wurde anschließend langsam auf Eis gegossen, mit Essigsäureethylester extrahiert und die organische Phase mit wässriger $NaHCO_3$-Lösung und Wasser gewaschen. Anschließend wurde über wasserfreiem Natriumsulfat getrocknet und unter verminderten Druck eingedampft. Der verbleibende Rückstand wurde aus Dichlormethan umkristallisiert.

Ausbeute: 24,2 g (62 % der Theorie). Fp: 127 - 128 ˚C
$^1$H-NMR: (300 MHz) 5.0 (s, 2 H, $CH_2$); 7.5-8.1 (4 d, 8H, ar) MS: 311,1 (M+H)

Schritt 2: 4-Chlorbiphenylethanbromid

Zu einer gerührten Suspension von $AlCl_3$ (20,0 g, 0,15 mol) in Dichloromethan (500 mL) wurde tert-Butylamineboran (27,5 g, 0,31 mol) bei 0˚C zugegeben. Nachdem die Mischung bei 0˚ C für 15 min gerührt wurde, wurde eine Lösung des Bromketons aus Schritt 1 (16,0 g, 50 mmol) in Dichloromethan (150 mL) zugegeben und bei 0 ˚C für weitere 4 h gerührt. Kalte verdünnte HCl (1N, 30 mL) wurde tropfenweise zugegeben, anschließend wurde mit Essigsäureethylester mehrfach extrahiert. Die vereinigten organischen Phasen wurden zuerst mit verdünnter HCl gewaschen, anschließend mit gesättigter Kochsalzlösung und eingedampft. Man erhielt eine ölige Verbindung, die über Flash-Chromatographie an Kieselgel gereinigt wurde.

| | |
|---|---|
| Ausbeute: 15 g (quantitativ). Fp: | 142 ˚C |
| $^1$H-NMR: | (300 M Hz) 3.2; 3.78 (2 t, 4 H, $CH_2$); 7.4-7.7 (4 d, 8H, ar) |
| MS: | 296,2 (M+H) |

Schritt 3: Natriumsalz der 4-Chlorbiphenylethansulfonsäure

Die Verbindung aus Schritt 2 (14,8 g, 50 mmol) wurde in einer Mischung aus Ethanol und Wasser gelöst (1: 1, 200 mL). Man gab Natriumsulfit (9.5 g, 75 mmol) und Tetrabutylammoniumjodid (1.8 g, 5 mmol) hinzu und erhitzte die Mischung unter Rückfluss für 16 h. Anschließend wurde von einer kleinen Menge eines Feststoffes abdekantiert und das Volumen der Reaktions-mischung wurde durch teilweises Eindampfen unter verminderten Druck reduziert. Unter Kühlung kristallisierte das Produkt, das abfiltriert und aus $MeOH/H_2O$ umkristallisiert wurde. Anschließend wurde es unter verminderten Druck getrocknet. Ausbeute: 13,9 g (94 % der Theorie).

$^1$H-NMR: (300 MHz) 2.6; 2.95 (2 m, 4 H, $CH_2$); 7.3-7.7 (4 d, 8H, ar)

Schritt 4: 4-Chlorobiphenylethansulfonylchlorid

Zu einer Suspension der Verbindung aus Schritt 3 (4,8 g, 15 mmol) in Phosphoroxychlorid (50 ml) wurde Phosphorpentachlorid (3.2 g, 15 mmol) zugegeben. Man erwärmte auf 60 ˚C für 6 h und goss die Mischung auf Eis, nachdem Methylenchlrorid hinzugegeben wurde. Es wurde mit gesättigter Natriumhydrogencarbonat-Lösung neutralisiert, die organische Phase wurde abgetrennt, getrocknet und unter verminderten Druck eingedampft.
Ausbeute: 5 g (quantitativ) $^1$H-NMR: (300 MHz) 2.9 (m, 4 H, $CH_2$); 7.3-7.7 (4 d, 8H, ar)

Trennung der Enantiomere von Herstellungsbeispiel 3 durch chirale

Hochdruckflüssigchromato-graphie (HPLC):

Verwendete Säule: Chiralpak AD$^R$, 250 x 4.6 mm, 30 ˚C, Laufzeit: 30 min, Einspritzvolumen: 5 μl, Flussrate: 1 ml/min, Lösungsmittel MeOH/EtOH 1:1 isokratisch, Detektion bei 277 nm. Isomer 1 (Beispiel 14): Laufzeit (RT) 6,33 min, 50,48 % and Isomer 2 (Beispiel 15): RT 14,65 min, 49,52 %.

Die Strukturbestimmung von Isomer 1 erfolgte durch 2-dimensionale-NMR-Spektroskopie. Die Methodik erlaubt nur die Bestimmung der relativen Stereochemie, d. h. die Stellung der Chiralitätszentren zueinander. Das heißt, daß bei Vorliegen nur eines einzigen Enantiomeren, wie dies nach einer Enantimerentrennung zu erwarten ist, dieses entweder die angegebene oder aber spiegelbildliche absolute Stereochemie haben kann. Der letztendliche Strukturbeweis kann nur durch Röntgenstrukturanalysen durchgeführt werden. Dies wurde sowohl im Sinne einer Kokristallisation mit MMP-13 wie auch im Sinne ein Einkristall-Strukturanalyse durchgeführt. Beide Methoden zeigten eindeutig, dass die angegebene Stereochemie der tatsächlichen Stereochemie entspricht.

Chirale Trennungen der anderen Verbindungen, die ebensolche Enantiomerenmischungen repräsentieren, da

ausgehend von den gleichen Decahydosiochinolinderivaten hergestellt, können ebenfalls per chiraler HPLC durchgeführt werden".

Die Ergebnisse zeigt Tabelle 1:

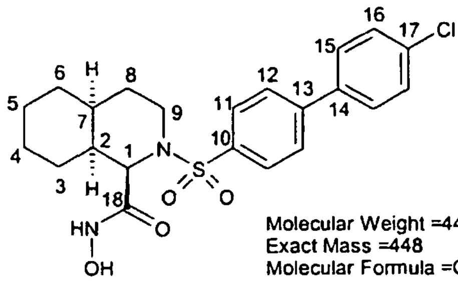

Molecular Weight =448,97
Exact Mass =448
Molecular Formula =C22H25ClN2O4S

Tabelle 1 : Chemischer Shift von Isomer 1 bei 300 K.

| | $^{1}H$ | $^{13}C$ |
|---|---|---|
| 1 | 4.12 | 55.15 |
| 2 | 1.93 | 35.68 |
| 3 | 1.67/1.41 | 26.37 |
| 4 | 1.19 | 21.03 |
| 5 | 1.48/1.16 | 24.26 |
| 6 | 1.86/1.12 | 26.06 |
| 7 | 1.52 | 32.00 |
| 8 | 1.55/1.45 | 28.88 |
| 9 | 3.75/3.53 | 38.95 |
| 10 | - | 138.99 |
| 11 | 7.81 | 127.29 |
| 12 | 7.87 | 127.26 |
| 13 | - | 142.45 |
| 14 | - | 137.19 |
| 15 | 7.78 | 128.81 |
| 16 | 7.57 | 129.05 |
| 17 | - | 133.42 |
| 18 | - | 166.96 |
| 18-NH | 10.86 | - |
| 18-NOH | 8.88 | - |

Herstellungsbeispiel 2: N-(4-Chlorbiphenylsulfonyl)-decahydroisochinolin-1-carbonsäure Decahydroisochinolincarbonsäure wurde wie in US 5,430,023 beschrieben hergestellt und eingesetzt. Man löste oder suspendierte die erhaltene Iminosäure (2,0 g, 9,1 mmol) in THF (20ml) und stellte am Autotitrator einen pH-Wert von 10,5 mit 1 molarer Natronlauge ein. Anschließend wurde 4-Chlorbiphenyl-sulfonylchlorid (2,745 g, 9,6 mmol, 1,05 eq.), gelöst in 10 ml THF, über einen Zeitraum von 2 Stunden unter pH-Konstanthaltung zugetropft. Nach weiteren 2 Stunden konnte kein weiterer Natronlauge-Verbrauch mehr festgestellt werden. Per LC-MS wurde eine Reaktionskontrolle durchgeführt und bestätigte dies. Anschließend wurde die Lösung mit verdünnter Salzsäure auf pH von 3 bis 4 eingestellt, mit 100 ml Ethylacetat versetzt und ausgeschüttelt. Die wässrige Phase wurde noch 2 Mal mit kleinen Portionen Ethylacetat extrahiert und die vereinigten organischen Phasen über Natriumsulfat getrocknet. Nach Entfernen des Lösemittels verblieb

ein öliger Rückstand, der im Ölpumpenvakuum fest wurde.
Ausbeute: 2,31 g (58 % der Theorie). Analytische Daten: siehe Tabelle 1.

Herstellungsbeispiel 3: N-(4-Chlorbiphenylsulfonyl)-decahydroisochinolin-1-(N-hydroxy)-carboxamid
Die Carbonsäure aus Beispiel 2 (2,3 g, 5,3 mmol) wurde in 50 ml Chloroform gelöst. Anschließend wurde Oxalylchlorid (1,345 g, 10,6 mmol, 0,924 ml) innerhalb von 10 min zugetropft und die erhaltene Reaktionsmischung für eine Stunde auf 45 ˚C erwärmt. Nach dieser Zeit wurde zur Reaktionskontrolle per HPLC-MS eine kleine Probe der Reaktionsmischung (0,1 ml) entnommen und mit 0,05 ml Benzylamin versetzt. Anschließend wurde das Lösemittel unter verminderten Druck abdestilliert und der erhaltene ölige Rückstand mit Toluol zur Entfernung von etwaigen Oxalylchlorid-Resten oder HCl geschleppt und unter verminderten Druck 15 min belassen. Dann wurde wiederum in Chloroform (50 ml) aufgenommen und bei RT mit O-Trimethylsilylhydroxylamin (2,23 g, 21,2 mmol, 2,593 ml) versetzt. Nach 2 Stunden wurde das Lösemittel unter verminderten Druck entfernt und der Rückstand in einer kleinen Menge einer Mischung aus Acetonitril-Wasser-0,01 % Trifluoressigsäure zur direkten präparativen RP-HPLC gelöst. Produktfraktionen wurden vereinigt, Acetonitril unter verminderten Druck entfernt und die verbleibende wässrige Phase gefriergetrocknet. Ausbeute: 749 mg (32 % der Theorie, daneben erhält man 36 mg eines weiteren Diastereomeren mit gleicher Molmasse). Nicht umgesetztes Säurechlorid konnte in Form der Carbonsäure reisoliert werden. Analytische Daten: siehe Tabelle 1.

Die nachfolgenden Beispiele wurden analog zu den vorher genannten Vorschriften hergestellt. Tabelle 2 zeigt die Ergebnisse.

Tabelle 2:

| Beispiel | Struktur | Molgewicht | ES+ | 1H-NMR |
|---|---|---|---|---|
| 1 | 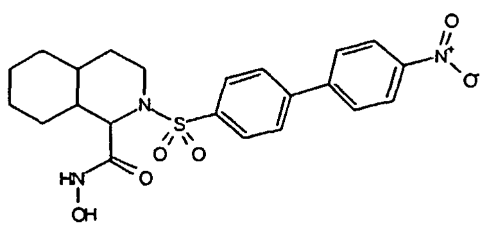 | 459,52 | 460,2<br>461,2 | 1,1-2,0 (4 m, 12 H); 3,5-3,9 (m, 2 H); 4,15 (d, 1 H); 7,88; 8,0; 8,07; 8,35 (4 d, 8 H); 10,9 (s, 1 H) |
| 2 | 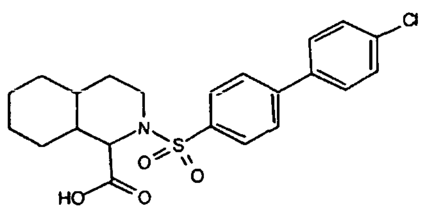 | 433,95 | 433,11 | 1,2-2,1 (4 m, 12 H); 3,4-3,6 (m, 2 H); 4,2 (d, 1 H); 7,60; 7,8; (2 d, 4 H); 7,9 (m, 4 H); 12,8 (s, 1 H) |
| 3 | 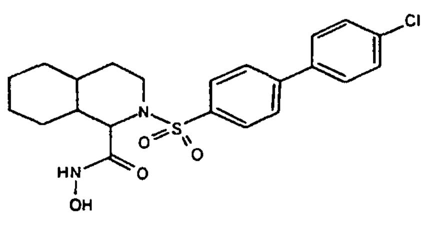 | 448,97 | 449,15<br>451,10 | 1,1-2,0 (4 m, 12 H); 3,5-3,9 (m, 2 H); 4,15 (d, 1 H); 7,60; 7,7; 7,8; 7,9 (4 d, 8 H); 10,9 (s, 1 H) |

(fortgesetzt)

| Beispiel | Struktur | Mol-gewicht | ES⁺ | 1H-NMR |
|---|---|---|---|---|
| 4 | 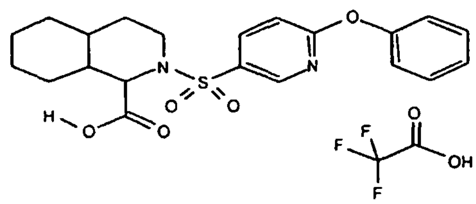 | 530, 52 | 415,1 6 (ES-) | |
| 5 | 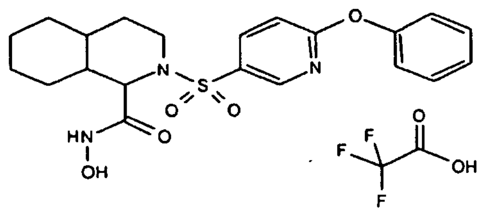 | 545,54 | ES-430,17 | 1,1-2,0 (4 m, 12 H); 3,4-3,8 (2m, 2 H); 4,08 (d, 1 H); 7,2 (m, 4 H); 7,45 (m, 2 H); 8,1 (dd, 1 H); 8,45 (s, 1 H); 10,8 (s, 1 H) |
| 6 | 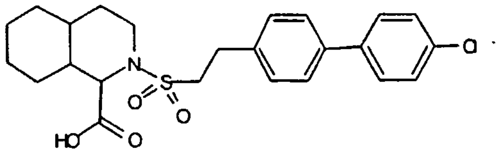 | 462,01 | 461,14 | 1,15-2,05 (4 m, 12 H); 2,9-3,5 (mm, etwa 6 H, überlappend mit Wasser); 3,85 (m, 1 H); 4,0 (d, 1 H); 7,48; 7,5; 7,61; 7,7 (4 d, 8 H) |
| 7 | 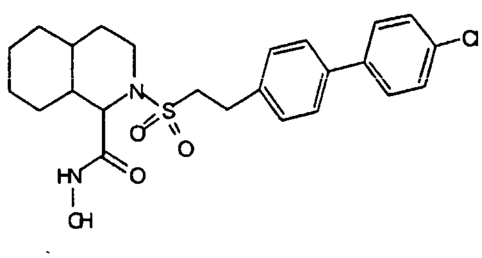 | 477,03 | 477,7 | 1,15-2,05 (m m, 12 H); 2,9-3,5 (mm, etwa 6 H, überlappend mit Wasser); 4,85 (m, 1 H); 7,48; 7,5; 7,65; 7,72 (4 d, 8 H) |

(fortgesetzt)

| Beispiel | Struktur | Mol-gewicht | ES+ | 1H-NMR |
|---|---|---|---|---|
| 8 | 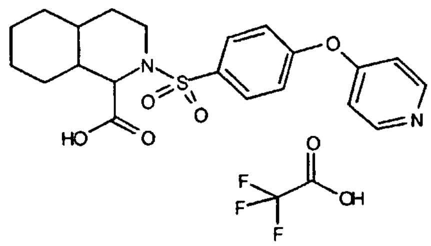 | 530,52 | 416,14 | 1,2-2,1 (4 m, 12 H); 2,9-4,3 (mm, etwa 3 H, überlappend mit Wasser); 7,3; 7,45; 7,95; 8,70 (4 m, 8 H); 12,8 (s, 1H) |
| 9 | 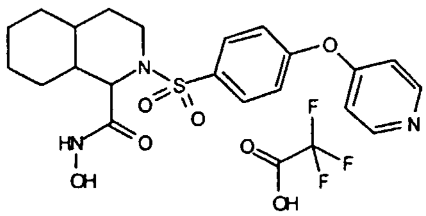 | 545,54 | 431,15 | 1,1-2,0 (4 m, 12 H); 3,55 (m, 1 H); 3,8 (m, 1 H); 4,1 (d, 1 H); 7,35; 7,45; 7,9; 8,70 (4 d, 8 H); 10,8 (s, 1H) |
| 10 | 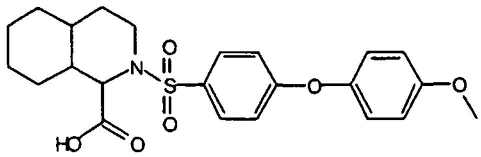 | 445,54 | 446,1<br>447,1 | |
| 11 | 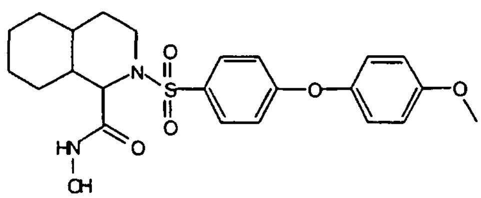 | 460,55 | 461,2<br>462,2 | 1,1-1,95 (4m, 12 H); 3,45 (m, 1 H); 3,7 (m, 1 H); 3,8 (s, 2 H); 4,05 (d, 1 H); 7,0 (m, 4 H); 7,1 (m, 2 H); 7,7 (m, 2 H); 10,8 (s, 1 H). |
| 12 | 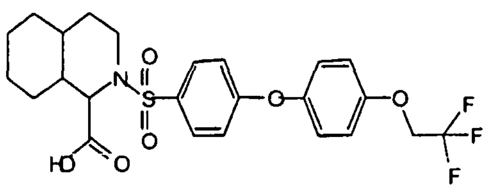 | 513,54 | 514,15<br>515,2 | |

(fortgesetzt)

| Beispiel | Struktur | Mol-gewicht | ES+ | 1H-NMR |
|---|---|---|---|---|
| 13 | 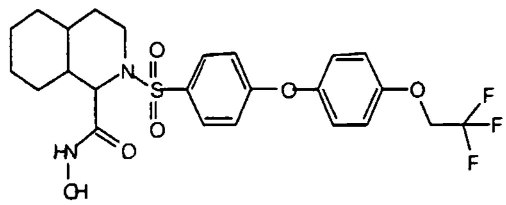 | 528,55 | 529,2<br>530,2 | 1,1-2,0 (4m, 12 H); 3,45 (m, 1 H); 3,7 (m, 1 H); 4,07 (d, 1 H); 7,0 (d, 2 H); 7,17 (s, 4 H); 7,7 (d, 2 H); 10,8 (s, 1 H). |
| 14 | Chemistry 16<br>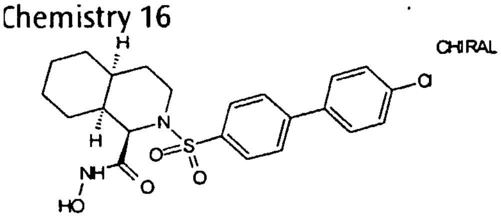 | 448,9726 | 449.15,<br>451.10 | (4 m, 12 H); 3,5-3,8 (2m, 2 H); 4,15 (d, 1 H); 7,55-7,9 (3 m, 8 H); 8,9 (s, 1 H); 10,9 (s, 1 H) |
| 15 | Chemistry 19<br>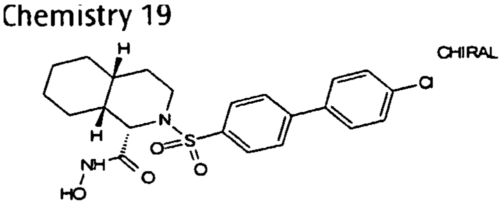 | 448,9726 | 449.15,<br>451.10 | 1,1-2,0 (4 m, 12 H); 3,5-3,8 (2m, 2 H); 4,15 (d, 1 H); 7,55-7,9 (3 m, 8 H); 8,9 (s, 1 H); 10,9 (s, 1 H) |
| 16 | Chemistry 35<br>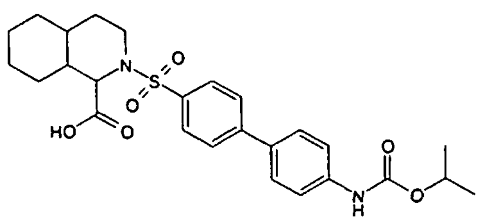 | 500,6187 | 501,18 | 1,25 (d, 6 H); 1,2-1,7 (m, 11 H); 2,05 (m, 1 H); 3,44; 4,20; 4,90 (3 m, 3-4 H); 7,6; 7,7 (dd, 4 H); 7,87 (m, 4 H); 9,8 (s, 1 H); 12,8 (s, 1 H) |
| 17 | Chemistry 36<br>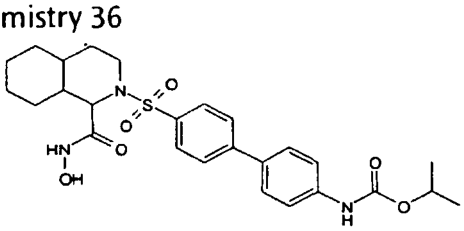 | 515,6334 | 516,19 | 1,25 (d, 6 H); 1,1-1,95 (m, 12 H); 3,55; 3,75; 4,12; 4,91 (4 m, 4 H); 7,6-7,88 (2 dd, 8 H) |

(fortgesetzt)

| Beispiel | Struktur | Mol-gewicht | ES+ | 1H-NMR |
|---|---|---|---|---|
| 18 | Chemistry 37<br>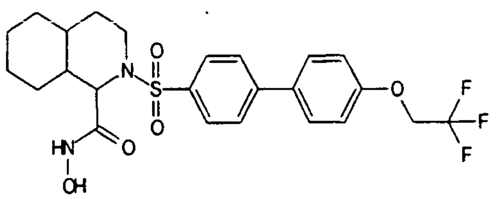 | 512,5524 | 513,13 | 1,1-2,0 (m, 12 H); 3,50; 3,75, 4,11; 4,83(4 m, 5 H); 7,2 (d, 2 H); 7,8 (m, 6 H); 8,9 (s, 1 H); 10,9 (s, 1 H). |
| 19 | Chemistry 38<br>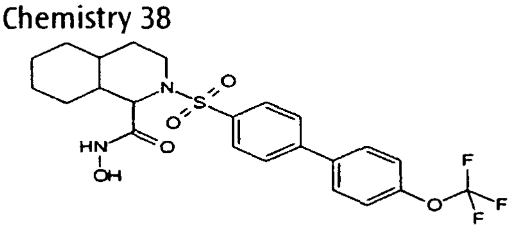 | 498,5253 | 450,08 | 1,1-2,0 (m, 12 H); 3,50; 3,75, 4,15 (3 m, 3 H); 7,5 (d, 2 H); 7,9 (m, 6 H); 8,9 (s, 1 H); 10,9 (s, 1 H). |
| 20 | Chemistry 39<br>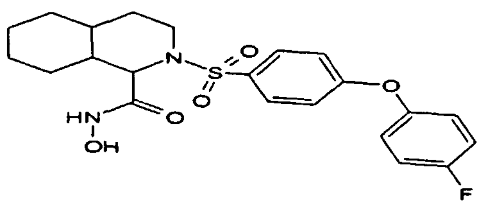 | 448,5174 | 449,24 | 1,1-2,0 (m, 12 H); 3,50; 3,75, 4,10 (3 m, 3 H); 7,0-7,7 (4 m. 8 H); 8,9 (s, 1 H); 10,9 (s, 1 H). |
| 21 | Chemistry 44<br>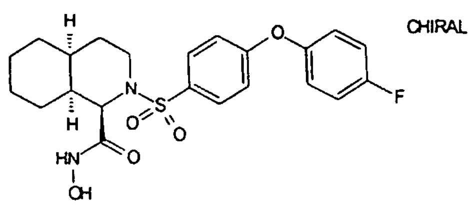 | 448,5174 | 449,21 | 1,1-2,0 (m, 12 H); 3,50; 3,75, 4,10 (3 m, 3 H); 7,0-7,7 (4 m. 8 H); 8,9 (s, 1 H); 10,9 (s, 1 H) |

(fortgesetzt)

| Beispiel | Struktur | Mol-gewicht | ES$^+$ | 1H-NMR |
|---|---|---|---|---|
| 22 | Chemistry 49 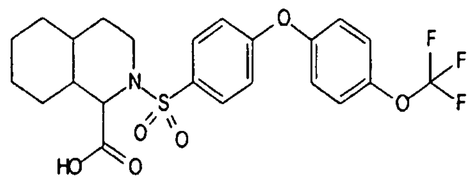 | 499,51 | 500,18 | 1,2-1,7 (m, 11 H); 2,05 (m, 1 H); 3,5 (m, +/- 2 H); 4,2 (d, 1 H); 7,15-7,80 (4 m ("d"), 8 H); 12,7 (s, 1 H) |
| 23 | Chemistry 48 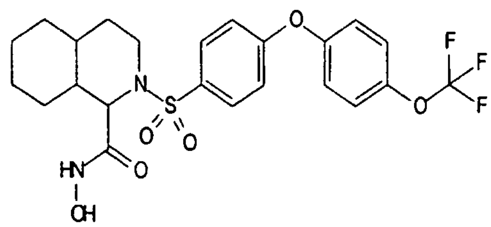 | 514,5247 | 515,21 | 1,1-2,0 (m, 12 H); 3,50; 3,75, 4,10 (3 m, 3 H); 7,1-7,8 (4 m ("d"), 8 H); 8,9 (s, 1 H); 10,9 (s, 1 H) |
| 24 | Chemistry 53 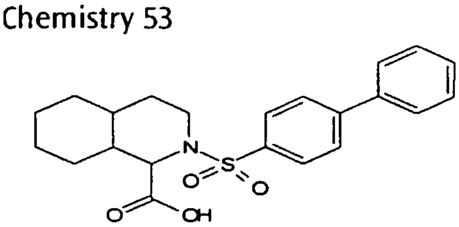 | 399,5129 | 400,24 | 1,1-2,0 (4 m, 12 H); 3,5- . 3,8 (2 m, 2 H); 4,15 (d, 1 H); 7,4-8,0 (m, 9 H) |
| 25 | Chemistry 52 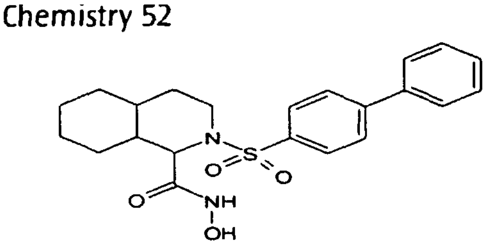 | 414,5275 | 415,26 | 1,1-2,0 (4 m, 12 H); 3,5-3,8 (2 m, 2 H); 4,15 (d, 1 H); 7,4-8,0 (m, 9 H); 8,9 (s, 1 H); 10,9 (s, 1 H) |

(fortgesetzt)

| Beispiel | Struktur | Mol-gewicht | ES+ | 1H-NMR |
|---|---|---|---|---|
| 26 | Chemistry 55 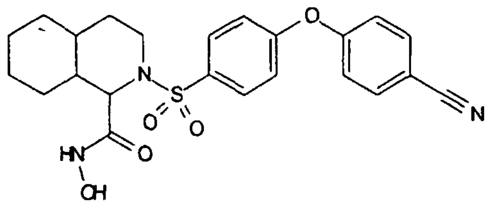 | 455,5368 | 456,30 | 1,1-2,0 (m, 12 H); 3,50; 3,75, 4,10 (3 m, 3 H); 7,2 (m, 4 H); 7,8-8,0 (dd, 4 H); 8,9 (s, 1 H); 10,9 (s, 1 H) |
| 27 | 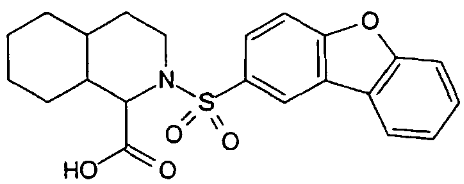 | 413,4963 | 414,14 | 1,2-1,7 (m, 11 H); 2,05 (m, 1 H); 3,5 (m, +/- 2 H); 4,3 (d, 1 H); 7,5-7,9 (6 m, 7 H); 8,7 (s, 1 H) |
| 28 | Chemistry 57 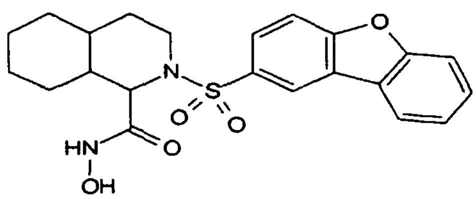 | 428,511 | 429,21 | 1,1-2,0 (m, 12 H); 3,60; 3,75, 4,10 (3 m, 3 H); 7,5-8,9 (7 m, 8 H); 10,9 (s, 1 H) |

(fortgesetzt)

| Beispiel | Struktur | Mol-gewicht | $ES^+$ | 1H-NMR |
|---|---|---|---|---|
| 29 | Chemistry 62 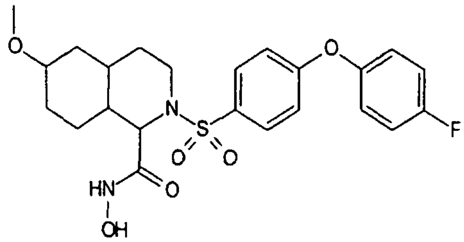 | 478,5438 | 479,22 | 1,0-1,95 (m) and 2,6-4,05 (m, together 14 H); 3,7 (s, 3 H); 6,7 - 7,8 (8 m, 8 H); 8,9 (s, 1 H); 10,9 (2 s, 1 H) |

Pharmakologische Beispiele

Bestimmung der enzymatischen Aktivität der katalytischen Domäne der humanen Kollagenase-1 (MMP-1).

Dieses Protein wird als, inaktives Pro-Enzym von der Fa. Biocol, Potsdam, erhalten (Katalog Nr. MMP1). Aktivierung des Proenzyms:

2 Volumenanteile Proenzym werden mit 1 Volumenanteil APMA-Lösung bei 37 °C für 1 Stunde inkubiert. Die APMA-Lösung wird aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wird durch Zugabe von 1 mmol/L HCl zwischen 7,0 und 7,5 eingestellt. Nach der Aktivierung des Enzyms wird dieses mit dem Tris/HCl Puffer auf eine Konzentration von 2,5 µg/mL verdünnt.

Zur Messung der Enzymaktivität werden 10 µl Enzymlösung mit 10 µL einer 3%igen (v/v) gepufferten Dimethylsulfoxid-Lösung (Reaktion 1) für 15 Minuten inkubiert. Zur Messung der Enzyminhibitoraktivität werden 10 µL Enzymlösung mit 10 µL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3%igen (v/v) wässrigen Dimethylsulfoxid-Lösung, die 0,3 mmol/L des Substrates enthält, die Enzymreaktion fluoreszenzspektroskopisch verfolgt (328 nm (Extinktion) / 393 nm(Emission)). Die Enzymaktivität wird dargestellt als Extinktionszunahme/Minute.

Die Inhibitorwirkung wird als prozentuale Hemmung nach folgender Formel berechnet:

% Hemmung = 100 – [(Extinktionszunahme/Minute in Reaktion 2) / (Extinktionszunahme/Minute in Reaktion 1) x 100].

Der $IC_{50}$, d.h. die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Die Pufferlösung enthält 0,05% Brij (Sigma, Deisenhofen, Deutschland) sowie
0,1 mol/L Tris/HCl, 0,1 mol/L NaCl, 0,01 mol/L $CaCl_2$ (pH=7,5).
Die Enzymlösung enthält 2,5 µg/mL der Enzymdomäne.

Die Substratlösung enthält 0,3 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Darstellung und Bestimmung der enzymatischen Aktivität der katalytischen Domäne des humanen Stromelysins (MMP-3) und der Neutrophilen-Kollagenase (MMP-8). Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur

Messung der Enzymaktivität oder der Enzyminhibitorwirkung wurden 10 µl Enzymlösung mit 10 µl einer 3 %igen (v/v) gepufferten Dimethylsulfoxid-Lösung, die gegebenenfalls den Enzyminhibitor enthielt, für 15 Minuten inkubiert. Nach Zugabe von 10 µl einer 3 %igen (v/v) wässrigen Dimethylsulfoxid-Lösung, die 1 mmol/l des Substrates enthielt, wurde die Enzymreaktion fluoreszenzspektroskopisch verfolgt (328 nm (ex) / 393 nm(em)).

Die Enzymaktivität wird dargestellt als Extinktionszunahme/Minute. Die in Tabelle 2 aufgeführten $IC_{50}$-Werte wurden als diejenige Inhibitorkonzentrationen ermittelt, die jeweils zu einer 50%igen Inhibierung des Enzyms führte.

Die Pufferlösung enthielt 0,05 % Brij (Sigma, Deisenhofen, Deutschland) sowie 0,1 mol/l Tris/HCl, 0,1 mol/l NaCl, 0,01 mol/l $CaCl_2$ und 0,1 mol/l Piperazin-N,N'-bis[2-ethan-sulfonsäure] (pH=7,5).

Die MMP-3 Enzymlösung enthielt 2,3 µg/ml, die MMP-8 Enzymlösung 0,6 µg/ml einer der nach Ye et al. dargestellten Enzymdomänen. Die Substratlösung enthielt 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Kollagenase -3 (MMP-13).

Dieses Protein wurde als inaktives Pro-Enzym von der Fa. INVITEK, Berlin, erhalten (Katalog Nr. 30 100 803). Aktivierung des Proenzyms:

2 Volumenanteile Proenzym wurden mit 1 Volumenanteil APMA-Lösung bei 37 °C für 1,5 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 1,67 µg/mL verdünnt.

Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2).

Sowohl bei Reaktion 1 als auch bei Reaktion 2 wurde nach Zugabe von 10 µL einer 3 %igen (v/v) wässrigen Dimethylsulfoxid-Lösung, die 0,075 mmol/L des Substrates enthielt, die Enzymreaktion fluoreszenzspektroskopisch verfolgt (328 nm (Extinktion) / 393 nm(Emission)). Die Enzymaktivität wurde dargestellt als Extinktionszunahme/Minute.

Die Inhibitorwirkung wurde als prozentuale Hemmung nach folgender Formel berechnet:

$$\%\ \text{Hemmung} = 100 - [(\text{Extinktionszunahme/Minute in Reaktion 2}) / (\text{Extinktionszunahme/Minute in Reaktion 1}) \times 100].$$

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.

Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 1,67 µg/mL der Enzymdomäne. Die Substratlösung enthielt 0,075 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Gelatinase - A(MMP-2).

Dieses Protein wurde als inaktives Pro-Enzym von der Fa. INVITEK, Berlin, erhalten (Katalog Nr. 30 100 602). Aktivierung des Proenzyms:

2 Volumenanteile Proenzym wurden mit 1 Volumenanteil APMA-Lösung bei 37 °C für 0,5 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1mmol/L HCl zwischen 7,0 und 7,5 eingestellt. Nach der Aktivierung des Enzyms wurde dieses mit dem Tris/HCl Puffer auf eine Konzentration von 0,83 µg/mL verdünnt.

Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2).

Sowohl bei Reaktion 1 als auch bei Reaktion 2 wurde nach Zugabe von 10 µL einer 3 %igen (v/v) wässrigen Dimethylsulfoxid-Lösung, die 0,3 mmol/L des Substrates enthielt, die Enzymreaktion fluoreszenzspektroskopisch verfolgt

(328 nm (Extinktion) / 393 nm(Emission)). Die Enzymaktivität wurde als Extinktionszunahme/Minute dargestellt.

Die Inhibitorwirkung wurde als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.

Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 0,83 µg/mL der Enzymdomäne. Die Substratlösung enthielt 0,3 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Gelatinase - B (MMP-9).

Dieses Protein wurde als inaktives Pro-Enzym von der Fa. Roche, Mannheim, erhalten (Katalog Nr. 1 758 896). Aktivierung des Proenzyms:

2 Volumenanteile Proenzym wurden mit 1 Volumenanteil APMA-Lösung bei 37 °C für 4 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 4,2 mU/mL verdünnt.

Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2).

Sowohl bei Reaktion 1 als auch bei Reaktion 2 wurde nach Zugabe von 10 µL einer 3 %igen (v/v) wässrigen Dimethylsulfoxid-Lösung, die 0,15 mmol/L des Substrates enthielt, die Enzymreaktion fluoreszenzspektroskopisch verfolgt (328 nm (Extinktion) / 393 nm(Emission)). Die Enzymaktivität wurde dargestellt als Extinktionszunahme/Minute.

Die Inhibitorwirkung wurde als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.

Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 4,2 mU/mL der Enzymdomäne. Die Substratlösung enthielt 0,15 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Die nachfolgende Tabelle 3 zeigt die Ergebnisse.

Tabelle 3:

| Beispiel | MMP-1 $IC_{50}$ [nM] | MMP-2 $IC_{50}$ [nM] | MMP-3 $IC_{50}$ [nM] | MMP-8 $IC_{50}$ [nM] | MMP-9 $IC_{50}$ [nM] | MMP-13 $IC_{50}$ [nM] |
|---|---|---|---|---|---|---|
| 3 | 400 | 30 | 40 | 3 | 100 | 3 |
| 5 | 70 | 2 | 28 | 2,5 | 1,2 | 1,8 |
| 7 | 600 | 4 | 65 | 13 | 3 | 7 |
| 9 | 1000 | 20 | 160 | 22 | 12 | 21 |
| 11 | 220 | 2 | 25 | 2,5 | 2 | 1,5 |

(fortgesetzt)

| Beispiel | MMP-1 $IC_{50}$ [nM] | MMP-2 $IC_{50}$ [nM] | MMP-3 $IC_{50}$ [nM] | MMP-8 $IC_{50}$ [nM] | MMP-9 $IC_{50}$ [nM] | MMP-13 $IC_{50}$ [nM] |
|---|---|---|---|---|---|---|
| 13 | 180 | 2,6 | 30 | 4 | 2 | 2 |
| 14 | 400 | 2 | 25 | 2,2 | 3,5 | 2 |
| 15 | 6000 | 2300 | 10000 | 2000 | 4000 | 2300 |
| 19 | 2100 | 3 | 59 | 10 | 5 | 3 |
| 20 | 41 | 2 | 26 | 3 | 2 | 2 |
| 21 | 21 | 1,5 | 23 | 2,3 | 0,45 | 1,3 |
| 23 | 290 | 2 | 50 | 9 | 2 | 2,1 |
| 26 | 450 | 3 | 41 | 16 | 3 | 3 |

**Patentansprüche**

**1.** Verbindung der Formel I

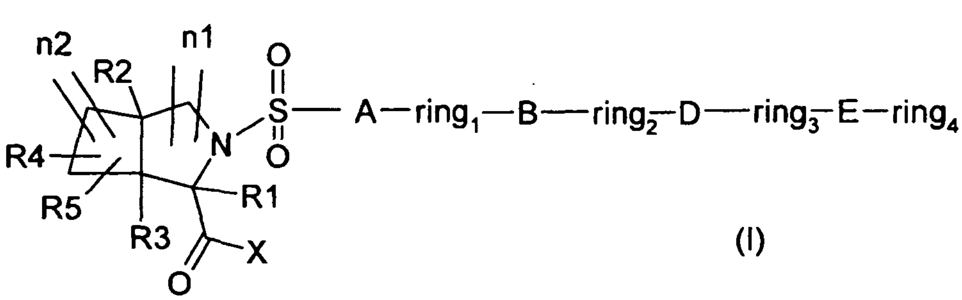

(I)

und/oder alle stereoisomeren Formen der Verbindung der Formel I und/oder Gemische dieser Formen in jedem Verhältnis, und/oder ein physiologisch verträgliches Salz der Verbindung der Formel I, wobei
A für -($C_0$-$C_4$)-Alkylen steht,
B, D und E gleich oder verschieden sind und unabhängig voneinander für -($C_0$-$C_4$)-Alkylen oder den Rest -B1-B2-B3- stehen, worin B1 für -$(CH_2)_n$- steht, worin n die ganze Zahl Null, 1 oder 2 bedeutet,
B3 für -$(CH_2)_m$- steht, worin m die ganze Zahl Null, 1 oder 2 bedeutet,
mit der Maßgabe, dass die Summe von n und m den Betrag Null, 1 oder 2 hat, und B2 für

1) -C(O)-
2) -($C_2$-$C_4$)-Alkenylen,
3) -$S(O)_o$-, wobei o die ganzen Zahlen Null, 1 oder 2 bedeutet,
4) -N(R6)-, worin R6 Wasserstoffatom, Methyl oder Ethyl bedeutet,
5) -N(R6)-C(Y)-, worin Y Sauerstoffatom oder Schwefelatom bedeutet und R6 wie oben definiert ist,
6) -C(Y)-N(R6)-, worin Y Sauerstoffatom oder Schwefelatom bedeutet und R6 wie oben definiert ist,
7) -N(R6)-$SO_2$-, worin R6 wie oben definiert ist,
8) -$SO_2$-N(R6)-, worin R6 wie oben definiert ist,
9) -N(R6)-$SO_2$-N(R6)-, worin R6 wie oben definiert ist,
10) -N(R6)-C(Y)-N(R6)-, worin Y Sauerstoffatom oder Schwefelatom bedeutet und R6 wie oben definiert ist,
11) -O-C(O)-N(R6)-,
12) -NH-C(O)-O-,
13) -O-,
14) -C(O)-O-,
15) -O-C(O)-,
16) -O-C(O)-O-,
17) -O-$CH_2$-C(O)-,

18) $-O-CH_2-C(O)-O-$,
19) $-O-CH_2-C(O)-N(R6)-$, worin R6 wie oben definiert ist,
20) $-C(O)-CH_2-O-$,
21) $-O-C(O)-CH_2-O-$,
22) $-N(R6)-C(O)-CH_2-O-$, worin R6 wie oben definiert ist,
23) $-O-(CH_2)_n-O-$, worin n die ganze Zahl 2 oder 3 bedeutet, oder
24) $-O-(CH_2)_m-N(R6)-$, worin m die ganze Zahl 2 oder 3 bedeutet und R6 wie oben definiert ist,
25) $-N(R6)-(CH_2)_m-O-$, worin m die ganze Zahl 2 oder 3 bedeutet und R6 wie oben definiert ist,
26) -N(R6)-N(R6)-, worin R6 wie oben definiert ist,
27) -N=N-,
28) -N(R6)-CH=N-, worin R6 wie oben definiert ist,
29) -N=CH-N(R6)-, worin R6 wie oben definiert ist,
30) -N(R6)-C(R7)=N-, worin R6 wie oben definiert ist und R7 -NH-R6 bedeutet,
31) -N=C(R7)-N(R6)-, worin R6 wie oben definiert ist und R7 -NH-R6 bedeutet, oder
32) $-(C_2-C_6)$-Alkinylen, steht,

ring1, ring2 oder ring3 gleich oder verschieden sind und unabhängig voneinander für

1) kovalente Bindung,
2) $-(C_6-C_{14})$-Aryl, worin Aryl unsubstituiert oder unabhängig voneinander ein-, zwei- oder dreifach durch G substituiert ist, oder
3) 4- bis 15-gliedriger Het-Ring, worin Het-Ring unsubstituiert oder unabhängig voneinander ein-, zwei- oder dreifach durch G substituiert ist, steht,

ring4 für

1) $-(C_6-C_{14})$-Aryl, worin Aryl unsubstituiert oder unabhängig voneinander ein-, zwei- oder dreifach durch G substituiert ist,
2) 4- bis 15-gliedriger Het-Ring, worin der Het-Ring unsubstituiert oder unabhängig voneinander ein-, zwei- oder dreifach durch G substituiert ist, oder
3) für einen der folgenden Reste

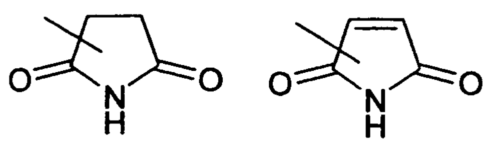

und diese Reste unsubstituiert oder einfach durch G substituiert sind, steht,

G für

1) Wasserstoffatom,
2) Halogen,
3) =O,
4) $-(C_1-C_6)$-Alkyl, worin Alkyl unsubstituiert oder ein-, zwei- oder dreifach durch Halogen, $-(C_3-C_6)$-Cycloalkyl, $-(C_2-C_6)$-Alkinyl, $-(C_6-C_{14})$-Aryl oder Het-Ring substituiert ist,
5) $-(C_6-C_{14})$-Aryl,
6) Het-Ring,
7) -C(O)-O-R10, worin R10

a) $-(C_1-C_6)$-Alkyl, worin Alkyl unsubstituiert oder ein- oder zweifach durch $-(C_3-C_6)$-Cycloalkyl, $-(C_2-C_6)$-Alkinyl, $-(C_6-C_{14})$-Aryl, oder Het-Ring substituiert ist, oder
b) $-(C_6-C_{14})$-Aryl oder Het-Ring, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Het-Ring substituiert ist, oder
b) -($C_6$-$C_{14}$)-Aryl oder Het-Ring, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist,
c) -($C_6$-$C_{14}$)-Aryl,
d) Het-Ring,
e) -C(O)-O-R13, worin R13

e)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, oder
e)2) -($C_6$-$C_{14}$)-Aryl oder Het-Ring, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, oder
g)2) -($C_6$-$C_{14}$)-Aryl oder Het-Ring bedeutet, oder

h) -C(S)-NH-R14, worin R14 wie oben definiert ist, bedeutet,

12) -C(O)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN,
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, bedeutet und R12 wie oben definiert ist, oder

17) -$SO_2$-N (R12)-R1, worin R12 wie oben definiert ist und R1 wie unten definiert ist, steht,

X für -OH oder -NH-OH steht,
n1 für die ganze Zahl 2 steht,
n2 für die ganze Zahl 3 steht,
R1, R2, R3, R4 und R5 gleich oder verschieden sind und unabhängig voneinander für

1) Wasserstoffatom,
2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, steht,
3) -C(O)-O-R8, worin R8

3)1) Wasserstoffatom,
3)2) -($C_1$-$C_6$)-Alkyl, worin Alkyl unsubstituiert oder ein- oder zweifach durch -($C_3$-$C_6$)-Cycloalkyl,-($C_2$-$C_6$)-Alkinyl, -($C_6$-$C_{14}$)-Aryl, oder Het-Ring oder ein- bis fünffach durch Fluor, substituiert ist, oder 3)3) -($C_6$-$C_{14}$)-Aryl oder Het-Ring bedeutet,

4) -O-R8, worin R8 die oben genannte Bedeutung hat, oder
5) -($C_3$-$C_6$)-Cycloalkyl, stehen.

**2.** Verbindung der Formel I gemäß Anspruch 1,
A für -($C_0$-$C_4$)-Alkylen steht,
B, D und E gleich oder verschieden sind und unabhängig voneinander für -($C_0$-$C_4$)-Alkylen oder den Rest -B1-B2-B3- stehen, worin B1 für -($(CH_2)_n$- steht, worin n die ganze Zahl Null, 1 oder 2 bedeutet,
B3 für -$(CH_2)_m$- steht, worin m die ganze Zahl Null, 1 oder 2 bedeutet,
mit der Maßgabe, dass die Summe von n und m den Betrag Null, 1 oder 2 hat, und B2 für

1) -C(O)-
2) -($C_2$-$C_4$)-Alkenylen,
3) -$S(O)_o$-, wobei o die ganzen Zahlen Null, 1 oder 2 bedeutet,
4) -N(R6)-, worin R6 Wasserstoffatom, Methyl oder Ethyl bedeutet,
5) -N(R6)-C(Y)-, worin Y Sauerstoffatom oder Schwefelatom bedeutet und R6 wie oben definiert ist,
6) -C(Y)-N(R6)-, worin Y Sauerstoffatom oder Schwefelatom bedeutet und R6 wie oben definiert ist,
7) -N(R6)-$SO_2$-, worin R6 wie oben definiert ist,
8) -$SO_2$-N(R6)-, worin R6 wie oben definiert ist,
9) -N(R6)-$SO_2$-N(R6)-, worin R6 wie oben definiert ist,
10) -N(R6)-C(Y)-N(R6)-, worin Y Sauerstoffatom oder Schwefelatom bedeutet und R6 wie oben definiert ist,
11) -O-C(O)-N(R6)-,
12) -NH-C(O)-O-,
13) -O-,
14) -C(O)-O-,
15) -O-C(O)-,
16) -O-C(O)-O-,
17) -O-$CH_2$-C(O)-,
18) -O-$CH_2$-C(O)-O-,
19) -O-$CH_2$-C(O)-N(R6)-, worin R6 wie oben definiert ist,
20) -C(O)-$CH_2$-O-,
21) -O-C(O)-$CH_2$-O-,
22) -N(R6)-C(O)-$CH_2$-O-, worin R6 wie oben definiert ist,
23) -O-$(CH_2)_n$-O-, worin n die ganze Zahl 2 oder 3 bedeutet, oder
24) -O-$(CH_2)_m$-N(R6)-, worin m die ganze Zahl 2 oder 3 bedeutet und R6 wie oben definiert ist,
25) -N(R6)-$(CH_2)_m$-O-, worin m die ganze Zahl 2 oder 3 bedeutet und R6 wie oben definiert ist,
26) -N(R6)-N(R6)-, worin R6 wie oben definiert ist,
27) -N=N-,
28) -N(R6)-CH=N-, worin R6 wie oben definiert ist,
29) -N=CH-N(R6)-, worin R6 wie oben definiert ist,
30) -N(R6)-C(R7)=N-, worin R6 wie oben definiert ist und R7 -NH-R6 bedeutet,
31) -N=C(R7)-N(R6)-, worin R6 wie oben definiert ist und R7 -NH-R6 bedeutet,
oder
32) -($C_2$-$C_6$)-Alkinylen, steht,

ring1, ring2 oder ring3 gleich oder verschieden sind und unabhängig voneinander für

1) kovalente Bindung,
2) Phenyl oder Naphthyl bedeutet und unsubstituiert oder unabhängig voneinander ein-, zwei- oder dreifach durch G substituiert sind, oder
3) 4- bis 15-gliedriger Het-Ring steht, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,4-Triazolyl, 1,2,5-Triazolyl, 1 ,3,4-Triazolyl und Xanthenyl ist und unsubstituiert oder unabhängig voneinander ein-, zwei- oder dreifach durch G substituiert sind,

ring4 für

1) -($C_6$-$C_{14}$)-Aryl steht, worin Aryl ein Rest aus der Reihe Phenyl, Naphthyl, 1-Naphthyl, 2-Naphthyl, Anthryl oder Fluorenyl bedeutet und unsubstituiert oder unabhängig voneinander ein-, zwei- oder dreifach durch G substituiert sind,
2) 4- bis 15-gliedriger Het-Ring steht, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, 2'-Methyl-biphenyl-2-ol, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,4-Triazolyl, 1,2,5-Triazolyl, 1,3,4-Triazolyl und Xanthenyl ist und unsubstituiert oder unabhängig voneinander ein-, zwei- oder dreifach durch G substituiert sind, oder
3) für einen der folgenden Reste

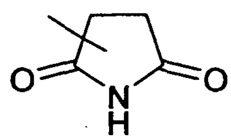 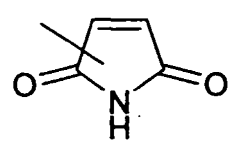

steht und diese Reste unsubstituiert oder einfach durch G substituiert sind,

G für

1) Wasserstoffatom,
2) Halogen,
3) =O,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist, wobei Aryl und Het-Ring wie oben definiert sind,
5) -($C_6$-$C_{14}$)-Aryl, wobei Aryl wie oben definiert ist,
6) Het-Ring, wobei Het-Ring wie oben definiert ist,
7) -C(O)-O-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl , -($C_6$-$C_{14}$)-Aryl, oder Het-Ring substituiert ist, wobei Aryl und Het-Ring wie oben definiert sind, oder
b) -($C_6$-$C_{14}$)-Aryl oder Het-Ring, wobei Aryl und Het-Ring wie oben definiert sind, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, wobei Aryl und Het-Ring wie oben definiert sind, oder
b) -($C_6$-$C_{14}$)-Aryl oder Het-Ring, wobei Aryl und Het-Ring wie oben definiert sind, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl oder Het-Ring substituiert ist, wobei Aryl und Het-Ring wie oben definiert sind,
c) -($C_6$-$C_{14}$)-Aryl, wobei Aryl wie oben definiert ist,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, wobei Aryl und Het-Ring wie oben definiert sind, oder
e)2) -($C_6$-$C_{14}$)-Aryl oder Het-Ring, wobei Aryl und Het-Ring wie oben definiert sind, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, wobei Aryl und Het-Ring wie oben definiert sind, oder
g)2) -($C_6$-$C_{14}$)-Aryl oder Het-Ring bedeutet, wobei Aryl und Het-Ring wie oben definiert sind, oder

h) -C(S)-NH-R14, worin R14 wie oben definiert ist, bedeutet,

12) -C(O)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN,
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, bedeutet und R12 wie oben definiert ist,

17) -$SO_2$-N (R12)-R1, worin R12 wie oben definiert ist und R1 wie unten definiert ist, steht,

X für -OH oder -NH-OH steht,
n1 für die ganze Zahl 2 steht,
n2 für die ganze Zahl 3 steht,
R1, R2, R3, R4 und R5 gleich oder verschieden sind und unabhängig voneinander für

1) Wasserstoffatom,
2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, steht,
3) -C(O)-O-R8, worin R8

3) 1) Wasserstoffatom,
3)2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring oder ein- bis fünffach durch Fluor, substituiert ist, oder
3)3) -($C_6$-$C_{14}$)-Aryl oder Het-Ring bedeutet,

4) -O-R8, worin R8 die oben genannte Bedeutung hat, oder
5) -($C_3$-$C_6$)-Cycloalkyl, stehen

**3.** Verbindung der Formel I gemäß der Ansprüche 1 oder 2, wobei A für -($C_0$-$C_4$)-Alkylen steht, B, D und E gleich oder verschieden sind und unabhängig voneinander für -($C_0$-$C_4$)-Alkylen oder den Rest -B1-B2-B3- stehen, worin B1 für $-(CH_2)_n-$ steht, worin n die ganze Zahl Null, 1 oder 2 bedeutet, B3 für $-(CH_2)_m-$ steht, worin m die ganze Zahl Null, 1 oder 2 bedeutet, mit der Maßgabe, dass die Summe von n und m den Betrag Null, 1 oder 2 hat, und B2 für

1) -($C_0$-$C_2$)-Alkylen,
2) Ethenylen,
3) Ethinylen,
4) -C(O)-
5) -N(R6)-C(O)-, worin R6 Wasserstoffatom, Methyl oder Ethyl bedeutet,
6) -C(O)-N(R6)-, worin R6 wie oben definiert ist,
7) -O- oder
8) -S-,

ring1, ring2 oder ring3 gleich oder verschieden sind und unabhängig voneinander für

1) kovalente Bindung stehen,
2) Phenyl oder Naphthyl bedeutet und unsubstituiert oder unabhängig voneinander ein- oder zweifach durch G substituiert sind, oder
3) Het-Ring stehen, worin der Het-Ring ein Rest aus der Reihe Dihydrofuranyl, Furanyl, Pyridinyl, Pyrimidinyl, Pyrrolyl, Thiadiazolyl, Thiazolyl oder Thiophenyl bedeutet und unsubstituiert oder unabhängig voneinander ein- oder zweifach durch G substituiert sind,

ring4 für

1) Phenyl oder Naphthyl steht und unsubstituiert oder unabhängig voneinander ein-, oder zweifach durch G substituiert ist,
2) Het-Ring steht, worin der Het-Ring ein Rest aus der Reihe Benzofuranyl, Dihydrofuranyl, Dibenzofuranyl, Dibenzothiophenyl, Furanyl, 2'-Methylbiphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und unsubstituiert oder unabhängig voneinander ein- oder zweifach durch G substituiert ist, oder
3) für den folgenden Rest

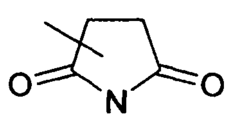

steht und dieser Rest unsubstituiert oder einfach durch G substituiert ist,

G für

1) Wasserstoffatom,
2) Br, Cl oder F,
3) -($C_1$-$C_4$)-Alkyl, worin Alkyl unsubstituiert oder ein-, oder zweifach durch F, Phenyl, -$C_3$-Cycloalkyl oder Het-Ring, wobei Het-Ring wie oben definiert ist,
4) Phenyl,
5) Het-Ring, wobei Het-Ring wie oben definiert ist,
6) -C(O)-O-R10, worin R10

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder

c) Het-Ring, wobei Het-Ring wie oben definiert ist, bedeutet,

7) -C(O)-NH-R11, worin R11

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

8) -O-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch Cyclopropyl, Phenyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-O-R13, worin R13 wie oben definiert ist, oder
g) -C(O)-NH-R14, worin R14

g)1) -($C_1$-$C_6$)-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) -$S(O)_p$-R12, worin R12 wie oben definiert ist und p die ganzen Zahlen 1 oder 2 bedeutet,
11) -$NO_2$,
12) -CN oder
13) -N(R15)-R12, worin R15

13)1) Wasserstoffatom oder
13)2) -($C_1$-$C_6$)-Alkyl bedeutet und R12 wie oben definiert ist, steht,

X für -OH oder -NH-OH steht,
n1 für die ganze Zahl 2 steht,
n2 für die ganze Zahl 3 steht,
R1, R2 und R3 jeweils für Wasserstoffatom stehen,
R4 und R5 gleich oder verschieden sind und unabhängig voneinander für

1) Wasserstoffatom,
2) Methyl,
3) Ethyl oder
4) -OH stehen.

**4.** Verbindung der Formel I gemäß einem oder mehreren der Ansprüche 1 bis 3, wobei
A für -($C_0$-$C_4$)-Alkylen steht,
B, D und E gleich oder verschieden sind und unabhängig voneinander für -($C_0$-$C_4$)-Alkylen oder den Rest -B1-B2-B3- stehen, worin B1 für $-(CH_2)_n-$ steht, worin n die ganze Zahl Null, 1 oder 2 bedeutet,
B3 für $-(CH_2)_m-$ steht, worin m die ganze Zahl Null, 1 oder 2 bedeutet,
mit der Maßgabe, dass die Summe von n und m den Betrag Null, 1 oder 2 hat, und B2 für

1) -($C_0$-$C_2$)-Alkylen,
2) Ethenylen oder

3) Ethinylen,

ring1, ring2 oder ring3 gleich oder verschieden sind und unabhängig voneinander für

1) kovalente Bindung stehen,
2) Phenyl bedeutet und unsubstituiert oder unabhängig voneinander ein- oder zweifach durch G substituiert sind, oder
3) Het-Ring stehen, worin der Het-Ring ein Rest aus der Reihe Dihydrofuranyl, Furanyl, Morpholinyl, Piperazinyl, Piperidinyl, Pyridinyl, Pyrimidinyl, Pyrrolyl, Thiazolyl oder Thiophenyl bedeutet und unsubstituiert oder unabhängig voneinander ein- oder zweifach durch G substituiert sind,

ring4 für

1) Phenyl steht und unsubstituiert oder unabhängig voneinander ein-, oder zweifach durch G substituiert ist,
2) Het-Ring steht, worin der Het-Ring ein Rest aus der Reihe Benzofuranyl, Dihydrofuranyl, Dibenzofuranyl, Dibenzothiophenyl, Furanyl, 2'-Methylbiphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und bedeutet und unsubstituiert oder unabhängig voneinander ein- oder zweifach durch G substituiert ist, oder
3) für den folgenden Rest

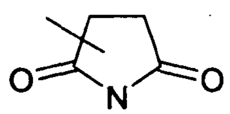

steht und dieser Rest unsubstituiert oder einfach durch G substituiert ist,

G für

1) Wasserstoffatom,
2) Br, Cl oder F,
3) -($C_1$-$C_4$)-Alkyl, worin Alkyl unsubstituiert oder ein-, zwei- oder dreifach durch Br, Cl, F, -$C_3$-Cycloalkyl, Phenyl oder Het-Ring, wobei Het-Ring wie oben definiert ist,
4) Phenyl,
5) Het-Ring, wobei Het-Ring wie oben definiert ist,
6) -C(O)-O-R10, worin R10

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

7) -C(O)-NH-R11, worin R11

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl oder Naphthyl, oder
c) Het-Ring, wobei Het-Ring wie oben definiert ist, bedeutet,

8) -O-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch Cyclopropyl, Phenyl, Naphthyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-O-R13, worin R13 wie oben definiert ist, oder
g) -C(O)-NH-R14, worin R14

g)1) -($C_1$-$C_6$)-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) -$S(O)_p$-R12, worin R12 wie oben definiert ist und p die ganzen Zahlen 1 oder 2 bedeutet,
11) -$NO_2$,
12) -CN oder
13) -N(R15)-R12, worin R15

13)1) Wasserstoffatom oder
13)2) -($C_1$-$C_6$)-Alkyl bedeutet und R12 wie oben definiert ist, steht,

X für -OH oder-NH-OH steht,
n1 für die ganze Zahl 2 steht,
n2 für die ganze Zahl 3 steht,
R1, R2, R3, R4 und R5 jeweils für Wasserstoffatom stehen.

**5.** Verbindung der Formel I gemäß einem oder mehreren der Ansprüche 1 bis 4, wobei
A für eine kovalente Bindung oder -$CH_2$-$CH_2$- steht,
B, D und E gleich oder verschieden sind und unabhängig voneinander für -($C_0$-$C_4$)-Alkylen oder den Rest -B1-B2-B3- stehen, worin B1 für -$(CH_2)_n$- steht, worin n die ganze Zahl Null, 1 oder 2 bedeutet,
B3 für -$(CH_2)_m$- steht, worin m die ganze Zahl Null, 1 oder 2 bedeutet,
mit der Maßgabe, dass die Summe von n und m den Betrag Null, 1 oder 2 hat, und B2 für

1) -C(O)-
2) -($C_2$-$C_4$)-Alkinylen,
3) -$S(O)_o$-, wobei o die ganzen Zahlen Null oder 1 bedeutet,
4) -N(R6)-C(Y)-, worin Y Sauerstoffatom und R6 Wasserstoffatom bedeutet,
5) -C(Y)-N(R6)-, worin Y Sauerstoffatom und R6 Wasserstoffatom bedeutet, oder
6) -O- steht

ring1, ring2 oder ring3 gleich oder verschieden sind und unabhängig voneinander für

1) kovalente Bindung stehen,
2) Phenyl bedeutet und unsubstituiert oder unabhängig voneinander ein- oder zweifach durch G substituiert sind, oder
3) Het-Ring stehen, worin der Het-Ring ein Rest aus der Reihe Furanyl, Pyridinyl, Pyrimidinyl oder Thiophenyl bedeutet und unsubstituiert oder unabhängig voneinander ein- oder zweifach durch G substituiert sind,

ring4 für

1) Phenyl steht und unsubstituiert oder unabhängig voneinander ein- oder zweifach durch G substituiert ist,
2) Het-Ring steht, worin der Het-Ring ein Rest aus der Reihe Benzofuranyl, Dibenzofuranyl, Furanyl, 2'-Methylbiphenyl-2-ol, Morpholinyl, Piperazinyl, Piperidinyl, Pyridinyl, Pyrimidinyl, Pyridothiophenyl, Pyrrolyl, Pyrrolidinyl, Thiazolyl oder Thiophenyl bedeutet und unsubstituiert oder unabhängig voneinander ein- oder zweifach durch G substituiert ist, oder
3) für den folgenden Rest

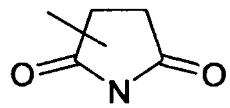

steht und dieser Rest unsubstituiert oder einfach durch G substituiert ist,

G für

1) Wasserstoffatom,
2) Br, Cl oder F,
3) -($C_1$-$C_4$)-Alkyl, worin Alkyl unsubstituiert oder ein-, zwei- oder dreifach durch Br, Cl, F, -$C_3$-Cycloalkyl, Phenyl oder Het-Ring, wobei Het-Ring wie oben definiert ist,
4) Phenyl,
5) Het-Ring, wobei Het-Ring wie oben definiert ist,
6) -C(O)-O-R10, worin R10

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

7) -C(O)-NH-R11, worin R11

a) -($C_1$-$C_6$)-Alkyl, worin Alkyl unsubstituiert oder ein- oder zweifach durch Cyclopropyl, Phenyl oder Het-Ring, wobei Het-Ring wie oben definiert ist, substituiert ist,
b) Phenyl, oder
c) Het-Ring, wobei Het-Ring wie oben definiert ist, bedeutet,

8) -O-R12, worin R12

a) Wasserstoffatom,
b) -($C_1$-$C_6$)-Alkyl, worin Alkyl unsubstituiert oder ein-, zwei- oder dreifach durch Halogen, Cyclopropyl, Phenyl oder Het-Ring substituiert ist, wobei Het-Ring wie oben definiert ist,
c) Phenyl,
d) Het-Ring, wobei Het-Ring wie oben definiert ist,
e) -C(O)-O-R13, worin R13

e)1) -($C_1$-$C_6$)-Alkyl, worin Alkyl unsubstituiert oder ein- oder zweifach durch Cyclopropyl, Phenyl oder Het-Ring substituiert ist, wobei Het-Ring wie oben definiert ist, oder
e)2) Phenyl oder Het-Ring, wobei Het-Ring wie oben definiert ist, bedeutet,

f) -C(S)-O-R13, worin R13 wie oben definiert ist, oder
g) -C(O)-NH-R14, worin R14

g)1) -($C_1$-$C_6$)-Alkyl, worin Alkyl unsubstituiert oder ein- oder zweifach durch Phenyl oder Het-Ring substituiert ist, wobei Het-Ring wie oben definiert ist, oder
g)2) Phenyl oder Het-Ring bedeutet, wobei Het-Ring wie oben definiert ist, bedeutet,

9) -C(O)-R10, worin R10 wie oben definiert ist,
10) -$S(O)_p$-R12, worin R12 wie oben definiert ist und p die ganzen Zahlen Null, 1 oder 2 bedeutet,
11) -$NO_2$,
12) -CN oder
13) -N(R15)-R12, worin R15

13)1) Wasserstoffatom oder
13)2) -($C_1$-$C_6$)-Alkyl bedeutet und R12 wie oben definiert ist, steht,

X für -NH-OH steht,
n1 für die ganze Zahl 2 steht,
n2 für die ganze Zahl 3 steht, und
R1, R2, R3, R4 und R5 jeweils für Wasserstoffatom stehen.

**6.** Verbindung der Formel I gemäß einem oder mehreren der Ansprüche 1 bis 5, wobei es eine Verbindung aus der Reihe
2-(4'-Nitro-biphenyl-4-sulfonyl)-decahydro-isochinolin-1-(N-hydroxy)-carboxamid,
2-(4'-Chlor-biphenyl-4-sulfonyl)-decahydro-isochinolin-1-carbonsäure,
2-(4'-Chlor-biphenyl-4-sulfonyl)-decahydro-isochinolin-1-(N-hydroxy)-carboxamid,
2-(6-Phenoxy-pyridin-3-sulfonyl)-decahydro-isochinolin-1- carbonsäure; Trifluoracetat,
2-(6-Phenoxy-pyridin-3-sulfonyl)-decahydro-isochinolin-1-(N-hydroxy)-carboxamid; Trifluoracetat,
2-[2-(4'-Chlor-biphenyl-4-yl)-ethanesulfonyl]-decahydro-isochinolin-1-carbonsäure,
2-[2-(4'-Chlor-biphenyl-4-yl)-ethanesulfonyl]-decahydro-isochinolin-1-(N-hydroxy)-carboxamid,
2-[4-(Pyridin-4-yloxy)-benzensulfonyl]-decahydro-isochinolin-1-carbonsäure; Trifluoracetat,
2-[4-(Pyridin-4-yloxy)-benzensulfonyl]-decahydro-isochinolin-1-(N-hydroxy)-carboxamid; Trifluoracetat,
2-[4-(4-Methoxy-phenoxy)-benzenesulfonyl]-decahydro-isochinolin-1-carbonsäure,
2-[4-(4-Methoxy-phenoxy)-benzenesulfonyl]-decahydro-isochinolin-1-(N-hydroxy)-carboxamid,
2-{4-[4-(2,2,2-Trifluor-ethoxy)-phenoxy]-benzensulfonyl}-decahydro-isochinolin-1-carbonsäure, 2-{4-[4-(2,2,2-Trifluor-ethoxy)-phenoxy]-benzensulfonyl}-decahydro-isochinolin-1-(N-hydroxy)-carboxamid,
2-[4'-(2,2,2-Trifluor-ethoxy)-biphenyl-4-sulfonyl]-decahydro-isochinolin-1-carbonsäure,
2-(4'-Isopropoxycarbonylamino-biphenyl-4-sulfonyl)-decahydro-isochinolin-1-carbonsäure,
[4'-(1-Hydroxycarbamoyl-octahydro-isochinolin-2-sulfonyl)-biphenyl-4-yl]-carboxamid isopropyl ester,
2-[4'-(2,2,2-Trifluor-ethoxy)-biphenyl-4-sulfonyl]-decahydro-isochinolin-1-carbonsäure hydroxyamid,
2-(4'-Trifluormethoxy-biphenyl-4-sulfonyl)-decahydro-isochinolin-1-carbonsäure hydroxyamid,
2-[4-(4-Fluor-phenoxy)-benzenesulfonyl]-decahydro-isochinolin-1-carbonsäure hydroxyamid,
2-[4-(4-Trifluormethoxy-phenoxy)-benzenesulfonyl]-decahydro-isochinolin-1-carbonsäure hydroxyamid,
2-[4-(4-Trifluormethoxy-phenoxy)-benzenesulfonyl]-decahydro-isochinolin-1-carbonsäure,
2-(Biphenyl-4-sulfonyl)-decahydro-isochinolin-1-carbonsäure hydroxyamid,
2-(Biphenyl-4-sulfonyl)-decahydro-isochinolin-1-carbonsäure,
2-[4-(4-Cyan-phenoxy)-benzensulfonyl]-decahydro-isochinolin-1 -carbonsäure hydroxyamid,
2-(Dibenzofuran-2-sulfonyl)-decahydro-isochinolin-1-carbonsäure,
2-(Dibenzofuran-2-sulfonyl)-decahydro-isochinolin-1-carbonsäure hydroxyamid oder
2-[4-(4-Fluor-phenoxy)-benzenesulfonyl]-6-methoxy-decahydro-isochinolin-1-carbonsäure hydroxyamid, sowie alle ihre Stereoisomeren Formen, ist.

**7.** Verfahren zur Herstellung der Verbindung der Formel I gemäß einem oder mehreren der Ansprüche 1 bis 6, **dadurch gekennzeichnet, dass** man

a) eine Verbindung der Formel IV,

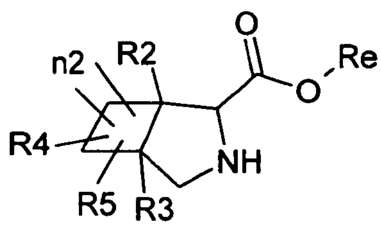

**(IV)**

worin Re ein Wasserstoffatom oder eine Ester-Schutzgruppe darstellt, mit einer Verbindung der Formel V,

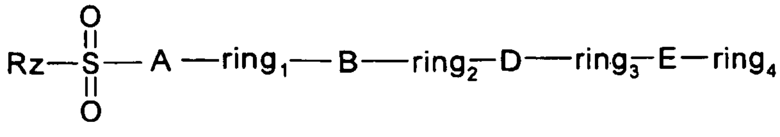

(V)

worin A, B, D, E und ring1, ring2, ring3, ring4 wie in Formel I definiert sind, und worin Rz Chloratom, Imidazoyl

oder OH bedeutet,
in Gegenwart einer Base oder nach Silylierung mit einem geeigneten Silylierungsmittel oder mit einem geeigneten wasserentziehenden Mittel für den Fall Rz = OH zu einer Verbindung der Formel VI umsetzt,

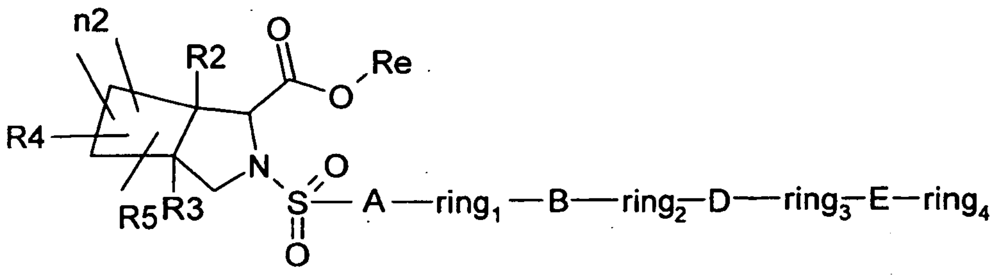

(VI)

worin A, B, D, E, Re und ring1, ring2, ring3 und ring4 wie oben definiert sind, oder
b) für den Fall Re = Ester eine nach a) hergestellte Verbindung der Formel VI mit einer Alkalilauge wie NaOH oder LiOH und anschließender Säurebehandlung zu der erfindungsgemäßen Carbonsäure der Formel I, worin X = OH (entspricht VII) ist, umsetzt, wobei gegebenenfalls vorher noch Modifikationen in einer der Seitenketten der Ringe ring1-ring4 vorgenommen wurden; oder den genannten Ester durch Behandlung mit Mineralsäure wie Salzsäure zur freien Carbonsäure VII umsetzt

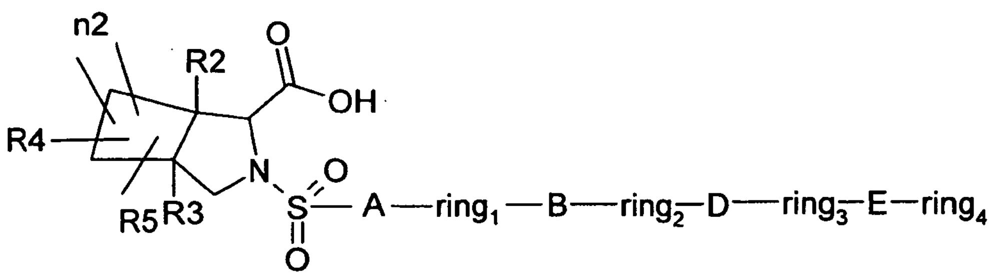

(VII)

und anschließend diese in die erfindungsgemäße Hydroxamsäure, worin X = NH-OH ist, der Formel I umwandelt, oder
c) eine nach Verfahren a) hergestellte Verbindung der Formel I, oder eine geeignete Vorstufe der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren b) oder c) hergestellte Verbindung der Formel I entweder in freier Form isoliert oder im Falle des Vorliegens von sauren oder basischen Gruppen in physiologisch verträgliche Salze umwandelt.

**8.** Arzneimittel, **gekennzeichnet durch** einen wirksamen Gehalt an mindestens einer Verbindung der Formel I gemäß einem oder mehreren der Ansprüche 1 bis 6 zusammen mit einem pharmazeutisch geeigneten und physiologisch verträglichen Trägerstoff, Zusatzstoff und/oder Hilfsstoff.

**9.** Verwendung der Verbindung der Formel I gemäß einem oder mehreren der Ansprüche 1 bis 6 zur Herstellung eines Arzneimittels zur Prophylaxe und Therapie von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, zur Behandlung der Ulceration, Atherosklerose und Stenosen, Behandlung von Entzündungen, Krebserkrankungen, Tumormetastasenbildung, Kachexie, Anorexie, Herzversagen und septischem Schock oder Prophylaxe von Myocard- und Cerebral-Infarkten.

**Claims**

**1.** Compound of the formula I

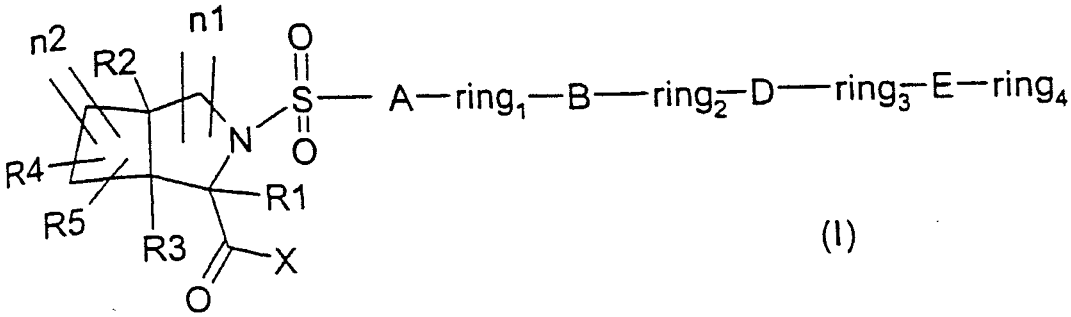

(I)

and/or all the stereoisomeric forms of the compound of the formula I and/or mixtures of these forms in any ratio, and/or a physiologically tolerated salt of the compound of the formula I, wherein

A is -($C_0$-$C_4$)-alkylene,

B, D and E are identical or different and are, independently of each other, -($C_0$-$C_4$)-alkylene or the radical -B1-B2-B3- in which

B1 is -$(CH_2)_n$-, in which n is the integer zero, 1 or 2,

B3 is -$(CH_2)_m$-, in which m is the integer zero, 1 or 2,

with the proviso that the sum of n and m amounts to zero, 1 or 2, and

B2 is

1) -C(O)-
2) -($C_2$-$C_4$)-alkenylene,
3) -$S(O)_o$-, where o is the integers zero, 1 or 2,
4) -N(R6)-, in which R6 is hydrogen atom, methyl or ethyl,
5) -N(R6)-C(Y)-, in which Y is oxygen atom or sulfur atom and R6 is defined as above,
6) -C(Y)-N(R6)-, in which Y is oxygen atom or sulfur atom and R6 is defined as above,
7) -N(R6)-$SO_2$-, in which R6 is defined as above,
8) -$SO_2$-N(R6)- in which R6 is defined as above,
9) -N(R6)-$SO_2$-N(R6)-, in which R6 is defined as above,
10) -N(R6)-C(Y)-N(R6)-, in which Y is oxygen atom or sulfur atom and R6 is defined as above,
11) -O-C(O)-N(R6)-,
12) -NH-C(O)-O-,
13) -O-,
14) -C(O)-O-,
15) -O-C(O)-,
16) -O-C(O)-O-,
17) -O-$CH_2$-C(O)- ,
18) -O-$CH_2$-C(O)-O-,
19) -O-$CH_2$-C(O)-N(R6)-, in which R6 is defined as above,
20) -C(O)-CH2-$_O$-,
21) -O-C(O)-$CH_2$-O-,
22) -N(R6)-C(O)-$CH_2$-O-, in which R6 is defined as above,
23) -O-$(CH_2)_n$-O-, in which n is the integer 2 or 3, or
24) -O-$(CH_2)_m$-N(R6)-, in which m is the integer 2 or 3 and R6 is defined as above,
25) -N(R6)-$(CH_2)_m$-O- , in which m is the integer 2 or 3 and R6 is defined as above,
26) -N(R6)-N(R6)-, in which R6 is defined as above,
27) -N=N-,
28) -N(R6)-CH=N-, in which R6 is defined as above,
29) -N=CH-N(R6)-, in which R6 is defined as above,
30) -N(R6)-C(R7)=N-, in which R6 is defined as above and R7 is -NH-R6,
31) -N=C(R7)-N(R6)-, in which R6 is defined as above and R7 is -NH-R6, or
32) -($C_2$-$C_6$)-alkynylene,

ring1, ring2 and ring3 are identical or different and are, independently of each other,

1) covalent bond,
2) -($C_6$-$C_{14}$)-aryl, in which aryl is unsubstituted or substituted, independently of each other, once, twice or three times, by G, or
3) 4- to 15-membered Het ring, in which Het ring is unsubstituted or substituted, independently of each other, once, twice or three times, by G,

ring4 is

1) -($C_6$-$C_{14}$)-aryl, in which aryl is unsubstituted or substituted, independently of each other, once, twice or three times, by G,
2) 4- to 15-membered Het ring, in which the Het ring is unsubstituted or substituted, independently of each other, once, twice or three times, by G, or
3) is one of the following radicals

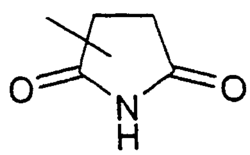
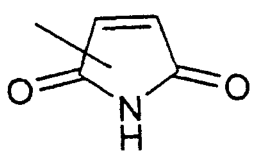

and these radicals are unsubstituted or substituted once by G,

G is

1) hydrogen atom,
2) halogen,
3) =O,
4) -($C_1$-$C_6$)-alkyl, in which alkyl is unsubstituted or substituted, once, twice or three times, by halogen,

- ($C_3$-$C_6$)-cycloalkyl, -($C_2$-$C_6$)-alkynyl,
- ($C_6$-$C_{14}$)-aryl or Het ring,

5) -($C_6$-$C_{14}$)-aryl ,
6) Het ring,
7) -C(O)-O-R10, in which R10 is

a) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, or
b) -($C_6$-$C_{14}$)-aryl or Het ring,

8) -C(S)-O-R10, in which R10 is defined as above,
9) -C(O)-NH-R11, in which R11 is

a) -($C_1$-$C_6$)-alkyl, in which alkyl is unsubstituted or substituted, once or twice, by -($C_3$-$C_6$)-cycloalkyl, -($C_6$-$C_{14}$)-aryl or Het ring, or
b) -($C_6$-$C_{14}$)-aryl or Het ring,

10) -C(S)-NH-R11, in which R11 is defined as above,
11) -O-R12, in which R12 is

a) hydrogen atom,
b) -($C_1$-$C_6$)-alkyl, in which alkyl is unsubstituted or substituted, once, twice or three times, by halogen, -($C_3$-$C_6$)-cycloalkyl, -($C_2$-$C_6$)-alkynyl, -($C_6$-$C_{14}$)-aryl or Het ring,
c) -($C_6$-$C_{14}$)-aryl,
d) Het ring,

e) -C(O)-O-R13, in which R13 is

e) 1) -($C_1$-$C_6$)-alkyl, in which alkyl is unsubstituted or substituted, once or twice, by - ($C_3$-$C_6$)-cycloalkyl, -($C_2$-$C_6$)-alkynyl, -($C_6$-$C_{14}$)-aryl, or Het ring, or
e)2) -($C_6$-$C_{14}$)-aryl or Het ring,

f) -C(S)-O-R13, in which R13 is defined as above,
g) -C(O)-NH-R14, in which R14 is

g)1) -($C_1$-$C_6$)-alkyl, in which alkyl is unsubstituted or substituted, once or twice, by - ($C_3$-$C_6$) -cycloalkyl, -($C_2$-$C_6$)-alkynyl, -($C_6$-$C_{14}$)-aryl or Het ring, or
g) 2) -($C_6$-$C_{14}$)-aryl or Het ring, or

h) -C(S)-NH-R14, in which R14 is defined as above,

12) -C(O)-R10, in which R10 is defined as above,
13) -$S(O)_p$-R12, in which R12 is defined as above and p is the integers zero, 1 or 2,
14) -$NO_2$,
15) -CN,
16) -N(R15)-R12, in which R15 is

16)1) hydrogen atom,
16)2) -($C_1$-$C_6$)-alkyl, or
16)3) -$SO_2$-($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, and R12 is defined as above, or

17) -$SO_2$-N (R12)-R1, in which R12 is defined as above and R1 is defined as below,

X is -OH or -NH-OH,
n1 is the integer 2,
n2 is the integer 3,
R1, R2, R3, R4 and R5 are identical or different and are, independently of each other,

1) hydrogen atom,
2) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring,
3) -C(O)-O-R8, in which R8 is

3)1) hydrogen atom,
3)2) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, or substituted once to five times, by fluorine, or
3)3) -($C_6$-$C_{14}$)-aryl or Het ring,

4) -O-R8, in which R8 has the abovementioned meaning, or
5) -($C_3$-$C_6$)-cycloalkyl.

**2.** Compound of the formula I according to Claim 1,
wherein
A is - ($C_0$-$C_4$)-alkylene,
B, D and E are identical or different and are, independently of each other, -($C_0$-$C_4$)-alkylene or the radical -B1-B2-B3- in which
B1 is -$(CH_2)_n$-, in which n is the integer zero, 1 or 2,
B3 is -$(CH_2)_m$-, in which m is the integer zero, 1 or 2,
with the proviso that the sum of n and m amounts to zero, 1 or 2, and
B2 is

1) -C(O)-
2) - ($C_2$-$C_4$)-alkenylene,

3) $-S(O)_o-$, where o is the integers zero, 1 or 2,
4) -N(R6)-, in which R6 is hydrogen atom, methyl or ethyl,
5) -N(R6)-C(Y)-, in which Y is oxygen atom or sulfur atom and R6 is defined as above,
6) -C(Y)-N(R6)-, in which Y is oxygen atom or sulfur atom and R6 is defined as above,
7) $-N(R6)-SO_2-$, in which R6 is defined as above,
8) $-SO_2-N(R6)-$, in which R6 is defined as above,
9) $-N(R6)-SO_2-N(R6)-$, in which R6 is defined as above,
10) -N(R6)-C(Y)-N(R6)-, in which Y is oxygen atom or sulfur atom and R6 is defined as above,
11) -O-C(O)-N(R6)-,
12) -NH-C(O)-O-,
13) -O-,
14) -C(O)-O-,
15) -O-C(O)-,
16) -O-C(O)-O-,
17) -O-CH2-C(O)-,
18) -O-CH2-C(O)-O-,
19) $-O-CH_2-C(O)-N(R6)-$, in which R6 is defined as above,
20) $-C(O)-CH_2-O-$,
21) $-O-C(O)-CH_2-O-$,
22) $-N(R6)-C(O)-CH_2-O-$, in which R6 is defined as above,
23) $-O-(CH_2)_n-O-$, in which n is the integer 2 or 3, or
24) $-O-(CH_2)_m-N(R6)-$, in which m is the integer 2 or 3 and R6 is defined as above,
25) $-N(R6)-(CH_2)_m-O-$, in which m is the integer 2 or 3 and R6 is defined as above,
26) -N(R6)-N(R6)-, in which R6 is defined as above,
27) -N=N-,
28) -N(R6)-CH=N-, in which R6 is defined as above,
29) -N=CH-N(R6)-, in which R6 is defined as above,
30) -N(R6)-C(R7)=N-, in which R6 is defined as above and R7 is -NH-R6,
31) -N=C(R7)-N(R6)-, in which R6 is defined as above and R7 is -NH-R6, or
32) $-(C_2-C_6)$-alkynylene,

ring1, ring2 and ring3 are identical or different and are, independently of each other,

1) covalent bond,
2) phenyl or naphthyl and are unsubstituted or substituted, independently of each other, once, twice or three times, by G, or
3) 4- to 15-membered Het ring, in which the Het ring is a radical from the series acridinyl, azepinyl, azetidinyl, aziridinyl, benzimidazalinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, decahydroquinolinyl, dibenzofuranyl, dibenzothiophenyl, dihydrofuran[2,3-b]tetrahydrofuranyl, dihydrofuranyl, dioxolyl, dioxanyl, 2H,6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyroazolidinyl, pyrazolinyl, pyrazolyl, pyridazinyl, pyridooxazolyl, pyridoimidazolyl, pyridothiazolyl, pyridothiophenyl, pyridinyl,
pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiomorpholinyl, thiophenyl, triazinyl, 1,2,3-triazolyl, 1,2,4-triazolyl, 1,2,5-triazolyl, 1,3,4-triazolyl and xanthenyl, and these radicals are unsubstituted or substituted, independently of each other, once, twice or three times, by G,

ring4 is

1) $-(C_6-C_{14})$-aryl, in which aryl is a radical from the series phenyl, naphthyl, 1-naphthyl, 2-naphthyl, anthryl and

fluorenyl, and these radicals are unsubstituted or substituted, independently of each other, once, twice or three times, by G,

2) 4- to 15-membered Het ring, in which the Het ring is a radical from the series acridinyl, azepinyl, azetidinyl, aziridinyl, benzimidazalinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, decahydroquinolinyl, dibenzofuranyl, dibenzothiophenyl, dihydrofuran[2,3-b]tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolidinyl, 2-isothiazolinyl, isothiazolyl, isoxazolyl, isoxazolidinyl, 2-isoxazolinyl, 2'-methylbiphenyl-2-ol, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyroazolidinyl, pyrazolinyl, pyrazolyl, pyridazinyl, pyridooxazolyl, pyridoimidazolyl, pyridothiazolyl, pyridothiophenyl, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiomorpholinyl, thiophenyl, triazinyl, 1,2,3-triazolyl, 1,2,4-triazolyl, 1,2,5-triazolyl, 1,3,4-triazolyl and xanthenyl, and are unsubstituted or substituted, independently of each other, once, twice or three times, by G, or

3) is one of the following radicals

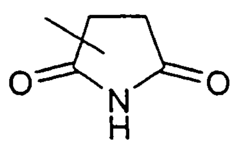 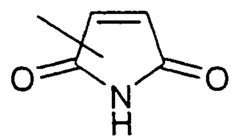

and these radicals are unsubstituted or substituted once by G,

G is

1) hydrogen atom,
2) halogen,
3) =O,
4) -($C_1$-$C_6$)-alkyl, in which alkyl is unsubstituted or substituted, once, twice or three times, by halogen, -($C_3$-$C_6$)-cycloalkyl, -($C_2$-$C_6$)-alkynyl, -($C_6$-$C_{14}$)-aryl or Het ring, where aryl and Het ring are defined as above,
5) -($C_6$-$C_{14}$)-aryl, where aryl is defined as above,
6) Het ring, where Het ring is defined as above,
7) -C(O)-O-R10, in which R10 is

a) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, where aryl and Het ring are defined as above, or
b) -($C_6$-$C_{14}$)-aryl or Het ring, where aryl and Het ring are defined as above,

8) -C(S)-O-R10, where R10 is defined as above,
9) -C(O)-NH-R11, in which R11 is

a) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, where aryl and Het ring are defined as above, or
b) -($C_6$-$C_{14}$)-aryl or Het ring, where aryl and Het ring are defined as above,

10) -C(S)-NH-R11, in which R11 is defined as above,
11) -O-R12, in which R12 is

a) hydrogen atom,
b) -($C_1$-$C_6$)-alkyl, in which alkyl is unsubstituted or substituted, once, twice or three times, by halogen, -($C_3$-$C_6$)-cycloalkyl, -($C_2$-$C_6$)-alkynyl, -($C_6$-$C_{14}$)-aryl or Het ring, where aryl and Het ring are defined as above,

c) -($C_6$-$C_{14}$)-aryl, where aryl is defined as above,
d) Het ring, where Het ring is defined as above,
e) -C(O)-O-R13, in which R13 is

e)1) -($C_1$-$C_6$)-alkyl, in which alkyl is unsubstituted or substituted, once or twice, by - ($C_3$-$C_6$)-cycloalkyl, -($C_2$-$C_6$)-alkynyl, -($C_6$-$C_{14}$)-aryl, or Het ring, where aryl and Het ring are defined as above, or
e)2) -($C_6$-$C_{14}$)-aryl or Het ring, where aryl and Het ring are defined as above,

f) -C(S)-O-R13, in which R13 is defined as above,
g) -C(O)-NH-R14, in which R14 is

g) 1) - ($C_1$-$C_6$)-alkyl, in which alkyl is unsubstituted or substituted, once or twice, by - ($C_3$-$C_6$)-cycloalkyl, - ($C_2$-$C_6$)-alkynyl, -($C_6$-$C_{14}$)-aryl or Het ring, where aryl and Het ring are defined as above, or
g)2) -($C_6$-$C_{14}$)-aryl or Het ring, where aryl and Het ring are defined as above, or

h) -C(S)-NH-R14, in which R14 is defined as above,

12) -C(O)-R10, in which R10 is defined as above,
13) -$S(O)_p$-R12, in which R12 is defined as above and p is the integers zero, 1 or 2,
14) -$NO_2$,
15) -CN, or
16) -N(R15)-R12, in which R15 is

16)1) hydrogen atom,
16)2) -($C_1$-$C_6$)-alkyl or
16)3) -$SO_2$-($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, and R12 is defined as above,

17) -$SO_2$-N (R12)-R1, in which R12 is defined as above and R1 is defined as below,

X is -OH or -NH-OH,
n1 is the integer 2,
n2 is the integer 3,
R1, R2, R3, R4 and R5 are identical or different and are, independently of each other,

1) hydrogen atom,
2) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring,
3) -C(O)-O-R8, in which R8 is

3)1) hydrogen atom,
3)2) -($C_1$-$C_6$)-alkyl, in which alkyl is unsubstituted or substituted, once or twice, by -($C_3$-$C_6$)-cycloalkyl, -($C_2$-$C_6$)-alkynyl, -($C_6$-$C_{14}$)-aryl or Het ring, or is substituted once to five times by fluorine, or
3)3) -($C_6$-$C_{14}$)-aryl or Het ring,

4) -O-R8, in which R8 has the abovementioned meaning, or
5) -($C_3$-$C_6$)-cycloalkyl.

**3.** Compound of the formula I according to Claim 1 or 2, wherein
A is -($C_0$-$C_4$)-alkylene,
B, D and E are identical or different and are, independently of each other, -($C_0$-$C_4$)-alkylene or the radical -B1-B2-B3- in which
B1 is -$(CH_2)_n$-, in which n is the integer zero, 1 or 2,
B3 is -$(CH_2)_m$-, in which m is the integer zero, 1 or 2,
with the proviso that the sum of n and m amounts to zero, 1 or 2, and
B2 is

1) -($C_0$-$C_2$)-alkylene,

2) ethenylene,
3) ethynylene,
4) -C(O)-
5) -N(R6)-C(O)-, in which R6 is hydrogen atom, methyl or ethyl,
6) -C(O)-N(R6)-, in which R6 is defined as above,
7) -O-, or
8) -S-,

ring1, ring2 and ring3 are identical or different and are, independently of each other,

1) covalent bond,
2) phenyl or naphthyl and are unsubstituted or substituted, independently of each other, once or twice, by G, or
3) Het ring, in which the Het ring is a radical from the series dihydrofuranyl, furanyl, pyridinyl, pyrimidinyl, pyrrolyl, thiadiazolyl, thiazolyl or thiophenyl, and the radicals are unsubstituted or substituted, independently of each other, once or twice, by G,

ring4 is

1) phenyl or naphthyl and is unsubstituted or substituted, independently of each other, once or twice, by G,
2) Het ring, in which the Het ring is a radical from the series benzofuranyl, dihydrofuranyl, dibenzofuranyl, dibenzothiophenyl, furanyl, 2'-methylbiphenyl-2-ol, morpholinyl, piperazinyl, piperidinyl, pyridinyl, pyrimidinyl, pyridothiophenyl, pyrrolyl, pyrrolidinyl, thiazolyl or thiophenyl and is unsubstituted or substituted, independently of each other, once or twice, by G, or
3) the following radical

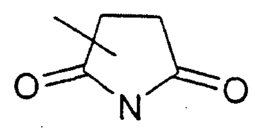

and this radical is unsubstituted or substituted once by G,

G is

1) hydrogen atom,
2) Br, Cl or F,
3) -($C_1$-$C_4$)-alkyl, in which alkyl is unsubstituted or substituted once or twice by F, phenyl, -$C_3$-cycloalkyl or Het ring, where Het ring is defined as above,
4) phenyl,
5) Het ring, where Het ring is defined as above,
6) -C(O)-O-R10, in which R10 is

a) -($C_1$-$C_6$)-alkyl, in which alkyl is unsubstituted or substituted, once or twice, by cyclopropyl, phenyl or Het ring, where Het ring is defined as above,
b) phenyl, or
c) Het ring, where Het ring is defined as above,

7) -C(O)-NH-R11, in which R11 is

a) -($C_1$-$C_6$)-alkyl, in which alkyl is unsubstituted or substituted, once or twice, by cyclopropyl, phenyl or Het ring, where Het ring is defined as above,
b) phenyl, or
c) Het ring, where Het ring is defined as above,

8) -O-R12, in which R12 is

a) hydrogen atom,

b) -($C_1$-$C_6$)-alkyl, in which alkyl is unsubstituted or substituted, once, twice or three times, by halogen, cyclopropyl, phenyl or Het ring, where Het ring is defined as above,
c) phenyl,
d) Het ring, where Het ring is defined as above,
e) -C(O)-O-R13, in which R13 is

e)1) -($C_1$-$C_6$)-alkyl, in which alkyl is unsubstituted or substituted, once or twice, by cyclopropyl, phenyl or Het ring, where Het ring is defined as above, or
e)2) phenyl or Het ring, where Het ring is defined as above,

f) -C(S)-O-R13, in which R13 is defined as above, or
g) -C(O)-NH-R14, in which R14 is

g)1) -($C_1$-$C_6$)-alkyl, in which alkyl is unsubstituted or substituted, once or twice, by phenyl or Het ring, where Het ring is defined as above, or
g)2) phenyl or Het ring, where Het ring is defined as above,

9) -C(O)-R10, in which R10 is defined as above,
10) -$S(O)_p$-R12, in which R12 is defined as above and p is the integers 1 or 2,
11) -$NO_2$,
12) -CN, or
13) -N(R15)-R12, in which R15 is

13)1) hydrogen atom, or
13)2) -($C_1$-$C_6$)-alkyl and R12 is defined as above,

X is -OH or -NH-OH,
n1 is the integer 2,
n2 is the integer 3,
R1, R2 and R3 are in each case hydrogen atom,
R4 and R5 are identical or different and are, independently of each other,

1) hydrogen atom,
2) methyl,
3) ethyl, or
4) -OH.

**4.** Compound of the formula I according to one or more of Claims 1 to 3, wherein
A is -($C_0$-$C_4$)-alkylene,
B, D and E are identical or different and are, independently of each other, -($C_0$-$C_4$)-alkylene or the radical -B1-B2-B3- in which
B1 is -$(CH_2)_n$-, in which n is the integer zero, 1 or 2,
B3 is -$(CH_2)_m$-, in which m is the integer zero, 1 or 2,
with the proviso that the sum of n and m amounts to zero, 1 or 2, and
B2 is

1) -($C_0$-$C_2$)-alkylene,
2) ethenylene, or
3) ethynylene,

ring1, ring2 and ring3 are identical or different and are, independently of each other,

1) covalent bond,
2) phenyl, and are unsubstituted or substituted, independently of each other, once or twice, by G, or
3) Het ring, in which the Het ring is a radical from the series dihydrofuranyl, furanyl, morpholinyl, piperazinyl, piperidinyl, pyridinyl, pyrimidinyl, pyrrolyl, thiazolyl or thiophenyl, and are unsubstituted or substituted, independently of each other, once or twice, by G,

ring4 is

1) phenyl, and is unsubstituted or substituted, independently of each other, once or twice, by G,
2) Het ring, in which the Het ring is a radical from the series benzofuranyl, dihydrofuranyl, dibenzofuranyl, dibenzothiophenyl, furanyl, 2'-methylbiphenyl-2-ol, morpholinyl, piperazinyl, piperidinyl, pyridinyl, pyrimidinyl, pyridothiophenyl, pyrrolyl, pyrrolidinyl, thiazolyl or thiophenyl and is unsubstituted or substituted, independently of each other, once or twice, by G, or
3) the following radical

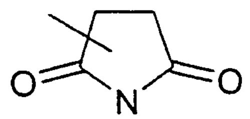

and this radical is unsubstituted or substituted once by G,

G is

1) hydrogen atom,
2) Br, Cl or F,
3) -($C_1$-$C_4$)-alkyl, in which alkyl is unsubstituted or substituted once, twice or three times, by Br, Cl, F, -$C_3$-cycloalkyl, phenyl or Het ring, where Het ring is defined as above,
4) phenyl,
5) Het ring, where Het ring is defined as above,
6) -C(O)-O-R10, in which R10 is

a) -($C_1$-$C_6$)-alkyl, in which alkyl is unsubstituted or substituted, once or twice, by cyclopropyl, phenyl or Het ring, where Het ring is defined as above,
b) phenyl, or
c) Het ring, where Het ring is defined as above,

7) -C(O)-NH-R11, in which R11 is

a) -($C_1$-$C_6$)-alkyl, in which alkyl is unsubstituted or substituted, once or twice, by cyclopropyl, phenyl or Het ring, where Het ring is defined as above,
b) phenyl or naphthyl, or
c) Het ring, where Het ring is defined as above,

8) -O-R12, in which R12 is

a) hydrogen atom,
b) -($C_1$-$C_6$)-alkyl, in which alkyl is unsubstituted or substituted, once, twice or three times, by halogen, cyclopropyl, phenyl or Het ring, where Het ring is defined as above,
c) phenyl,
d) Het ring, where Het ring is defined as above,
e) -C(O)-O-R13, in which R13 is

e)1) -($C_1$-$C_6$)-alkyl, in which alkyl is unsubstituted or substituted, once or twice, by cyclopropyl, phenyl, naphthyl, or Het ring, where Het ring is defined as above, or
e)2) phenyl or Het ring, where Het ring is defined as above,

f) -C(S)-O-R13, in which R13 is defined as above, or
g) -C(O)-NH-R14, in which R14 is

g)1) -($C_1$-$C_6$)-alkyl, in which alkyl is unsubstituted or substituted, once or twice, by phenyl or Het ring, where Het ring is defined as above, or
g)2) phenyl or Het ring, where Het ring is defined as above,

9) -C(O)-R10, in which R10 is defined as above,
10) $-S(O)_p$-R12, in which R12 is defined as above and p is the integers 1 or 2,
11) $-NO_2$,
12) -CN, or
13) -N(R15)-R12, in which R15 is

13)1) hydrogen atom, or
13)2) $-(C_1-C_6)$-alkyl and R12 is defined as above,

X is -OH or -NH-OH,
n1 is the integer 2,
n2 is the integer 3,
R1, R2, R3, R4 and R5 are in each case hydrogen atom.

**5.** Compound of the formula I according to one or more of Claims 1 to 4, wherein
A is a covalent bond or $-CH_2-CH_2-$,
B, D and E are identical or different and are, independently of each other, $-(C_0-C_4)$-alkylene or the radical -B1-B2-B3- in which
B1 is $-(CH_2)_n-$, in which n is the integer zero, 1 or 2,
B3 is $-(CH_2)_m-$, in which m is the integer zero, 1 or 2,
with the proviso that the sum of n and m amounts to zero, 1 or 2, and
B2 is

1) -C(O)-
2) $-(C_2-C_4)$-alkynylene,
3) $-S(O)_o-$, where o is the integers zero or 1,
4) -N(R6)-C(Y)-, in which Y is oxygen atom and R6 is hydrogen atom,
5) -C(Y)-N(R6)-, in which Y is oxygen atom and R6 is hydrogen atom, or
6) -O-,

ring1, ring2 and ring3 are identical or different and are, independently of each other,

1) covalent bond,
2) phenyl and are unsubstituted or substituted, independently of each other, once or twice, by G, or
3) Het ring, in which the Het ring is a radical from the series furanyl, pyridinyl, pyrimidinyl or thiophenyl, and are unsubstituted or substituted, independently of each other, once or twice, by G,

ring4 is

1) phenyl and is unsubstituted or substituted, independently of each other, once or twice, by G,
2) Het ring, in which the Het ring is a radical from the series benzofuranyl, dibenzofuranyl, furanyl, 2'-methylbiphenyl-2-ol, morpholinyl, piperazinyl, piperidinyl, pyridinyl, pyrimidinyl, pyridothiophenyl, pyrrolyl, pyrrolidinyl, thiazolyl or thiophenyl and is unsubstituted or substituted, independently of each other, once or twice, by G, or
3) the following radical

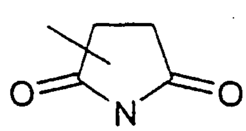

and this radical is unsubstituted or substituted once by G,

G is

1) hydrogen atom,
2) Br, Cl or F,
3) $-(C_1-C_4)$-alkyl, in which alkyl is unsubstituted or substituted once, twice or three times, by Br, Cl, F, $-C_3$-cy-

cloalkyl, phenyl or Het ring, where Het ring is defined as above,
4) phenyl,
5) Het ring, where Het ring is defined as above,
6) -C(O)-O-R10, in which R10 is

a) -($C_1$-$C_6$)-alkyl, in which alkyl is unsubstituted or substituted, once or twice, by cyclopropyl, phenyl or Het ring, where Het ring is defined as above,
b) phenyl, or
c) Het ring, where Het ring is defined as above,

7) -C(O)-NH-R11, in which R11 is

a) -($C_1$-$C_6$)-alkyl, in which alkyl is unsubstituted or substituted, once or twice, by cyclopropyl, phenyl or Het ring, where Het ring is defined as above,
b) phenyl, or
c) Het ring, where Het ring is defined as above,

8) -O-R12, in which R12 is

a) hydrogen atom,
b) -($C_1$-$C_6$)-alkyl, in which alkyl is unsubstituted or substituted, once,
twice or three times, by halogen, cyclopropyl, phenyl or Het ring, where Het ring is defined as above,
c) phenyl,
d) Het ring, where Het ring is defined as above,
e) -C(O)-O-R13, in which R13 is

e)1) -($C_1$-$C_6$)-alkyl, in which alkyl is unsubstituted or substituted, once or twice, by cyclopropyl, phenyl or Het ring, where Het ring is defined as above, or
e)2) phenyl or Het ring, where Het ring is defined as above,

f) -C(S)-O-R13, in which R13 is defined as above, or
g) -C(O)-NH-R14, in which R14 is

g)1) -($C_1$-$C_6$)-alkyl, in which alkyl is unsubstituted or substituted, once or twice, by phenyl or Het ring, where Het ring is defined as above, or
g)2) phenyl or Het ring, where Het ring is defined as above,

9) -C(O)-R10, in which R10 is defined as above,
10) -$S(O)_p$-R12, in which R12 is defined as above and p is the integers zero, 1 or 2,
11) -$NO_2$,
12) -CN, or
13) -N(R15)-R12, in which R15 is

13)1) hydrogen atom, or
13)2) -($C_1$-$C_6$)-alkyl and R12 is defined as above,

X is -NH-OH,
n1 is the integer 2,
n2 is the integer 3, and
R1, R2, R3, R4 and R5 are in each case hydrogen atom.

**6.** Compound of the formula I according to one or more of Claims 1 to 5, which is a compound from the series
2-(4'-nitrobiphenyl-4-sulfonyl)decahydroisoquinoline-1-(N-hydroxy)carboxamide,
2-(4'-chlorobiphenyl-4-sulfonyl)decahydroisoquinoline-1-carboxylic acid,
2-(4'-chlorobiphenyl-4-sulfonyl)decahydroisoquinoline-1-(N-hydroxy)carboxamide,
2-(6-phenoxypyridine-3-sulfonyl)decahydroisoquinoline-1-carboxylic acid; trifluoroacetate,
2-(6-phenoxypyridine-3-sulfonyl)decahydroisoquinoline-1-(N-hydroxy)carboxamide; trifluoroacetate,
2-[2-(4'-chlorobiphenyl-4-yl)ethanesulfonyl]decahydroisoquinoline-1-carboxylic acid,

2-[2-(4'-chlorobiphenyl-4-yl)ethanesulfonyl]decahydroisoquinoline-1-(N-hydroxy)carboxamide,
2-[4-(pyridin-4-yloxy)benzenesulfonyl]decahydroisoquinoline-1-carboxylic acid; trifluoroacetate,
2-[4-(pyridin-4-yloxy)benzenesulfonyl]decahydroisoquinoline-1-(N-hydroxy)carboxamide; trifluoroacetate,
2-[4-(4-methoxyphenoxy)benzenesulfonyl]decahydroisoquinoli ne-1-carboxylic acid,
2-[4-(4-methoxyphenoxy)benzenesulfonyl]decahydroisoquinoli ne-1-(N-hydroxy)carboxamide,
2-{4-[4-(2,2,2-trifluoroethoxy)phenoxy]benzenesulfonyl}decahydro-isoquinoline-1-carboxylic acid,
2-{4-[4-(2,2,2-trifluoroethoxy)phenoxy]benzenesulfonyl}decahydro-isoquinoline-1-(N-hydroxy)carboxamide,
2-[4'-(2,2,2-trifluoroethoxy)biphenyl-4-sulfonyl]decahydroisoquinoline-1-carboxylic acid,
2-(4'-isopropoxycarbonylaminobiphenyl-4-sulfonyl)decahydroisoquinoline-1-carboxylic acid,
[4'-(1-hydroxycarbamoyloctahydroisoquinoline-2-sulfonyl)biphenyl-4-yl]carboxamide isopropyl ester,
2-[4'-(2,2,2-trifluoroethoxy)biphenyl-4-sulfonyl]decahydroisoquinoline-1-(N-hydroxy)carboxamide,
2-(4'-trifluoromethoxybiphenyl-4-sulfonyl)decahydroisoquinoline-1-(N-hydroxy)carboxamide,
2-[4-(4-fluorophenoxy)benzenesulfonyl]decahydroisoquinolin e-1-(N-hydroxy)carboxamide,
2-[4-(4-trifluoromethoxyphenoxy)benzenesulfonyl]decahydroi soquinoline-1-(N-hydroxy)carboxamide,
2-[4-(4-trifluoromethoxyphenoxy)benzenesulfonyl]decahydroi soquinoline-1-carboxylic acid,
2-(biphenyl-4-sulfonyl)decahydroisoquinoline-1-(N-hydroxy)carboxamide,
2-(biphenyl-4-sulfonyl)decahydroisoquinoline-1-carboxylic acid,
2-[4-(4-cyanophenoxy)benzenesulfonyl]decahydroisoquinoline -1-(N-hydroxy)carboxamide,
2-(dibenzofuran-2-sulfonyl)decahydroisoquinoline-1-carboxylic acid,
2-(dibenzofuran-2-sulfonyl)decahydroisoquinoline-1-(N-hydroxy)carboxamide, or
2-[4-(4-fluorophenoxy)benzenesulfonyl]-6-methoxydecahydroisoquinoline-1-(N-hydroxy)carboxamide, and also all the stereoisomeric forms of the abovementioned compounds.

**7.** Process for preparing the compound of the formula I according to one or more of Claims 1 to 6, **characterized in that**

a) a compound of the formula IV,

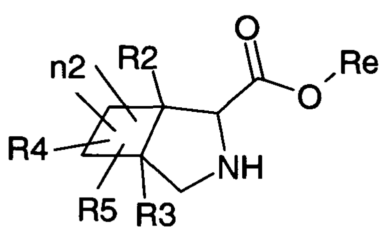

**(IV)**

in which Re is a hydrogen atom or an ester-protecting group,
is reacted with a compound of the formula V,

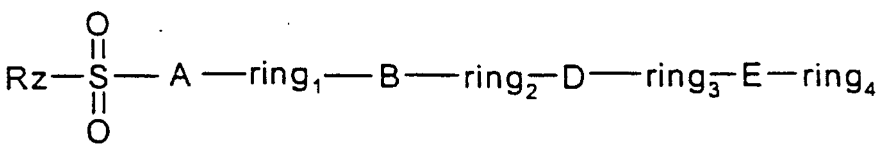

(V)

in which A, B, D, E and ring1, ring2, ring3 and ring4 are defined as in formula I, and in which Rz is chlorine atom, imidazoyl or OH,
in the presence of a base or following silylation with a suitable silylating agent, or using a suitable dehydrating agent when Rz = OH, to give a compound of the formula VI,

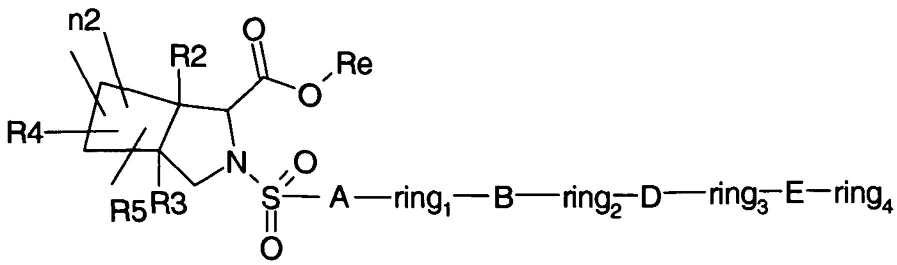

(VI)

in which A, B, D, E, Re and ring1, ring2, ring3 and ring4 are defined as above, or

b) when Re = ester a compound of the formula VI prepared as described in a) is reacted with a solution of alkali such as NaOH or LiOH, and then the product is treated with acid, to give the carboxylic acid according to the invention of the formula I, in which X = OH (corresponding to VII), with modifications in one of the side chains of the rings ringl-ring4 also having previously been made, where appropriate; or said ester is converted, by treating it with a mineral acid, such as hydrochloric acid, into the free carboxylic acid VII

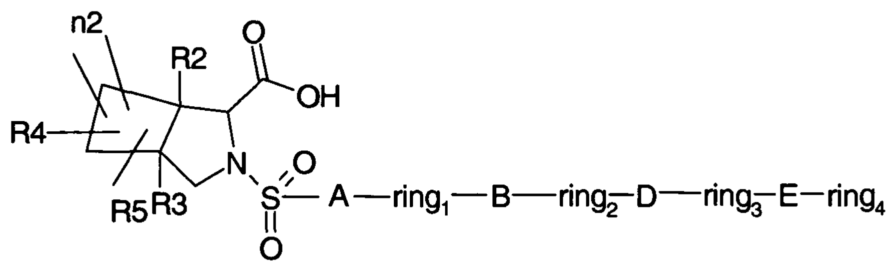

(VII)

and then this is converted into the hydroxamic acid according to the invention, in which X = NH-OH, of the formula I, or

c) salt formation with enantiomerically pure acids or bases, chromatography on chiral stationary phases, or derivatization with chiral, enantiomerically pure compounds such as amino acids, separation of the resulting diastereomers and elimination of the chiral auxiliary groups, are used to separate a compound of formula I prepared as described in procedure a), or a suitable precursor of the formula I, which arises in enantiomeric forms due to its chemical structure, into the pure enantiomers, or

d) either the compound of the formula I prepared as described in procedures b) or c) is isolated in free form, or, when acid or basic groups are present, it is converted into physiologically tolerated salts.

**8.** Pharmaceutical, **characterized by** an effective content of at least one compound of the formula I according to one or more of Claims 1 to 6 together with a pharmaceutically suitable and physiologically tolerated carrier substance, additive and/or auxiliary substance.

**9.** Use of the compound of formula I according to one or more of Claims 1 to 6 for producing a pharmaceutical for the prophylaxis and therapy of degenerative joint diseases such as osteoarthroses, spondyloses and chondrolysis following joint trauma or a relatively long period of joint immobilization following meniscus injuries or patella injuries or ligament ruptures, diseases of the connective tissue such as collagenoses, periodontal diseases, wound healing disturbances and chronic diseases of the locomotory apparatus such as inflammatory, immunologically determined or metabolism-determined acute and chronic arthritides, arthropathies, myalgias and disturbances of bone metabolism, for the treatment of ulceration, atherosclerosis and stenoses, for the treatment of inflammations, cancer diseases, tumor metastases formation, cachexia, anorexia, heart failure and septic shock, or for the prophylaxis of myocardial and cerebral infarctions.

**Revendications**

**1.** Composé de formule I

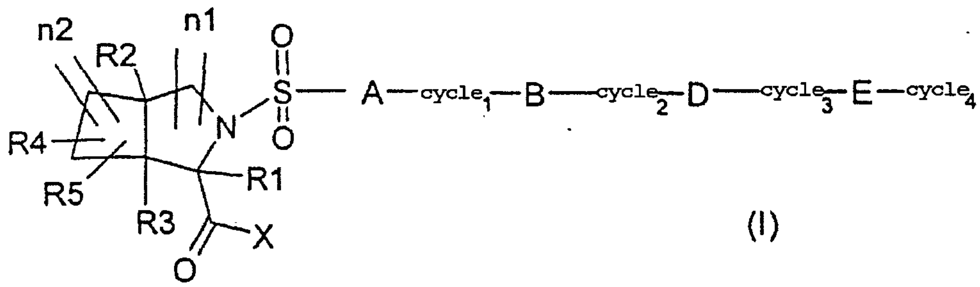

(I)

et/ou toutes les formes stéréoisomères du composé de formule I et/ou les mélanges de ces formes dans un rapport quelconque, et/ou un sel physiologiquement tolérable du composé de formule I, dans laquelle
A est - ($C_0$-$C_4$)-alkylène,
B, D et E sont identiques ou différents et sont, indépendamment les uns des autres, -($C_0$-$C_4$)-alkylène ou le radical -B1-B2-B3- dans lequel
B1 est $-(CH_2)_n-$, dans lequel n est l'entier zéro, 1 ou 2,
B3 est $-(CH_2)_m-$, dans lequel m est l'entier zéro, 1 ou 2,
à condition que la somme de n et de m vaille zéro, 1 ou 2, et
B2 est

1) -C(O)-
2) -($C_2$-$C_4$)-alcénylène,
3) $-S(O)_o-$, où o est l'entier zéro, 1 ou 2,
4) -N(R6)-, dans lequel R6 est un atome d'hydrogène, méthyle ou éthyle,
5) -N(R6)-C(Y)-, dans lequel Y est un atome d'oxygène ou un atome de soufre et R6 est tel que défini ci-dessus,
6) -C(Y)-N(R6)-, dans lequel Y est un atome d'oxygène ou un atome de soufre et R6 est tel que défini ci-dessus,
7) -N(R6)-$SO_2$-, dans lequel R6 est tel que défini ci-dessus,
8) -$SO_2$-N(R6)-, dans lequel R6 est tel que défini ci-dessus,
9) -N(R6)-$SO_2$-N(R6)-, dans lequel R6 est tel que défini ci-dessus,
10) -N(R6)-C(Y)-N(R6)-, dans lequel Y est un atome d'oxygène ou un atome de soufre et R6 est tel que défini ci-dessus,
11) -O-C(O)-N(R6)-,
12) -NH-C(O)-O-,
13) -O-,
14) -C(O)-O-,
15) -O-C(O)-,
16) -O-C(O)-O-,
17) -O-CH2-C(O)-,
18) -O-CH2-C(O)-O-,
19) -O-$CH_2$-C(O)-N(R6)-, dans lequel R6 est tel que défini ci-dessus,
20) -C(O)-$CH_2$-O-,
21) -O-C(O)-CH2-O-,
22) -N(R6)-C(O)-CH2-O-, dans lequel R6 est tel que défini ci-dessus,
23) -O-(CH2)n-O-, dans lequel n est l'entier 2 ou 3, ou
24) -O-(CH2)m-N(R6)-, dans lequel m est l'entier 2 ou 3 et R6 est tel que défini ci-dessus,
25) -N(R6)-(CH2)m-O-, dans lequel m est l'entier 2 ou 3 et R6 est tel que défini ci-dessus,
26) -N(R6)-N(R6)-, dans lequel R6 est tel que défini ci-dessus,
27) -N=N-,
28) -N(R6)-CH=N-, dans lequel R6 est tel que défini ci-dessus,
29) -N=CH-N(R6)-, dans lequel R6 est tel que défini ci-dessus,
30) -N(R6)-C(R7)=N-, dans lequel R6 est tel que défini ci-dessus et R7 est -NH-R6,
31) -N=C(R7)-N(R6)-, dans lequel R6 est tel que défini ci-dessus et R7 est -NH-R6, ou
32) -(C2-C6)-alcynylène,

le cycle1, le cycle2 et le cycle3 sont identiques ou différents et sont, indépendamment les uns des autres,

1) une liaison covalente,

2) -($C_6$-$C_{14}$)-aryle, dans lequel aryle est non substitué ou substitué, indépendamment les uns des autres, une, deux ou trois fois, par G, ou
3) un cycle Het à 4 à 15 chaînons, dans lequel le cycle Het est non substitué ou substitué, indépendamment les uns des autres, une, deux, ou trois fois, par G,

le cycle4 est

1) -($C_6$-$C_{14}$)-aryle, dans lequel aryle est non substitué ou substitué, indépendamment les uns des autres, une, deux ou trois fois, par G,
2) un cycle Het à 4 à 15 chaînons, dans lequel le cycle Het est non substitué ou substitué, indépendamment les uns des autres, une, deux, ou trois fois, par G, ou
3) l'un des radicaux suivants

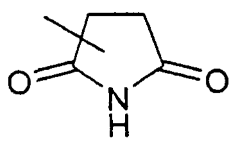 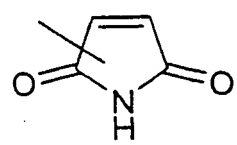

et ces radicaux sont non substitués ou substitués une fois par G,

G est

1) un atome d'hydrogène,
2) halogène,
3) =O,
4) -($C_1$-$C_6$)-alkyle, dans lequel alkyle est non substitué ou substitué, une, deux ou trois fois, par halogène, -($C_3$-$C_6$)-cycloalkyle, -($C_2$-$C_6$)-alcynyle, -($C_6$-$C_{14}$)-aryle ou le cycle Het,
5) -($C_6$-$C_{14}$)-aryle,
6) le cycle Het,
7) -C(O)-O-R10, dans lequel R10 est

a) -($C_1$-$C_6$)-alkyle, dans lequel alkyle est non substitué ou substitué, une ou deux fois, par - ($C_3$-$C_6$) -cycloalkyle, -($C_2$-$C_6$)-alcynyle, -($C_6$-$C_{14}$)-aryle ou le cycle Het, ou
b) -($C_6$-$C_{14}$)-aryle ou le cycle Het,

8) -C(S)-O-R10, dans lequel R10 est tel que défini ci-dessus,
9) -C(O)-NH-R11, dans lequel R11 est

a) -($C_1$-$C_6$)-alkyle, dans lequel alkyle est non substitué ou substitué, une ou deux fois, par -($C_3$-$C_6$)-cycloalkyle, -($C_6$-$C_{14}$)-aryle ou le cycle Het, ou
b) -($C_6$-$C_{14}$)-aryle ou le cycle Het,

10) -C(S)-NH-R11, dans lequel R11 est tel que défini ci-dessus,
11) -O-R12, dans lequel R12 est

a) un atome d'hydrogène,
b) -($C_1$-$C_6$)-alkyle, dans lequel alkyle est non substitué ou substitué, une, deux ou trois fois, par halogène, -($C_3$-$C_6$)-cycloalkyle, -($C_2$-$C_6$)-alcynyle, -($C_6$-$C_{14}$)-aryle ou le cycle Het,
c) -($C_6$-$C_{14}$)-aryle,
d) le cycle Het,
e) -C(O)-O-R13, dans lequel R13 est

e) 1) -($C_1$-$C_6$)-alkyle, dans lequel alkyle est non substitué ou substitué, une ou deux fois, par - ($C_3$-$C_6$) -cycloalkyle, - ($C_2$-$C_6$)-alcynyle, -($C_6$-$C_{14}$)-aryle ou le cycle Het, ou
e)2) -($C_6$-$C_{14}$)-aryle ou le cycle Het,

f) -C(S)-O-R13, dans lequel R13 est tel que défini ci-dessus,

g) -C(O)-NH-R14, dans lequel R14 est

g)1) -($C_1$-$C_6$)-alkyle, dans lequel alkyle est non substitué ou substitué, une ou deux fois, par -($C_3$-$C_6$)-cycloalkyle, -($C_2$-$C_6$)-alcynyle, -($C_6$-$C_{14}$)-aryle ou le cycle Het, ou
g)2) - ($C_6$-$C_{14}$) -aryle ou le cycle Het, ou

h) -C(S)-NH-R14, dans lequel R14 est tel que défini ci-dessus,

12) -C(O)-R10, dans lequel R10 est tel que défini ci-dessus,
13) -$S(O)_p$-R12, dans lequel R12 est tel que défini ci-dessus et p est l'entier zéro, 1 ou 2,
14) -$NO_2$,
15) -CN,
16) -N(R15)-R12, dans lequel R15 est

16)1) un atome d'hydrogène,
16)2) -($C_1$-$C_6$)-alkyle, ou
16)3) -$SO_2$-($C_1$-$C_6$)-alkyle, dans lequel alkyle est non substitué ou substitué, une ou deux fois, par -($C_3$-$C_6$)-cycloalkyle, - ($C_2$-$C_6$)-alcynyle, -($C_6$-$C_{14}$)-aryle ou le cycle Het, et R12 est tel que défini ci-dessus, ou

17) -$SO_2$-N(R12)-R1, dans lequel R12 est tel que défini ci-dessus et R1 est tel que défini ci-dessous,
X est -OH ou -NH-OH,
n1 est l'entier 2,
n2 est l'entier 3,
R1, R2, R3, R4 et R5 sont identiques ou différents et sont, indépendamment les uns des autres,

1) un atome d'hydrogène,
2) -($C_1$-$C_6$)-alkyle, dans lequel alkyle est non substitué ou substitué, une ou deux fois, par -($C_3$-$C_6$)-cycloalkyle, -($C_2$-$C_6$)-alcynyle, -($C_6$-$C_{14}$)-aryle ou le cycle Het,
3) -C(O)-O-R8, dans lequel R8 est,

3)1) un atome d'hydrogène,
3)2) -($C_1$-$C_6$)-alkyle, dans lequel alkyle est non substitué ou substitué, une ou deux fois, par -($C_3$-$C_6$)-cycloalkyle, -($C_2$-$C_6$)-alcynyle, -($C_6$-$C_{14}$)-aryle ou le cycle Het, ou est substitué une à cinq fois par fluor, ou
3)3) -($C_6$-$C_{14}$)-aryle ou le cycle Het,

4) -O-R8, dans lequel R8 a la signification mentionnée ci-dessus, ou
5) -($C_3$-$C_6$)-cycloalkyle.

**2.** Composé de formule I selon la revendication 1, dans lequel
A est -($C_0$-$C_4$)-alkylène,
B, D et E sont identiques ou différents et sont, indépendamment les uns des autres, -($C_0$-$C_4$)-alkylène ou le radical -B1-B2-B3- dans lequel B1 est -$(CH_2)_n$-, dans lequel n est l'entier zéro, 1 ou 2,
B3 est -$(CH_2)_m$-, dans lequel m est l'entier zéro, 1 ou 2,
à condition que la somme de n et de m vaille zéro, 1 ou 2, et
B2 est

1) -C(O)-
2) -($C_2$-$C_4$)-alcénylène,
3) -$S(O)_o$-, où o est l'entier zéro, 1 ou 2,
4) -N(R6)-, dans lequel R6 est un atome d'hydrogène, méthyle ou éthyle,
5) -N(R6)-C(Y)-, dans lequel Y est un atome d'oxygène ou un atome de soufre et R6 est tel que défini ci-dessus,
6) -C(Y)-N(R6)-, dans lequel Y est un atome d'oxygène ou un atome de soufre et R6 est tel que défini ci-dessus,
7) -N(R6)-$SO_2$-, dans lequel R6 est tel que défini ci-dessus,
8) -$SO_2$-N(R6)-, dans lequel R6 est tel que défini ci-dessus,
9) -N(R6)-$SO_2$-N(R6)-, dans lequel R6 est tel que défini ci-dessus,
10) -N(R6)-C(Y)-N(R6)-, dans lequel Y est un atome d'oxygène ou un atome de soufre et R6 est tel que défini ci-dessus,
11) -O-C(O)-N(R6)-,

12) -NH-C(O)-O-,
13) -O-,
14) -C (O)-O-,
15) -O-C(O)-,
16) -O-C(O)-O-,
17) -O-$CH_2$-C(O)-,
18) -O-$CH_2$-C(O)-O-,
19) -O-$CH_2$-C(O)-N(R6)-, dans lequel R6 est tel que défini ci-dessus,
20) -C (O)-$CH_2$-O-,
21) -O-C(O)-$CH_2$-O-,
22) -N(R6)-C(O)-$CH_2$-O-, dans lequel R6 est tel que défini ci-dessus,
23) -O-$(CH_2)_n$-O-, dans lequel n est l'entier 2 ou 3, ou
24) -O-$(CH_2)_m$-N(R6)-, dans lequel m est l'entier 2 ou 3 et R6 est tel que défini ci-dessus,
25) -N(R6)-$(CH_2)_m$-O-, dans lequel m est l'entier 2 ou 3 et R6 est tel que défini ci-dessus,
26) -N(R6)-N(R6)-, dans lequel R6 est tel que défini ci-dessus,
27) -N=N-,
28) -N(R6)-CH=N-, dans lequel R6 est tel que défini ci-dessus,
29) -N=CH-N(R6)-, dans lequel R6 est tel que défini ci-dessus,
30) -N(R6)-C(R7)=N-, dans lequel R6 est tel que défini ci-dessus et R7 est -NH-R6,
31) -N=C(R7)-N(R6)-, dans lequel R6 est tel que défini ci-dessus et R7 est -NH-R6, ou
32) -($C_2$-$C_6$)-alcynylène,

le cycle1, le cycle2 et le cycle3 sont identiques ou différents et sont, indépendamment les uns des autres,

1) une liaison covalente,
2) phényle ou naphtyle et sont non substitués ou substitués, indépendamment les uns des autres, une, deux ou trois fois, par G, ou
3) le cycle Het à 4 à 15 chaînons, dans lequel le cycle Het est un radical de la série acridinyle, azépinyle, azétidinyle, aziridinyle, benzimidazalinyle, benzimidazolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, quinazolinyle, quinoléinyle, 4H-quinolizinyle, quinoxalinyle, quinuclidinyle, chromanyle, chroményle, cinnolinyle, décahydroquinoléinyle, dibenzofuranyle, dibenzothiophényle, dihydrofurane[2,3-b]tétrahydrofuranyle, dihydrofuranyle, dioxolyle, dioxanyle, 2H,6H-1,5,2-dithiazinyle, furanyle, furazanyle, imidazolidinyle, imidazolinyle, imidazolyle, 1H-indazolyle, indolinyle, indolizinyle, indolyle, 3H-indolyle, isobenzofuranyle, isochromanyle, isoindazolyle, isoindolinyle, isoindolyle, isoquinoléinyle (benzimidazolyle), isothiazolidinyle, 2-isothiazolinyle, isothiazolyle, isoxazolyle, isoxazolidinyle, 2-isoxazolinyle, morpholinyle, naphtyridinyle, octahydroisoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyroazolidinyle, pyrazolinyle, pyrazolyle, pyridazinyle, pyridooxazolyle, pyridoimidazolyle, pyridothiazolyle, pyridothiophényle, pyridinyle, pyridyle, pyrimidinyle, pyrrolidinyle, pyrrolinyle, 2H-pyrrolyle, pyrrolyle, tétrahydrofuranyle, tétrahydroisoquinoléinyle, tétrahydroquinoléinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiénooxazolyle, thiénoimidazolyle, thiomorpholinyle, thiophényle, triazinyle, 1,2,3-triazolyle, 1,2,4-triazolyle, 1,2,5-triazolyle, 1,3,4-triazolyle et xanthényle, et ces radicaux sont non substitués ou substitués, indépendamment les uns des autres, une, deux ou trois fois, par G,

le cycle4 est

1) -($C_6$-$C_{14}$)-aryle, dans lequel aryle est un radical de la série phényle, naphtyle, 1-naphtyle, 2-naphtyle, anthryle et fluorényle, et ces radicaux sont non substitués ou substitués, indépendamment les uns des autres, une, deux ou trois fois, par G,
2) le cycle Het à 4 à 15 chaînons, dans lequel le cycle Het est un radical de la série acridinyle, azépinyle, azétidinyle, aziridinyle, benzimidazalinyle, benzimidazolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, quinazolinyle, quinoléinyle, 4H-quinolizinyle, quinoxalinyle, quinuclidinyle, chromanyle, chroményle, cinnolinyle, décahydroquinoléinyle, dibenzofuranyle, dibenzothiophényle, dihydrofurane[2,3-b]tétrahydrofuranyle, dihydrofuranyle, dioxolyle, dioxanyle, 2H,6H-1,5,2-dithiazinyle, furanyle,

furazanyle, imidazolidinyle, imidazolinyle, imidazolyle, 1H-indazolyle, indolinyle, indolizinyle, indolyle, 3H-indolyle, isobenzofuranyle, isochromanyle, isoindazolyle, isoindolinyle, isoindolyle, isoquinoléinyle (benzimidazolyle), isothiazolidinyle, 2-isothiazolinyle, isothiazolyle, isoxazolyle, isoxazolidinyle, 2-isoxazolinyle, 2'-méthylbiphényl-2-ol, morpholinyle, naphtyridinyle, octahydroisoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyroazolidinyle, pyrazolinyle, pyrazolyle, pyridazinyle, pyridooxazolyle, pyridoimidazolyle, pyridothiazolyle, pyridothiophényle, pyridinyle, pyridyle, pyrimidinyle, pyrrolidinyle, pyrrolinyle, 2H-pyrrolyle, pyrrolyle, tétrahydrofuranyle, tétrahydroisoquinoléinyle, tétrahydroquinoléinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiénooxazolyle, thiénoimidazolyle, thiomorpholinyle, thiophényle, triazinyle, 1,2,3-triazolyle, 1,2,4-triazolyle, 1,2,5-triazolyle, 1,3,4-triazolyle et xanthényle, et sont non substitués ou substitués, indépendamment les uns des autres, une, deux ou trois fois, par G, ou

3) l'un des radicaux suivants

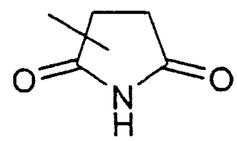 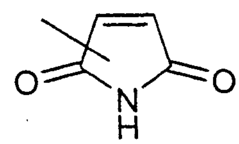

et ces radicaux sont non substitués ou substitués une fois par G,

G est

1) un atome d'hydrogène,
2) halogène,
3) =O,
4) -($C_1$-$C_6$)-alkyle, dans lequel alkyle est non substitué ou substitué, une, deux ou trois fois, par halogène, -($C_3$-$C_6$)-cycloalkyle, -($C_2$-$C_6$)-alcynyle, -($C_6$-$C_{14}$)-aryle ou le cycle Het, où aryle et le cycle Het sont tels que définis ci-dessus,
5) -($C_6$-$C_{14}$)-aryle, où aryle est tel que défini ci-dessus,
6) le cycle Het, où le cycle Het est tel que défini ci-dessus,
7) -C(O)-O-R10, dans lequel R10 est

a) -($C_1$-$C_6$)-alkyle, dans lequel alkyle est non substitué ou substitué, une ou deux fois, par -($C_3$-$C_6$)-cycloalkyle, -($C_2$-$C_6$)-alcynyle, -($C_6$-$C_{14}$)-aryle ou le cycle Het, où aryle et le cycle Het sont tels que définis ci-dessus, ou
b) -($C_6$-$C_{14}$)-aryle ou le cycle Het, où aryle et le cycle Het sont tels que définis ci-dessus,

8) -C(S)-O-R10, où R10 est tel que défini ci-dessus,
9) -C(O)-NH-R11, dans lequel R11 est

a) -($C_1$-$C_6$)-alkyle, dans lequel alkyle est non substitué ou substitué, une ou deux fois, par -($C_3$-$C_6$)-cycloalkyle, -($C_2$-$C_6$)-alcynyle, -($C_6$-$C_{14}$)-aryle ou le cycle Het, où aryle et le cycle Het sont tels que définis ci-dessus, ou
b) -($C_6$-$C_{14}$)-aryle ou le cycle Het, où aryle et le cycle Het sont tels que définis ci-dessus,

10) -C(S)-NH-R11, dans lequel R11 est tel que défini ci-dessus,
11) -O-R12, dans lequel R12 est

a) un atome d'hydrogène,
b) -($C_1$-$C_6$)-alkyle, dans lequel alkyle est non substitué ou substitué, une, deux ou trois fois, par halogène, - ($C_3$-$C_6$)-cycloalkyle, -($C_2$-$C_6$)-alcynyle, -($C_6$-$C_{14}$)-aryle ou le cycle Het, où aryle et le cycle Het sont tels que définis ci-dessus,
c) -($C_6$-$C_{14}$)-aryle, où aryle est tel que défini ci-dessus,
d) le cycle Het, où le cycle Het est tel que défini ci-dessus,

e) -C(O)-O-R13, dans lequel R13 est

e)1) $-(C_1-C_6)$-alkyle, dans lequel alkyle est non substitué ou substitué, une ou deux fois, par $-(C_3-C_6)$-cycloalkyle, $-(C_2-C_6)$-alcynyle, $-(C_6-C_{14})$-aryle ou le cycle Het, où aryle et le cycle Het sont tels que définis ci-dessus, ou
e)2) - $(C_6-C_{14})$ -aryle ou le cycle Het, où aryle et le cycle Het sont tels que définis ci-dessus,

f) -C(S)-O-R13, dans lequel R13 est tel que défini ci-dessus,
g) -C(O)-NH-R14, dans lequel R14 est

g)1) $-(C_1-C_6)$-alkyle, dans lequel alkyle est non substitué ou substitué, une ou deux fois, par $-(C_3-C_6)$-cycloalkyle, $-(C_2-C_6)$-alcynyle, $-(C_6-C_{14})$-aryle ou le cycle Het, où aryle et le cycle Het sont tels que définis ci-dessus, ou
g)2) - $(C_6-C_{14})$-aryle ou le cycle Het, où aryle et le cycle Het sont tels que définis ci-dessus, ou

h) -C(S)-NH-R14, dans lequel R14 est tel que défini ci-dessus,

12) -C(O)-R10, dans lequel R10 est tel que défini ci-dessus,
13) $-S(O)_p$-R12, dans lequel R12 est tel que défini ci-dessus et p est l'entier zéro, 1 ou 2,
14) $-NO_2$,
15) -CN,
16) -N(R15)-R12, dans lequel R15 est

16)1) un atome d'hydrogène,
16)2) $-(C_1-C_6)$-alkyle ou
16)3) $-SO_2-(C_1-C_6)$-alkyle, dans lequel alkyle est non substitué ou substitué, une ou deux fois, par $-(C_3-C_6)$-cycloalkyle, - $(C_2-C_6)$-alcynyle, $-(C_6-C_{14})$-aryle ou le cycle Het, et R12 est tel que défini ci-dessus,

17) $-SO_2$-N (R12)-R1, dans lequel R12 est tel que défini ci- dessus et R1 est tel que défini ci- dessous,

X est -OH ou -NH-OH,
n1 est l'entier 2,
n2 est l'entier 3,
R1, R2, R3, R4 et R5 sont identiques ou différents et sont, indépendamment les uns des autres,

1) un atome d'hydrogène,
2) $-(C_1-C_6)$-alkyle, dans lequel alkyle est non substitué ou substitué, une ou deux fois, par $-(C_3-C_6)$-cycloalkyle, $-(C_2-C_6)$-alcynyle, $-(C_6-C_{14})$-aryle ou le cycle Het,
3) -C(O)-O-R8, dans lequel R8 est

3)1) un atome d'hydrogène,
3)2) $-(C_1-C_6)$-alkyle, dans lequel alkyle est non substitué ou substitué, une ou deux fois, par $-(C_3-C_6)$-cycloalkyle, $-(C_2-C_6)$-alcynyle, $-(C_6-C_{14})$-aryle ou le cycle Het, ou est substitué une à cinq fois par fluor, ou
3)3) $(C_6-C_{14})$-aryle ou le cycle Het,

4) -O-R8, dans lequel R8 a la signification mentionnée ci-dessus, ou
5) $-(C_3-C_6)$-cycloalkyle.

**3.** Composé de formule I selon la revendication 1 ou 2, dans lequel
A est - $(C_0-C_4)$-alkylène,
B, D et E sont identiques ou différents et sont, indépendamment les uns des autres, $-(C_0-C_4)$-alkylène ou le radical -B1-B2-B3- dans lequel B1 est $-(CH_2)_n-$, dans lequel n est l'entier zéro, 1 ou 2,
B3 est $-(CH_2)_m-$, dans lequel m est l'entier zéro, 1 ou 2,
à condition que la somme de n et de m vaille zéro, 1 ou 2, et
B2 est

1) $-(C_0-C_2)$-alkylène,
2) éthénylène,

3) éthynylène,
4) -C(O)-
5) -N(R6)-C(O)-, dans lequel R6 est un atome d'hydrogène, méthyle ou éthyle,
6) -C(O)-N(R6)-, dans lequel R6 est tel que défini ci-dessus,
7) -O-, ou
8) -S-,

le cycle1, le cycle2 et le cycle3 sont identiques ou différents et sont, indépendamment les uns des autres,

1) une liaison covalente,
2) phényle ou naphtyle et sont non substitués ou substitués, indépendamment les uns des autres, une ou deux fois, par G, ou
3) le cycle Het, dans lequel le cycle Het est un radical de la série dihydrofuranyle, furanyle, pyridinyle, pyrimidinyle, pyrrolyle, thiadiazolyle, thiazolyle ou thiophényle, et les radicaux sont non substitués ou substitués, indépendamment les uns des autres, une ou deux fois, par G,

le cycle4 est

1) phényle ou naphtyle et est non substitué ou substitué, indépendamment les uns des autres, une ou deux fois, par G,
2) le cycle Het, dans lequel le cycle Het est un radical de la série benzofuranyle, dihydrofuranyle, dibenzofuranyle, dibenzothiophényle, furanyle, 2'-méthylbiphényl-2-ol, morpholinyle, pipérazinyle, pipéridinyle, pyridinyle, pyrimidinyle, pyridothiophényle, pyrrolyle, pyrrolidinyle, thiazolyle ou thiophényle et est non substitué ou substitué, indépendamment les uns des autres, une ou deux fois, par G, ou
3) le radical suivant

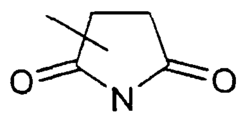

et ce radical est non substitué ou substitué une fois par G,

G est

1) un atome d'hydrogène,
2) Br, Cl ou F,
3) -($C_1$-$C_4$)-alkyle, dans lequel alkyle est non substitué ou substitué une ou deux fois, par F, phényle, -$C_3$-cycloalkyle ou le cycle Het, où le cycle Het est tel que défini ci-dessus,
4) phényle,
5) le cycle Het, où le cycle Het est tel que défini ci-dessus,
6) -C(O)-O-R10, dans lequel R10 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle, ou
c) le cycle Het, où le cycle Het est tel que défini ci-dessus,

7) -C(O)-NH-R11, dans lequel R11 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle, ou
c) le cycle Het, où le cycle Het est tel que défini ci-dessus,

8) -O-R12, dans lequel R12 est

a) un atome d'hydrogène,

b) -($C_1$-$C_6$)-alkyle, dans lequel alkyle est non substitué ou substitué, une, deux ou trois fois, par halogène, cyclopropyle, phényle ou le cycle Het, où le cycle Het est tel que défini ci-dessus,
c) phényle,
d) le cycle Het, où le cycle Het est tel que défini ci-dessus,
e) -C(O)-O-R13, dans lequel R13 est

e)1) -($C_1$-$C_6$)-alkyle, dans lequel alkyle est non substitué ou substitué, une ou deux fois, par cyclopropyle, phényle ou le cycle Het, où le cycle Het est tel que défini ci-dessus, ou
e)2) phényle ou le cycle Het, où le cycle Het est tel que défini ci-dessus,

f) -C(S)-O-R13, dans lequel R13 est tel que défini ci-dessus, ou
g) -C(O)-NH-R14, dans lequel R14 est

g)1) -($C_1$-$C_6$)-alkyle, dans lequel alkyle est non substitué ou substitué, une ou deux fois, par phényle ou le cycle Het, où le cycle Het est tel que défini ci-dessus, ou
g)2) phényle ou le cycle Het, où le cycle Het est tel que défini ci-dessus,

9) -C(O)-R10, dans lequel R10 est tel que défini ci-dessus,
10) -$S(O)_p$-R12, dans lequel R12 est tel que défini ci-dessus et p est l'entier 1 ou 2,
11) -$NO_2$,
12) -CN, ou
13) -N(R15)-R12, dans lequel R15 est

13)1) un atome d'hydrogène, ou
13)2) -($C_1$-$C_6$)-alkyle et R12 est tel que défini ci-dessus,

X est -OH ou -NH-OH,
n1 est l'entier 2,
n2 est l'entier 3,
R1, R2 et R3 sont dans chaque cas, un atome d'hydrogène,
R4 et R5 sont identiques ou différents et sont, indépendamment l'un de l'autre,

1) un atome d'hydrogène,
2) méthyle,
3) éthyle, ou
4) -OH.

**4.** Composé de formule I selon une ou plusieurs des revendications 1 à 3, dans lequel
A est -($C_0$-$C_4$)-alkylène,
B, D et E sont identiques ou différents et sont, indépendamment les uns des autres, -($C_0$-$C_4$)-alkylène ou le radical -B1-B2-B3- dans lequel B1 est -$(CH_2)_n$-, dans lequel n est l'entier zéro, 1 ou 2,
B3 est -$(CH_2)_m$-, dans lequel m est l'entier zéro, 1 ou 2,
à condition que la somme de n et de m vaille zéro, 1 ou 2, et
B2 est

1) -($C_0$-$C_2$) -alkylène,
2) éthénylène, ou
3) éthynylène,

le cycle1, le cycle2 et le cycle3 sont identiques ou différents et sont, indépendamment les uns des autres,

1) une liaison covalente,
2) phényle, et sont non substitués ou substitués, indépendamment les uns des autres, une ou deux fois, par G, ou
3) le cycle Het, dans lequel le cycle Het est un radical de la série dihydrofuranyle, furanyle, morpholinyle, pipérazinyle, pipéridinyle, pyridinyle, pyrimidinyle, pyrrolyle, thiazolyle ou thiophényle, et sont non substitués ou substitués, indépendamment les uns des autres, une ou deux fois, par G,

le cycle4 est

1) phényle, et est non substitué ou substitué, indépendamment les uns des autres, une ou deux fois, par G,
2) le cycle Het, dans lequel le cycle Het est un radical de la série benzofuranyle, dihydrofuranyle, dibenzofuranyle, dibenzothiophényle, furanyle, 2'-méthylbiphényl-2-ol, morpholinyle, pipérazinyle, pipéridinyle, pyridinyle, pyrimidinyle, pyridothiophényle, pyrrolyle, pyrrolidinyle, thiazolyle ou thiophényle et est non substitué ou substitué, indépendamment les uns des autres, une ou deux fois, par G, ou
3) le radical suivant

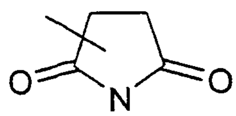

et ce radical est non substitué ou substitué une fois par G,

G est

1) un atome d'hydrogène,
2) Br, Cl ou F,
3) -($C_1$-$C_4$)-alkyle, dans lequel alkyle est non substitué ou substitué une, deux, ou trois fois par Br, Cl, F, -$C_3$-cycloalkyle, phényle ou le cycle Het, où le cycle Het est tel que défini ci-dessus,
4) phényle,
5) le cycle Het, où le cycle Het est tel que défini ci-dessus,
6) -C(O)-O-R10, dans lequel R10 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle, ou
c) le cycle Het, où le cycle Het est tel que défini ci-dessus,

7) -C(O)-NH-R11, dans lequel R11 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle ou naphtyle, ou
c) le cycle Het, où le cycle Het est tel que défini ci-dessus,

8) -O-R12, dans lequel R12 est

a) un atome d'hydrogène,
b) -($C_1$-$C_6$)-alkyle, dans lequel alkyle est non substitué ou substitué, une, deux ou trois fois, par halogène, cyclopropyle, phényle ou le cycle Het, où le cycle Het est tel que défini ci-dessus,
c) phényle,
d) le cycle Het, où le cycle Het est tel que défini ci-dessus,
e) -C(O)-O-R13, dans lequel R13 est

e)1) -($C_1$-$C_6$)-alkyle, dans lequel alkyle est non substitué ou substitué, une ou deux fois, par cyclopropyle, phényle, naphtyle ou le cycle Het, où le cycle Het est tel que défini ci-dessus, ou
e)2) phényle ou le cycle Het, où le cycle Het est tel que défini ci-dessus,

f) -C(S)-O-R13, dans lequel R13 est tel que défini ci-dessus, ou
g) -C(O)-NH-R14, dans lequel R14 est

g)1) -($C_1$-$C_6$)-alkyle, dans lequel alkyle est non substitué ou substitué, une ou deux fois, par phényle ou le cycle Het, où le cycle Het est tel que défini ci-dessus, ou
g)2) phényle ou le cycle Het, où le cycle Het est tel que défini ci-dessus,

9) -C(O)-R10, dans lequel R10 est tel que défini ci-dessus,
10) -S(O)$_p$-R12, dans lequel R12 est tel que défini ci-dessus et p est l'entier 1 ou 2,

11) $-NO_2$,
12) -CN, ou
13) -N(R15)-R12, dans lequel R15 est

13)1) un atome d'hydrogène, ou
13)2) $-(C_1-C_6)$-alkyle et R12 est tel que défini ci-dessus,

X est -OH ou -NH-OH,
n1 est l'entier 2,
n2 est l'entier 3,
R1, R2, R3, R4 et R5 sont dans chaque cas, un atome d'hydrogène.

**5.** Composé de formule I selon une ou plusieurs des revendications 1 à 4, dans lequel
A est une liaison covalente ou $-CH_2-CH_2-$, B, D et E sont identiques ou différents et sont, indépendamment les uns des autres, $-(C_0-C_4)$-alkylène ou le radical -B1-B2-B3- dans lequel B1 est $-(CH_2)_n-$, dans lequel n est l'entier zéro, 1 ou 2,
B3 est $(CH_2)_m-$, dans lequel m est l'entier zéro, 1 ou 2,
à condition que la somme de n et de m vaille zéro, 1 ou 2, et
B2 est

1) -C (O) -
2) $-(C_2-C_4)$-alcynylène,
3) $-S(O)_o-$, où o est l'entier zéro ou 1,
4) -N(R6)-C(Y)-, dans lequel Y est un atome d'oxygène et R6 est un atome d'hydrogène,
5) -C(Y)-N(R6)-, dans lequel Y est un atome d'oxygène et R6 est un atome d'hydrogène, ou
6) -O-,

le cycle1, le cycle2 et le cycle3 sont identiques ou différents et sont, indépendamment les uns des autres,

1) une liaison covalente,
2) phényle et sont non substitués ou substitués, indépendamment les uns des autres, une ou deux fois, par G, ou
3) le cycle Het, dans lequel le cycle Het est un radical de la série furanyle, pyridinyle, pyrimidinyle ou thiophényle, et sont non substitués ou substitués, indépendamment les uns des autres, une ou deux fois, par G,

le cycle4 est

1) phényle et est non substitué ou substitué, indépendamment les uns des autres, une ou deux fois, par G,
2) le cycle Het, dans lequel le cycle Het est un radical de la série benzofuranyle, dibenzofuranyle, furanyle, 2'-méthylbiphényl-2-ol, morpholinyle, pipérazinyle, pipéridinyle, pyridinyle, pyrimidinyle, pyridothiophényle, pyrrolyle, pyrrolidinyle, thiazolyle ou thiophényle et est non substitué ou substitué, indépendamment les uns des autres, une ou deux fois, par G, ou
3) le radical suivant

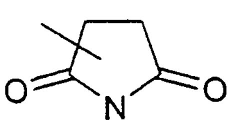

et ce radical est non substitué ou substitué une fois par G,

G est

1) un atome d'hydrogène,
2) Br, Cl ou F,
3) $-(C_1-C_4)$-alkyle, dans lequel alkyle est non substitué ou substitué une, deux, ou trois fois par Br, Cl, F, $-C_3$-cycloalkyle, phényle ou le cycle Het, où le cycle Het est tel que défini ci-dessus,
4) phényle,

5) le cycle Het, où le cycle Het est tel que défini ci-dessus,
6) -C(O)-O-R10, dans lequel R10 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle, ou
c) le cycle Het, où le cycle Het est tel que défini ci-dessus,

7) -C(O)-NH-R11, dans lequel R11 est

a) -($C_1$-$C_6$)-alkyle, dans lequel alkyle est non substitué ou substitué, une ou deux fois, par cyclopropyle, phényle ou le cycle Het, où le cycle Het est tel que défini ci-dessus,
b) phényle, ou
c) le cycle Het, où le cycle Het est tel que défini ci-dessus,

8) -O-R12, dans lequel R12 est

a) un atome d'hydrogène,
b) -($C_1$-$C_6$)-alkyle, dans lequel alkyle est non substitué ou substitué, une, deux ou trois fois, par halogène, cyclopropyle, phényle ou le cycle Het, où le cycle Het est tel que défini ci-dessus,
c) phényle,
d) le cycle Het, où le cycle Het est tel que défini ci-dessus,
e) -C(O)-O-R13, dans lequel R13 est

e)1) -($C_1$-$C_6$)-alkyle, dans lequel alkyle est non substitué ou substitué, une ou deux fois, par cyclopropyle, phényle ou le cycle Het, où le cycle Het est tel que défini ci-dessus, ou
e)2) phényle ou le cycle Het, où le cycle Het est tel que défini ci-dessus,

f) -C(S)-O-R13, dans lequel R13 est tel que défini ci-dessus, ou
g) -C(O)-NH-R14, dans lequel R14 est

g)1) -($C_1$-$C_6$)-alkyle, dans lequel alkyle est non substitué ou substitué, une ou deux fois, par phényle ou le cycle Het, où le cycle Het est tel que défini ci-dessus, ou
g)2) phényle ou le cycle Het, où le cycle Het est tel que défini ci-dessus,

9) -C(O)-R10, dans lequel R10 est tel que défini ci-dessus,
10) -$S(O)_p$-R12, dans lequel R12 est tel que défini ci-dessus et p est l'entier zéro, 1 ou 2,
11) -$NO_2$,
12) -CN, ou
13) -N(R15)-R12, dans lequel R15 est

13)1) un atome d'hydrogène, ou
13)2) -($C_1$-$C_6$)-alkyle et R12 est tel que

défini ci-dessus,

X est -NH-OH,
n1 est l'entier 2,
n2 est l'entier 3, et
R1, R2, R3, R4 et R5 sont dans chaque cas, un atome d'hydrogène.

**6.** Composé de formule I selon une ou plusieurs des revendications 1 à 5, dans lequel il s'agit d'un composé de la série 2-(4'-nitrobiphényl-4-sulfonyl)décahydroisoquinoléine-1-(N-hydroxy)carboxamide, acide 2-(4'-chlorobiphényl-4-sulfonyl)décahydroisoquinoléine-1-carboxylique,
2-(4'-chlorobiphényl-4-sulfonyl)décahydroisoquinoléine-1-(N-hydroxy)carboxamide, acide 2-(6-phénoxypyridine-3-sulfonyl)décahydroisoquinoléine-1-carboxylique ; trifluoroacétate,
2-(6-phénoxypyridine-3-sulfonyl)décahydroisoquinoléine-1-(N-hydroxy)carboxamide; trifluoroacétate, acide 2-[2-(4'-chlorobiphényl-4-yl)éthanesulfonyl]décahydroisoquinoléine-1-carboxylique,

2-[2-(4'-chlorobiphényl-4-yl)éthanesulfonyl]décahydroisoquinoléine-1-(N-hydroxy)carboxamide, acide 2-[4-(pyridin-4-yloxy)benzènesulfonyl]décahydroisoquinoléine-1-carboxylique; trifluoroacétate, 2-[4-(pyridin-4-yloxy)benzènesulfonyl]décahydroisoquinoléine-1-(N-hydroxy)carboxamide; trifluoroacétate, acide 2-[4-(4-méthoxyphénoxy)benzènesulfonyl]décahydroisoquinoléine-1-carboxylique, 2-[4-(4-méthoxyphénoxy)benzènesulfonyl]décahydroisoquinoléi ne-1-(N-hydroxy)carboxamide, acide 2-{4-[4-(2,2,2-trifluoroéthoxy)phénoxy]benzènesulfonyl}décahydroisoquinoléine-1-carboxylique, 2-{4-[4-(2,2,2-trifluoroéthoxy)phénoxy]benzènesulfonyl}décahydroisoquinoléine-1-(N-hydroxy)carboxamide, acide 2-[4'-(2,2,2-trifluoroéthoxy)biphényl-4-sulfonyl]décahydroisoquinoléine-1-carboxylique, acide 2-(4'-isopropoxycarbonylaminobiphényl-4-sulfonyl)décahydroisoquinoléine-1-carboxylique, ester isopropylique de [4'-(1-hydroxycarbamoyloctahydroisoquinoléine-2-sulfonyl)biphényl-4-yl]carboxamide, 2-[4'-(2,2,2-trifluoroéthoxy)biphényl-4-sulfonyl]décahydroisoquinoléine-1-(N-hydroxy)carboxamide, 2-(4'-trifluorométhoxybiphényl-4-sulfonyl)décahydroisoquinoléine-1-(N-hydroxy)carboxamide, 2-[4-(4-fluorophénoxy)benzènesulfonyl]décahydroisoquinoléin e-1-(N-hydroxy)carboxamide, 2-[4-(4-trifluorométhoxyphénoxy)benzènesulfonyl]décahydrois oquinoléine-1-(N-hydroxy)carboxamide, acide 2-[4-(4-trifluorométhoxyphénoxy)benzènesulfonyl]décahydrois oquinoléine-1-carboxylique, 2-(biphényl-4-sulfonyl)décahydroisoquinoléine-1-(N-hydroxy)carboxamide, acide 2-(biphényl-4-sulfonyl)décahydroisoquinoléine-1-carboxylique, 2-[4-(4-cyanophénoxy)benzènesulfonyl]décahydroisoquinoléine -1-(N-hydroxy)carboxamide, acide 2-(dibenzofurane-2-sulfonyl)décahydroisoquinoléine-1-carboxylique, 2-(dibenzofurane-2-sulfonyl)décahydroisoquinoléine-1-(N-hydroxy)carboxamide, ou 2-[4-(4-fluorophénoxy)benzènesulfonyl]-6-méthoxydécahydroisoquinoléine-1-(N-hydroxy)carboxamide, et également toutes ses formes stéréoisomères.

**7.** Procédé de préparation du composé de formule I selon une ou plusieurs des revendications 1 à 6, **caractérisé en ce qu'**il comprend

a) la réaction d'un composé de formule IV,

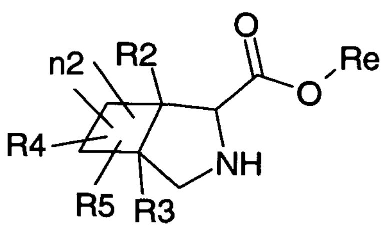

**(IV)**

dans laquelle Re est un atome d'hydrogène ou un groupement protecteur d'ester,
avec un composé de formule V,

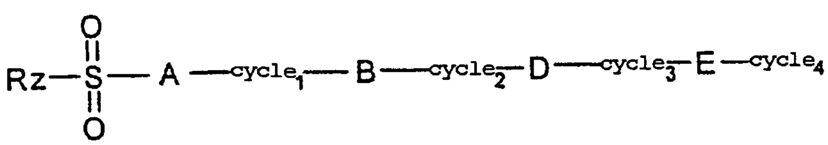

(V)

dans laquelle A, B, D, E et le cycle1, le cycle2, le cycle3 et le cycle4 sont tels que définis dans la formule I, et dans laquelle Rz est un atome de chlore, imidazolyle ou OH,
en présence d'une base ou à la suite d'une silylation avec un agent de silylation convenable, ou en utilisant un agent de déshydratation convenable lorsque Rz = OH, pour donner un composé de formule VI,

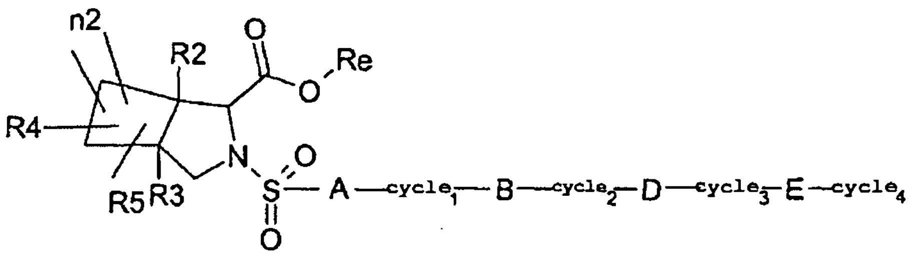

(VI)

dans laquelle A, B, D, E, Re et le cycle1, le cycle2, le cycle3 et le cycle4 sont tels que définis ci-dessus, ou
b) lorsque Re = ester, la réaction d'un composé de formule VI préparé comme décrit dans a) avec une lessive alcaline tel que NaOH ou LiOH, puis le traitement du produit avec un acide, pour donner l'acide carboxylique selon l'invention de formule I, dans laquelle X = OH (correspondant à VII), des modifications dans l'une des chaînes latérales des cycles cycle1-cycle4 ayant également été réalisées auparavant, le cas échéant ; ou la transformation dudit ester, en le traitant avec un acide minéral, tel que l'acide chlorhydrique, en l'acide carboxylique libre VII

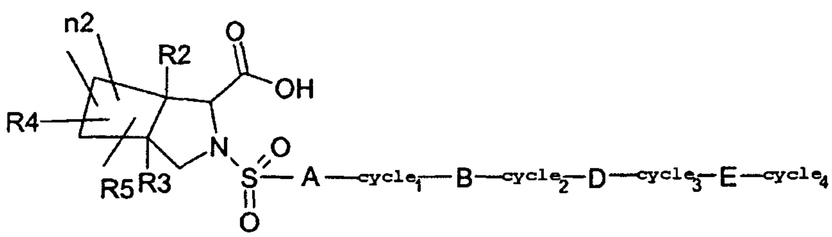

(VII)

puis la transformation de celui-ci en l'acide hydroxamique selon l'invention de formule I, dans laquelle X = NH-OH, ou
c) en utilisant la formation de sel avec des acides ou des bases énantiomériquement purs, la chromatographie en phases stationnaires chirales ou la dérivatisation avec des composés chiraux, énantiomériquement purs tels que les acides aminés, la séparation des diastéréoisomères résultants et l'élimination des groupements auxiliaires chiraux, pour séparer un composé de formule I préparé comme décrit dans le procédé a), ou un précurseur convenable de formule I, qui se présente sous des formes énantiomères en raison de sa structure chimique, en les énantiomères purs, ou
d) soit l'isolement du composé de formule I préparé comme décrit dans les procédés b) ou c) sous forme libre, soit, lorsque des groupements acides ou basiques sont présents, sa transformation en des sels physiologiquement tolérés.

8. Produit pharmaceutique, **caractérisé par** une teneur efficace d'au moins un composé de formule I selon une ou plusieurs des revendications 1 à 6, conjointement avec une substance support, un additif et/ou une substance auxiliaire pharmaceutiquement convenables et physiologiquement tolérés.

9. Utilisation du composé de formule I selon une ou plusieurs des revendications 1 à 6, pour la production d'un produit pharmaceutique destiné à la prophylaxie et à la thérapie des maladies articulaires dégénératives telles que les ostéoarthroses, les spondyloses et la chondrolyse suivant un traumatisme articulaire ou une période relativement longue d'immobilisation articulaire à la suite de blessures du ménisque ou de blessures de la patella ou de ruptures de ligaments, des maladies du tissu conjonctif telles que les collagénoses, les maladies parodontales, les perturbations de la cicatrisation et les maladies chroniques de l'appareil locomoteur telles que les arthrites aiguës et chroniques inflammatoires, déterminées immunologiquement ou déterminées par le métabolisme, les arthropathies, les myalgies, et les perturbations du métabolisme osseux, pour le traitement de l'ulcération, de l'athérosclérose et des sténoses, pour le traitement des inflammations, des maladies cancéreuses, de la formation des métastases tumorales, de la cachexie, de l'anorexie, de la défaillance cardiaque et du choc septique, ou pour la prophylaxie des infarctus du myocarde et cérébraux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0606046 A
- WO 9428889 A
- WO 9627583 A
- US 5430023 A
- US 5726159 A
- EP 643073 A
- US 4902695 A
- WO 2003041641 A
- WO 9718194 A


### In der Beschreibung aufgeführte Nicht-Patentliteratur


- **D. Yip et al.** *Investigational New Drugs,* 1999, vol. 17, 387-399
- **Michaelides et al.** *Current Pharmaceutical Design,* 1999, vol. 5, 787-819
- **S. J. George.** *Exp. Opin. Invest. Drugs,* 2000, vol. 9 (5), 993-1007
- **E. J. M. Creemers et al.** *Circulation Res.,* 2001, vol. 89, 201-210
- *Drugs,* 2001, vol. 61, 1239-1252
- *Current Medicinal Chemistry,* 2001, vol. 8, 425-74
- **Massova I et al.** *The FASEB Journal,* 1998, vol. 12, 1075-1095
- *Tetrahedron,* 1992, vol. 48, 4659-76
- **T.H. Greene ; P. G. M. Wuts.** Protective Groups in Organic Synthesis. Wiley-Interscience, 1999
- **Ye et al.** *Biochemistry,* 1992, vol. 31, 11231-11235